# EUROPEAN PATENT SPECIFICATION

(11) **EP 2 917 212 B1**
(45) Date of publication and mention of the grant of the patent: **12.06.2019**
(21) Application number: 13798455.5
(22) Date of filing: 08.11.2013
(51) Int. Cl.: C07D 413/12, C07D 417/12, C07C 235/88, C07D 277/64, C07D 513/04, A61P 31/04, A61K 31/422, A61K 31/428

(54) **ANTIMICROBIAL AGENTS**
ANTIMIKROBIELLE WIRKSTOFFE
AGENTS ANTIMICROBIENS

(30) Priority: 08.11.2012 US 201261724182 P
(43) Date of publication of application: 16.09.2015
(73) Proprietor: Rutgers, The State University of New Jersey, New Brunswick, NJ 08903 (US); Taxis Pharmaceuticals, Inc., North Brunswick, New Jersey 08902 (US)
(72) Inventor: LAVOIE, Edmond J., New Brunswick, New Jersey 08903 (US); PARHI, Ajit, New Brunswick, New Jersey 08903 (US); PILCH, Daniel S., New Brunswick, New Jersey 08903 (US); ZHANG, Yongzheng, North Brunswick, New Jersey 08902 (US); KAUL, Malvika, New Brunswick, New Jersey 08903 (US)
(74) Representative: Reitstötter Kinzebach
(86) International application number: PCT/US2013/069316
(87) International publication number: WO 2014/074932

(56) References cited:
- WO-A1-2007/107758
- WO-A1-2012/142671
- WO-A2-2004/018414
- US-A- 4 826 990
- US-A1- 2002 040 147
- US-A1- 2002 055 516
- JASBIR SINGH ET AL: "Structure-Activity Relationship Studies Leading to the Identification of (2 E )-3-[l-[(2,4-Dichlorophenyl)methyl]-5-fluo ro-3-methyl-l H -indol-7-yl]- N -[(4,5-dichloro-2-thienyl)sulfonyl]-2-prop enamide (DG-041), a Potent and Selective Prostanoid EP3 Receptor Antagonist, as a Novel Antiplatelet Agent", JOURNAL OF MEDICINAL CHEMISTRY, vol. 53, no. 1, 14 January 2010 (2010-01-14), pages 18-36, XP055101178, ISSN: 0022-2623, DOI: 10.1021/jm9005912
- MUSSER J H ET AL: "N-Ú(ARYLMETHOXY)PHENYL 3/4 CARBOXYLIC ACIDS, HYDROXAMIC ACIDS, TETRAZOLES, AND SULFONYL CARBOXAMIDES. ÖPOTENT ORALLY ACTIVE LEUKOTRIENE D4 ANTAGONISTS OF NOVEL STRUCTURE", JOURNAL OF MEDICINAL CHEMISTRY, AMERICAN CHEMICAL SOCIETY, US, vol. 33, no. 1, 1 January 1990 (1990-01-01), pages 240-245, XP000567005, ISSN: 0022-2623, DOI: 10.1021/JM00163A039

## Description

### Background of the Invention

The emergence of Multidrug Resistant (MDR) bacterial pathogens (e.g. methicillin-resistant *Staphylococcus aureus* (MRSA), *Acinetobacter baumannii-calcoaceticus* complex (ABC), etc.) has increased concerns as to the adequacy of current antimicrobials and pathogen treatment methods. The lethality of such pathogens, particularly MRSA, has often led to treatment methods that are experimental or would otherwise normally be avoided in standard clinical practice. For example, the antibiotic colistin was traditionally considered too nephrotoxic and neurotoxic for clinical use, but is nevertheless used to treat many MDR bacterial infections due to a paucity of available active drugs. The growing threat from MDR pathogens highlights a critical need for additional antimicrobials. In this connection, there is a pressing need for new antibiotics that exhibit novel mechanisms of action or that are able to circumvent known resistance pathways.

Elements of the bacterial cell division machinery present appealing targets for antimicrobial compounds because (i) they are essential for bacterial viability, (ii) they are widely conserved among bacterial pathogens, and (iii) they often have markedly different structures than their eukaryotic homologs. One such protein that has been identified as a potential target is the FtsZ protein. During the division process, FtsZ, along with approximately 15 other proteins, assemble at mid-cell into a large cell division complex (termed the divisome), ultimately facilitating cell cytokinesis. More importantly, FtsZ is widely conserved among many bacterial strains.

US 2002/040147 A1 describes compounds comprising a phenyl core and their use as antimicrobial agents.

US 2002/055516 A1 describes compounds comprising a fused bicyclic core and their use as a phosphodiesterase-4 inhibitor.

Jasbir Singh et al., "Structure-Activity Relationship Studies Leading to the Identification of (2E)-3-[1-[(2,4-Dichlorophenyl)methyl]-5-fluoro-3-methyl-1H-indol-7-yl]-N-[(4,5-dichloro-2-thienyl)sulfonyl]-2-propenamide (DG-041), a Potent and Selective Prostanoid EP3 Receptor Antagonist, as a Novel Antiplatelet Agent That Does Not Prolong Bleeding", Journal of Medicinal Chemistry, vol. 53, no. 1, 14 January 2010, pages 18-36 describe compounds comprising a fused bicyclic core and their use as EP₃ receptors.

Musser J. H. et al, "N-[(Arylmethoxy)phenyl] carboxylic acids, hydroxamic acids, tetrazoles, and sulfonyl carboxamides. Potent orally active leukotriene D4 antagonists of novel structure", Journal of Medicinal Chemistry, vol. 33, no. 1, 1 January 1990, pages 240-245 describe N-[(Arylmethoxy)phenyl] compounds and their use as LTD₄ antagonists.

US 4,826,990 A describes 2-aryl substituted heterocyclic compounds and their use as antiallergic and anti-inflammatory agents.

International Patent Application Publication Number WO 2007/107758 discusses certain compounds of the following formula: 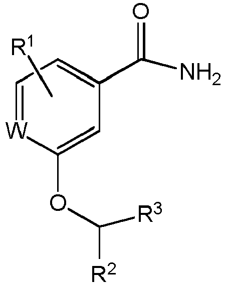 wherein W, R¹, R², and R³ have the values defined in the application; the compounds are reported to have antibiotic activity. Unfortunately, certain of the compounds discussed in this publication have solubility properties that may severely limit their use as pharmaceutical agents. Accordingly, there remains a need for antibacterial compounds that have physical properties (e.g. solubility) that make them useful as pharmaceutical agents.

### Summary of the Invention

Applicant has identified a series of antibiotic compounds that are highly soluable and that can be formulated for administration as antibiotic agents. Accordingly, in one embodiment the invention provides a compound of the invention which is a compound of formula (I): 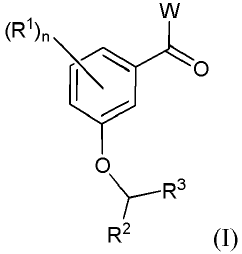 wherein:
each R¹ is independently selected from hydrogen, halo, cyano, nitro, (C₁-C₆)alkyl, (C₁-C₆)alkoxy, (C₁-C₆)alkanoyl, (C₁-C₆)alkoxycarbonyl, (C₁-C₆)alkanoyloxy, aryl, heteroaryl, heterocycle, and NR^{e}R^{f}, wherein each (C₁-C₆)alkyl, (C₁-C₆)alkoxy, (C₁-C₆)alkanoyl, (C₁-C₆)alkoxycarbonyl, (C₁-C₆)alkanoyloxy, aryl, heteroaryl, and heterocycle is optionally substituted with one or more groups independently selected from halo, cyano, nitro, NR^{e}R^{f}, -CR^{g}(=N)N(R^{g})₂, -NR^{g}C(=N)-N(R^{g})₂, -NR^{g}-C(=NR^{g})R^{g}, (C₁-C₃)alkyl , (C₁-C₃)alkoxy, (C₁-C₃)alkanoyl, (C₁-C₃)alkoxycarbonyl, (C₁-C₃)alkanoyloxy, aryl, heteroaryl, and heterocycle;
R² is H or (C₁-C₆)alkyl that is optionally substituted with one or more groups independently selected from -OR^{k}, halo, NR^{e}R^{f}, NR^{e}R^{f}, -CR^{g}(=N)N(R^{g})₂, -NR^{g}C(=N)-N(R^{g})₂, and -NR^{g}-C(=NR^{g})R^{g};
R³ is aryl or heteroaryl, which aryl or heteroaryl is optionally substituted with one or more groups independently selected from R^{h}, halo, hydroxy, -NR^{e}R^{f}, -CR^{g}(=N)N(R^{g})₂, -NR^{g}C(=N)-N(R^{g})₂, -NR^{g}-C(=NR^{g})R^{g}, (C₁-C₆)alkyl, and (C₃-C₈)cycloalkyl, wherein any (C₁-C₆)alkyl and (C₃-C₈)cycloalkyl is optionally substituted with one or more groups independently selected from halo, hydroxy, -NR^{e}R^{f}, -CR^{g}(=N)N(R^{g})₂, -NR^{g}C(=N)-N(R^{g})₂, -NR^{g}-C(=NR^{g})R^{g}, and (C₁-C₆)alkyl that is optionally substituted with one or more groups independently selected from halo;
W is -NHCOR^{a}, -N(COR^{a})(COR^{b}), -N=C(R^{c})NR^{a}R^{b}, -NR^{a}CH₂OR^{a}, -NHC(=O)OR^{a}, -NHC(=O)NR^{a}R^{b}, or -N(R^{a})SOₘR^{d};
each R^{a} is independently selected from H, aryl, heteroaryl, heterocycle, (C₃-C₈)cycloalkyl, (C₃-C₈)cycloalkyl(C₁-C₆)alkyl and (C₁-C₆)alkyl that is optionally substituted with one or more groups independently selected from hydroxy, halo, cyano, (C₁-C₆)alkoxycarbonyl, aryl, heteroaryl, -NR^{e}R^{f}, -CR^{g}(=N)N(R^{g})₂, -NR^{g}C(=N)-N(R^{g})₂, -NR^{g}-C(=NR^{g})R^{g} and heterocycle; wherein any aryl, heteroaryl, heterocycle, and (C₃-C₈)cycloalkyl(C₁-C₆)alkyl of R^{a} is optionally substituted with one or more groups independently selected from hydroxy, halo, cyano, trifluoromethoxy, (C₁-C₆)alkyl, (C₁-C₆)alkoxy, halo(C₁-C₆)alkyl, -NR^{e}R^{f}, -CR^{g}(=N)N(R^{g})₂, -NR^{g}C(=N)-N(R^{g})₂, -NR^{g}-C(=NR^{g})R^{g}, and (C₁-C₆)alkoxycarbonyl;
each R^{b} is independently selected from H and (C₁-C₆)alkyl that is optionally substituted with one or more groups independently selected from hydroxy, halo, cyano, (C₁-C₆)alkoxycarbonyl, aryl, heteroaryl, -NR^{e}R^{f}, -CR^{g}(=N)N(R^{g})₂, -NR^{g}C(=N)-N(R^{g})₂, -NR^{g}-C(=NR^{g})R^{g}, and heterocycle;
each R^{c} is independently selected from H and (C₁-C₆)alkyl that is optionally substituted with one or more groups independently selected from halo;
each R^{d} is independently selected from OH, -NH₂, -NR^{e}R^{f}, heterocycle, and (C₁-C₆)alkyl that is substituted with one or more groups independently selected from hydroxy, halo, cyano, (C₁-C₆)alkoxycarbonyl, aryl, heteroaryl, -NR^{e}R^{f}, -CH(=N)NH₂, -NHC(=N)-NH₂, -NH-C(=NH)R, and heterocycle;
each R^{e} is independently selected from H, aryl, heteroaryl, heterocycle, and (C₁-C₆)alkyl that is optionally substituted with one or more groups independently selected from hydroxy, halo, cyano, (C₁-C₆)alkoxycarbonyl, aryl, heteroaryl, and heterocycle; and each R^{f} is independently selected from H and (C₁-C₆)alkyl that is optionally substituted with one or more groups independently selected from hydroxyl, halo, cyano, (C₁-C₆)alkoxycarbonyl, aryl, heteroaryl, and heterocycle; or R^{e} and R^{f} together with the nitrogen to which they are attached form a aziridino, azetidino, morpholino, piperazino, pyrrolidino or piperidino;
each R^{g} is independently selected from H and (C₁-C₆)alkyl that is optionally substituted with one or more groups independently selected from halo;
each R^{h} is independently selected from aryl and heteroaryl, wherein any aryl and heteroaryl of R^{h} is optionally substituted with one or more groups independently selected from halo, hydroxy, -NR^{e}R^{f}, -CR^{g}(=N)N(R^{g})₂, -NR^{g}C(=N)-N(R^{g})₂, -NR^{g}-C(=NR^{g})R^{g} and (C₁-C₆)alkyl that is optionally substituted with one or more groups independently selected from hydroxy, halo, -NR^{e}R^{f}, -CR^{g}(=N)N(R^{g})₂, -NR^{g}C(=N)-N(R^{g})₂, and -NR^{g}-C(=NR^{g})R^{g};
each R^{k} is independently selected from H or (C₁-C₆)alkyl that is optionally substituted with one or more groups independently selected from hydroxy, halo, oxo, carboxy, (C₁-C₆)alkoxy, (C₁-C₆)alkoxycarbonyl, and (C₁-C₆)alkanoyloxy;
each aryl is independently a single aromatic ring or a multiple condensed ring system having 9 to 20 carbon atoms in which at least one ring is aromatic;
each cycloalkyl is independently a saturated or partially unsaturated carbocyclic ring system;
m is 0, 1, or 2; and
n is 1, 2, 3, or 4;
or a salt thereof.

The invention also provides a compound of formula I or a pharmaceutically acceptable salt thereof for use in a method for treating a bacterial infection in a mammal comprising administering to the mammal an effective amount of a compound of formula I or a pharmaceutically acceptable salt thereof.

The invention also provides a composition comprising a compound of formula I, or a pharmaceutically acceptable salt thereof, and a pharmaceutically acceptable vehicle.

The invention also provides a compound of formula I or a pharmaceutically acceptable salt thereof for the prophylactic or therapeutic treatment of a bacterial infection.

The invention also provides a compound of formula I or a pharmaceutically acceptable salt thereof for use in medical treatment.

The invention also provides the use of a compound of formula I or a pharmaceutically acceptable salt thereof for the preparation of a medicament for treating a bacterial infection in a mammal.

The invention also provides processes and intermediates disclosed herein that are useful for preparing compounds of formula I or salts thereof.

### Detailed Description

The following definitions are used, unless otherwise described: halo is fluoro, chloro, bromo, or iodo. Alkyl and alkoxy, etc. denote both straight and branched groups but reference to an individual radical such as propyl embraces only the straight chain radical (a branched chain isomer such as isopropyl being specifically referred to).

As used herein, the term "(Cₐ-C_{b})alkyl" wherein a and b are integers refers to a straight or branched chain alkyl radical having from a to b carbon atoms. Thus when a is 1 and b is 6, for example, the term includes methyl, ethyl, n-propyl, isopropyl, n-butyl, isobutyl, sec-butyl, t-butyl, n-pentyl and n-hexyl.

The term "aryl" as used herein refers to a single aromatic ring or a multiple condensed ring system. For example, an aryl group can have 6 to 20 carbon atoms, 6 to 14 carbon atoms, or 6 to 12 carbon atoms. Aryl includes a phenyl radical. Aryl also includes multiple condensed ring systems (e.g. ring systems comprising 2, 3 or 4 rings) having 9 to 20 carbon atoms in which at least one ring is aromatic. Such multiple condensed ring systems may be optionally substituted with one or more (e.g. 1, 2 or 3) oxo groups on any carbocycle portion of the multiple condensed ring system. It is to be understood that the point of attachment of a multiple condensed ring system, as defined above, can be at any position of the ring system including an aryl or a carbocycle portion of the ring. Typical aryl groups include, but are not limited to, phenyl, indenyl, naphthyl, 1, 2, 3, 4-tetrahydronaphthyl, anthracenyl, and the like.

The term "heteroaryl" as used herein refers to a single aromatic ring or a multiple condensed ring system. The term includes single aromatic rings of from about 1 to 6 carbon atoms and about 1-4 heteroatoms selected from the group consisting of oxygen, nitrogen and sulfur in the rings. The sulfur and nitrogen atoms may also be present in an oxidized form provided the ring is aromatic. Such rings include but are not limited to pyridyl, pyrimidinyl, oxazolyl or furyl. The term also includes multiple condensed ring systems (e.g. ring systems comprising 2, 3 or 4 rings) wherein a heteroaryl group, as defined above, can be condensed with one or more heteroaryls (e.g. naphthyridinyl), heterocycles, (e.g. 1, 2, 3, 4-tetrahydronaphthyridinyl), carbocycles (e.g. 5,6,7,8-tetrahydroquinolyl) or aryls (e.g. indazolyl) to form a multiple condensed ring system. Such multiple condensed ring systems may be optionally substituted with one or more (e.g. 1, 2, 3 or 4) oxo groups on the carbocycle or heterocycle portions of the condensed ring. It is to be understood that the point of attachment of a multiple condensed ring system (as defined above for a heteroaryl) can be at any position of the multiple condensed ring system including a heteroaryl, heterocycle, aryl or carbocycle portion of the multiple condensed ring system and at any suitable atom of the multiple condensed ring system including a carbon atom and heteroatom (e.g. a nitrogen). Exemplary heteroaryls include but are not limited to pyridyl, pyrrolyl, pyrazinyl, pyrimidinyl, pyridazinyl, pyrazolyl, thienyl, indolyl, imidazolyl, oxazolyl, thiazolyl, furyl, oxadiazolyl, thiadiazolyl, quinolyl, isoquinolyl, benzothiazolyl, benzoxazolyl, indazolyl, quinoxalyl, quinazolyl, 5,6,7,8-tetrahydroisoquinolinyl, benzofuranyl, benzimidazolyl and thianaphthenyl.

The term "heterocyclyl" or "heterocycle" as used herein refers to a single saturated or partially unsaturated ring or a multiple condensed ring system. The term includes single saturated or partially unsaturated rings (e.g. 3, 4, 5, 6 or 7-membered rings) from about 1 to 6 carbon atoms and from about 1 to 3 heteroatoms selected from the group consisting of oxygen, nitrogen and sulfur in the ring. The ring may be substituted with one or more (e.g. 1, 2 or 3) oxo groups and the sulfur and nitrogen atoms may also be present in their oxidized forms. Such rings include but are not limited to azetidinyl, tetrahydrofuranyl or piperidinyl. The term "heterocycle" also includes multiple condensed ring systems (e.g. ring systems comprising 2, 3 or 4 rings) wherein a single heterocycle ring (as defined above) can be condensed with one or more heterocycles (e.g. decahydronapthyridinyl), carbocycles (e.g. decahydroquinolyl) or aryls. The rings of a multiple condensed ring system can be connected to each other via fused, spiro and bridged bonds when allowed by valency requirements. It is to be understood that the point of attachment of a multiple condensed ring system (as defined above for a heterocycle) can be at any position of the multiple condensed ring system including a heterocycle, aryl and carbocycle portion of the ring. It is also to be understood that the point of attachment for a heterocycle or heterocycle multiple condensed ring system can be at any suitable atom of the heterocycle or heterocycle multiple condensed ring system including a carbon atom and a heteroatom (e.g. a nitrogen). Exemplary heterocycles include, but are not limited to aziridinyl, azetidinyl, pyrrolidinyl, piperidinyl, homopiperidinyl, morpholinyl, thiomorpholinyl, piperazinyl, tetrahydrofuranyl, dihydrooxazolyl, tetrahydropyranyl, tetrahydrothiopyranyl, 1,2,3,4- tetrahydroquinolyl, benzoxazinyl, dihydrooxazolyl, chromanyl, 1,2-dihydropyridinyl, 2,3-dihydrobenzofuranyl, 1,3-benzodioxolyl and 1,4-benzodioxanyl.

The term "halo(C₁-C₆)alkyl" includes an alkyl group as defined herein that is substituted with one or more (e.g. 1, 2, 3, or 4) halo groups.

The term "(C₃-C₈)cycloalkyl" includes saturated and partially unsaturated carbocyclic ring systems, which may include mono, fused and spiro ring systems.

Specific values listed below for radicals, substituents, and ranges, are for illustration only; they do not exclude other defined values or other values within defined ranges for the radicals and substituents.

Specifically, (C₁-C₆)alkyl can be methyl, ethyl, propyl, isopropyl, butyl, iso-butyl, sec-butyl, pentyl, 3-pentyl, or hexyl; (C₁-C₆)alkoxy can be methoxy, ethoxy, propoxy, isopropoxy, butoxy, iso-butoxy, sec-butoxy, pentoxy, 3-pentoxy, or hexyloxy; (C₃-C₈)cycloalkyl can be cyclopropyl, cyclobutyl, cyclopentyl, or cyclohexyl; (C₁-C₆)alkanoyl can be acetyl, propanoyl or butanoyl; (C₁-C₆)alkoxycarbonyl can be methoxycarbonyl, ethoxycarbonyl, propoxycarbonyl, isopropoxycarbonyl, butoxycarbonyl, pentoxycarbonyl, or hexyloxycarbonyl; halo(C₁-C₆)alkyl can be iodomethyl, bromomethyl, chloromethyl, fluoromethyl, trifluoromethyl, 2-chloroethyl, 2-fluoroethyl, 2,2,2-trifluoroethyl, or pentafluoroethyl; (C₂-C₆)alkanoyloxy can be acetoxy, propanoyloxy, butanoyloxy, isobutanoyloxy, pentanoyloxy, or hexanoyloxy; aryl can be phenyl, indenyl, or naphthyl; and heteroaryl can be furyl, imidazolyl, triazolyl, triazinyl, oxazoyl, isoxazoyl, thiazolyl, isothiazoyl, pyrazolyl, pyrrolyl, pyrazinyl, tetrazolyl, pyridyl, (or its N-oxide), thienyl, pyrimidinyl (or its N-oxide), indolyl, isoquinolyl (or its N-oxide) or quinolyl (or its N-oxide).

In one embodiment of the invention each R¹ is halo.

In one embodiment of the invention R² is H.

In one embodiment of the invention R² is (C₁-C₆)alkyl that is optionally substituted with one or more groups independently selected from hydroxy, NR^{e}R^{f}, -CH(=N)NH₂, -NHC(=N)-NH₂, and -NH-C(=NH)R.

In one embodiment of the invention R² is (C₁-C₆)alkyl that is optionally substituted with one or more groups independently selected from hydroxy, halo, -NR^{e}R^{f}, -CR^{g}(=N)N(R^{g})₂, -NR^{g}C(=N)-N(R^{g})₂, and -NR^{g}-C(=NR^{g})R^{g}.

In one embodiment of the invention R³ is aryl, which is optionally substituted with one or more groups independently selected from halo, hydroxy, NR^{e}R^{f}, -CR^{g}(=N)N(R^{g})₂,-NR^{g}C(=N)-N(R^{g})₂, -NR^{g}-C(=NR^{g})R^{g}, and (C₁-C₆)alkyl that is optionally substituted with one or more groups independently selected from hydroxy, NR^{e}R^{f}, -CR^{g}(=N)N(R^{g})₂, -NR^{g}C(=N)-N(R^{g})₂, and -NR^{g}-C(=NR^{g})R^{g}.

In one embodiment of the invention R³ is heteroaryl, which is optionally substituted with one or more groups independently selected from halo, hydroxy, NR^{e}R^{f}, -CR^{g}(=N)N(R^{g})₂, -NR^{g}C(=N)-N(R^{g})₂, -NR^{g}-C(=NR^{g})R^{g}, and (C₁-C₆)alkyl that is optionally substituted with one or more groups independently selected from hydroxy, NR^{e}R^{f}, -CR^{g}(=N)N(R^{g})₂, -NR^{g}C(=N)-N(R^{g})₂, and -NR^{g}-C(=NR^{g})R^{g}.

In one embodiment of the invention R³ is: 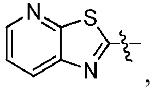 which is optionally substituted with one or more groups independently selected from halo, hydroxy, NR^{e}R^{f}, -CR^{g}(=N)N(R^{g})₂, -NR^{g}C(=N)-N(R^{g})₂, -NR^{g}-C(=NR^{g})R^{g}, and (C₁-C₆)alkyl that is optionally substituted with one or more groups independently selected from hydroxy, NR^{e}R^{f}, -CR^{g}(=N)N(R^{g})₂, -NR^{g}C(=N)-N(R^{g})₂, and -NR^{g}-C(=NR^{g})R^{g}.

In one embodiment of the invention R³ is: 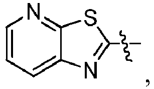 which is optionally substituted with one or more groups independently selected from halo.

In one embodiment of the invention R³ is:

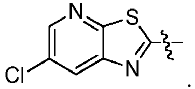

In one embodiment of the invention R³ is: 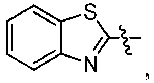 which is optionally substituted with one or more groups independently selected from halo, hydroxy, NR^{e}R^{f}, -CR^{g}(=N)N(R^{g})₂, -NR^{g}C(=N)-N(R^{g})₂, -NR^{g}-C(=NR^{g})R^{g}, and (C₁-C₆)alkyl that is optionally substituted with one or more groups independently selected from hydroxy, NR^{e}R^{f}, -CR^{g}(=N)N(R^{g})₂, -NR^{g}C(=N)-N(R^{g})₂, and -NR^{g}-C(=NR^{g})R^{g}.

In one embodiment of the invention R³ is: 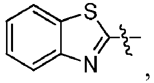 which is optionally substituted with one or more groups independently selected from halo.

In one embodiment of the invention R³ is:

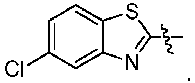

In one embodiment of the invention W is -NHC(=O)H, -NHC(=O)CH₃, -NHC(=O)CH₂CH₃, -NHC(=O)CH₂CH₂CH₃, -N=NCH-N(CH₃)₂, -NHCH₂OH, -N=NC(CH₃)-N(CH₃)₂,

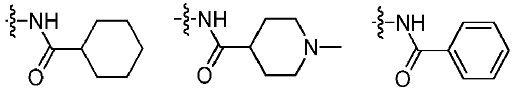

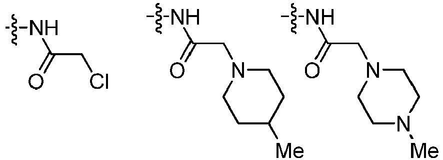

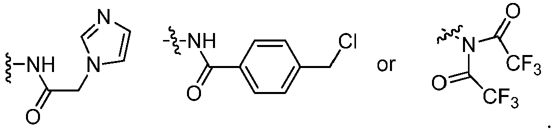

In one embodiment the invention provides a compound of formula (la): 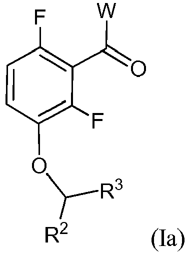 or a salt thereof.

In one embodiment:
each R¹ is independently selected from hydrogen, halo, cyano, nitro, (C₁-C₆)alkyl, (C₁-C₆)alkoxy, (C₁-C₆)alkanoyl, (C₁-C₆)alkoxycarbonyl, (C₁-C₆)alkanoyloxy, aryl, heteroaryl, heterocycle, and NR^{e}R^{f}, wherein each (C₁-C₆)alkyl , (C₁-C₆)alkoxy, (C₁-C₆)alkanoyl, (C₁-C₆)alkoxycarbonyl, (C₁-C₆)alkanoyloxy, aryl, heteroaryl, and heterocycle is optionally substituted with one or more groups independently selected from halo, cyano, nitro, -NR^{e}R^{f}, -CR^{g}(=N)N(R^{g})₂, -NR^{g}C(=N)-N(R^{g})₂, -NR^{g}-C(=NR^{g})R^{g}, (C₁-C₃)alkyl, (C₁-C₃)alkoxy, (C₁-C₃)alkanoyl, (C₁-C₃)alkoxycarbonyl, (C₁-C₃)alkanoyloxy, aryl, heteroaryl, and heterocycle;
R² is H or (C₁-C₆)alkyl that is optionally substituted with one or more groups independently selected from hydroxy, -NR^{e}R^{f}, -CR^{g}(=N)N(R^{g})₂, -NR^{g}C(=N)-N(R^{g})₂, and -NR^{g}-C(=NR^{g})R^{g};
R³ is aryl or heteroaryl, which aryl or heteroaryl is optionally substituted with one or more groups independently selected from R^{h}, halo, hydroxy, -NR^{e}R^{f}, -CR^{g}(=N)N(R^{g})₂, -NR^{g}C(=N)-N(R^{g})₂, -NR^{g}-C(=NR^{g})R^{g} and (C₁-C₆)alkyl that is optionally substituted with one or more groups independently selected from hydroxy, -NR^{e}R^{f}, -CR^{g}(=N)N(R^{g})₂, -NR^{g}C(=N)-N(R^{g})₂, and -NR^{g}-C(=NR^{g})R^{g};
W is -NHCOR^{a}, -N(COR^{a})(COR^{b}), -N=C(R^{c})NR^{a}R^{b}, -NR^{a}CH₂OR^{a}, or -N(R^{a})SOₘR^{d}_{;}
each R^{a} is independently selected from H, aryl, heteroaryl, heterocycle, (C₃-C₈)cycloalkyl, (C₃-C₈)cycloalkyl(C₁-C₆)alkyl and (C₁-C₆)alkyl that is optionally substituted with one or more groups independently selected from hydroxyl, halo, cyano, (C₁-C₆)alkoxycarbonyl, aryl, heteroaryl, -NR^{e}R^{f}, -CR^{g}(=N)N(R^{g})₂, -NR^{g}C(=N)-N(R^{g})₂, -NR^{g}-C(=NR^{g})R^{g} and heterocycle; wherein any aryl, heteroaryl, heterocycle, and (C₃-C₈)cycloalkyl(C₁-C₆)alkyl of R^{a} is optionally substituted with one or more groups independently selected from hydroxy, halo, cyano, trifluoromethoxy, (C₁-C₆)alkyl, halo(C₁-C₆)alkyl, -NR^{e}R^{f}, -CR^{g}(=N)N(R^{g})₂, -NR^{g}C(=N)-N(R^{g})₂, -NR^{g}-C(=NR^{g})R^{g}, and (C₁-C₆)alkoxycarbonyl;
each R^{b} is independently selected from H and (C₁-C₆)alkyl that is optionally substituted with one or more groups independently selected from hydroxyl, halo, cyano, (C₁-C₆)alkoxycarbonyl, aryl, heteroaryl, -NR^{e}R^{f}, -CR^{g}(=N)N(R^{g})₂, -NR^{g}C(=N)-N(R^{g})₂, -NR^{g}-C(=NR^{g})R^{g}, and heterocycle;
each R^{c} is independently selected from H and (C₁-C₆)alkyl;
each R^{d} is independently selected from OH, -NH₂, -NR^{e}R^{f}, heterocycle, and (C₁-C₆)alkyl that is substituted with one or more groups independently selected from hydroxy, halo, cyano, (C₁-C₆)alkoxycarbonyl, aryl, heteroaryl, -NR^{e}R^{f}, -CH(=N)NH₂, -NHC(=N)-NH₂, -NH-C(=NH)R, and heterocycle;
each R^{e} is independently selected from H, aryl, heteroaryl, heterocycle, and (C₁-C₆)alkyl that is optionally substituted with one or more groups independently selected from hydroxyl, halo, cyano, (C₁-C₆)alkoxycarbonyl, aryl, heteroaryl, and heterocycle; and each R^{f} is independently selected from H and (C₁-C₆)alkyl that is optionally substituted with one or more groups independently selected from hydroxyl, halo, cyano, (C₁-C₆)alkoxycarbonyl, aryl, heteroaryl, and heterocycle; or R^{e} and R^{f} together with the nitrogen to which they are attached form a aziridino, azetidino, morpholino, piperazino, pyrrolidino or piperidino;
each R^{g} is independently selected from H and (C₁-C₆)alkyl;
each R^{h} is independently selected from aryl and heteroaryl, wherein any aryl and heteroaryl of R^{h} is optionally substituted with one or more groups independently selected from halo, hydroxy, -NR^{e}R^{f}, -CR^{g}(=N)N(R^{g})₂, -NR^{g}C(=N)-N(R^{g})₂, -NR^{g}-C(=NR^{g})R^{g} and (C₁-C₆)alkyl that is optionally substituted with one or more groups independently selected from hydroxy, -NR^{e}R^{f}, -CR^{g}(=N)N(R^{g})₂, -NR^{g}C(=N)-N(R^{g})₂, and -NR^{g}-C(=NR^{g})R^{g};
m is 0, 1, or 2; and
n is 1, 2, 3, or 4.

In one embodiment the invention provides a compound selected from: 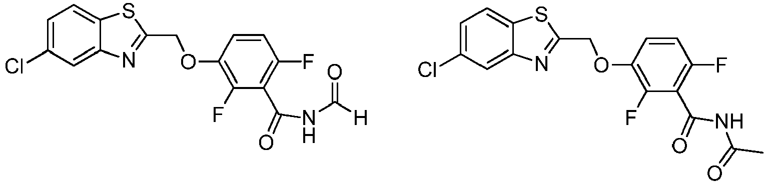 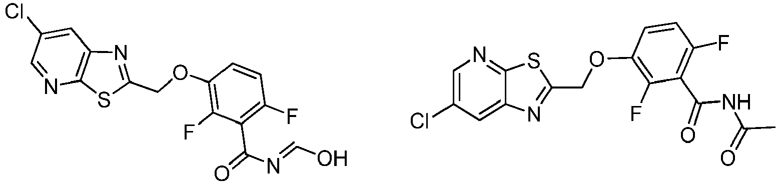 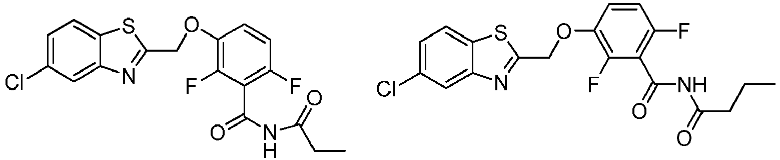 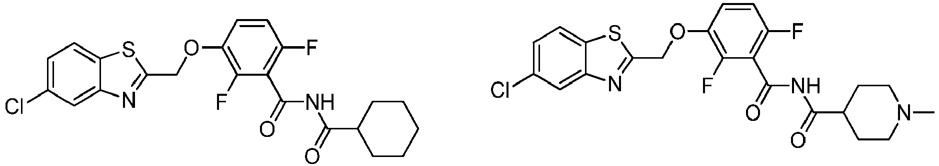 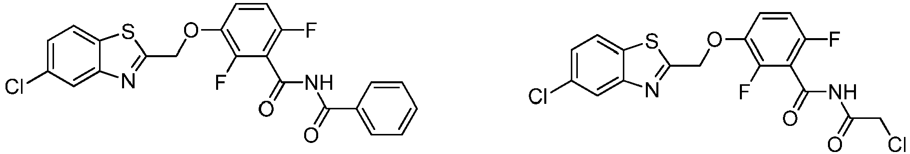 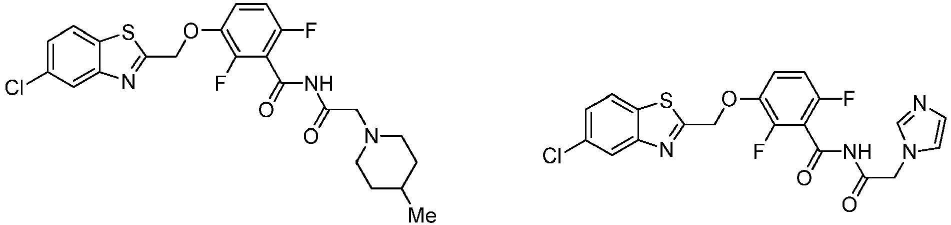 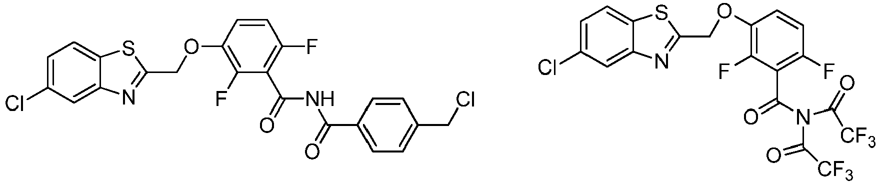 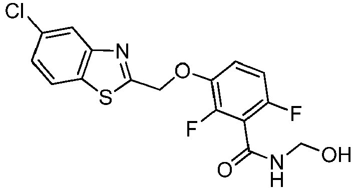 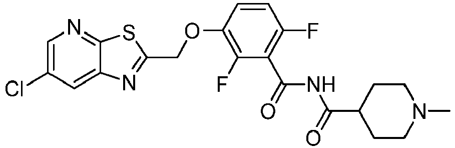 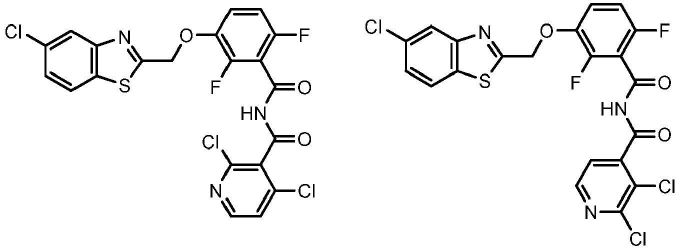 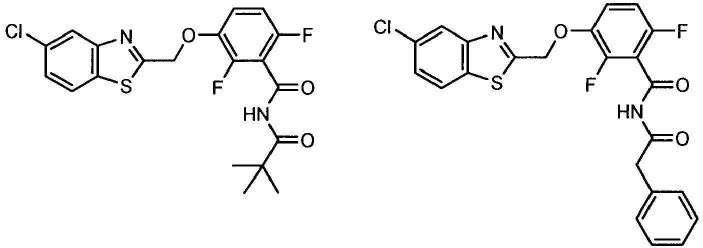 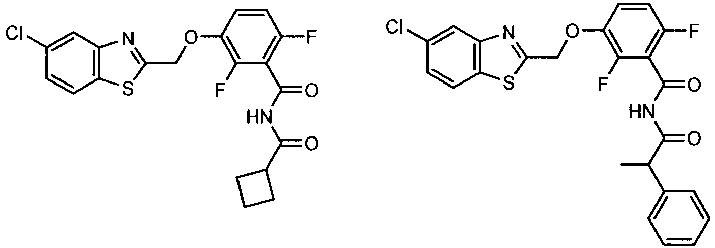 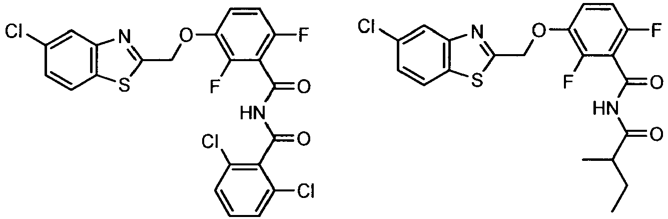 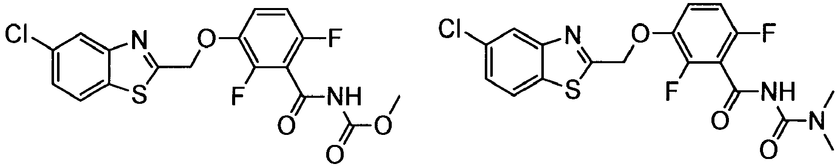 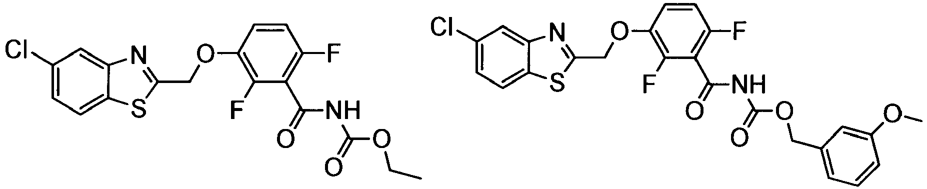 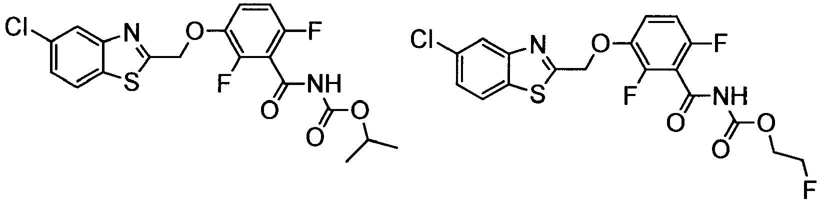 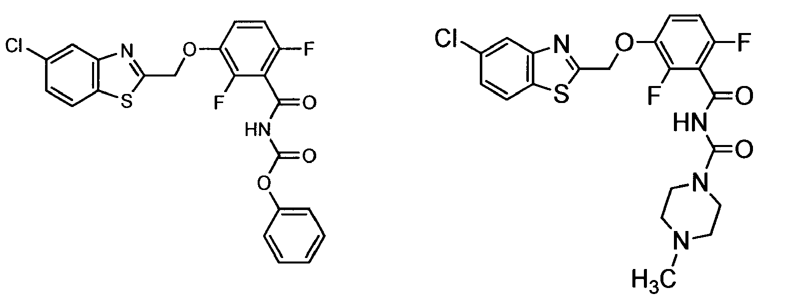 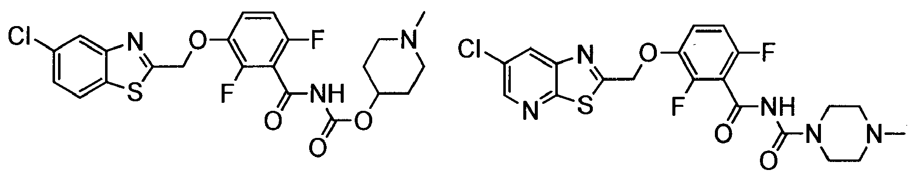 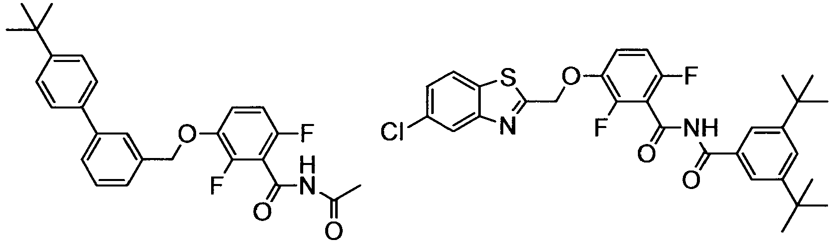 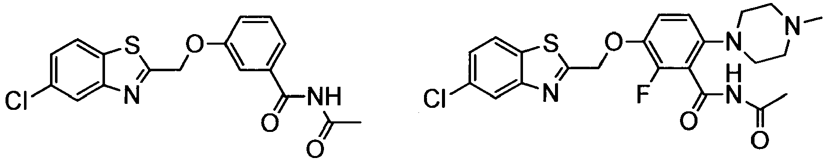 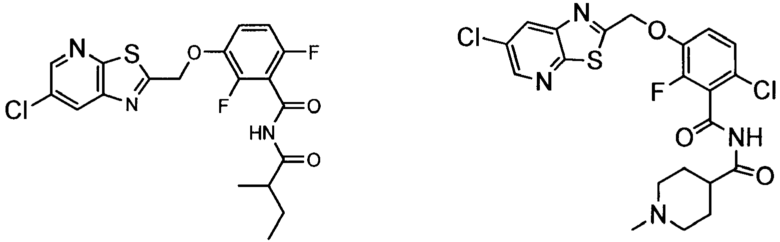 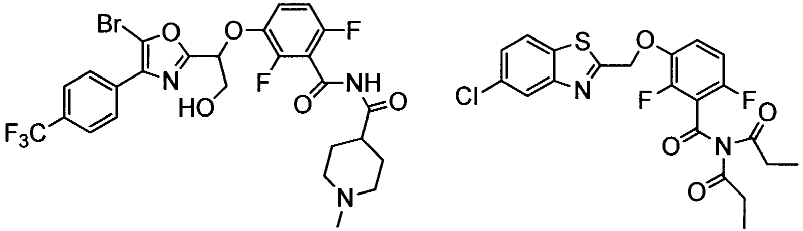 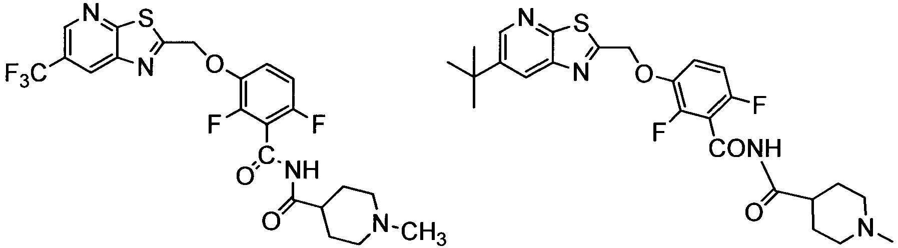 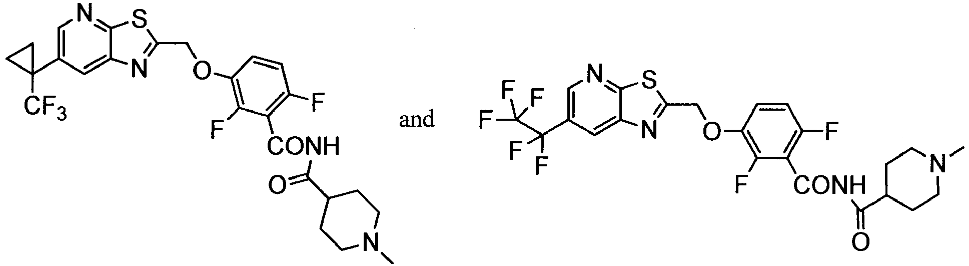 and salts thereof.

In one embodiment the invention provides a compound selected from: 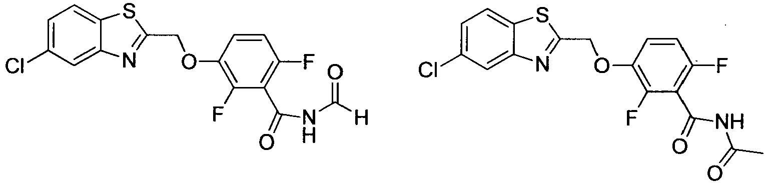 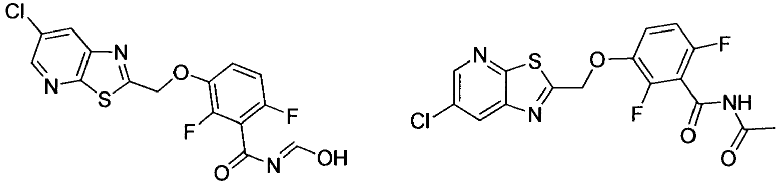 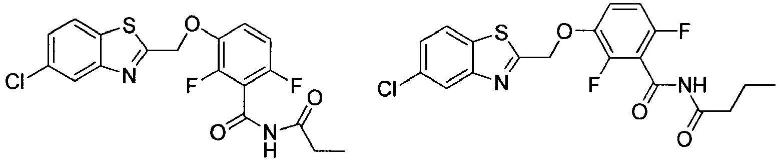 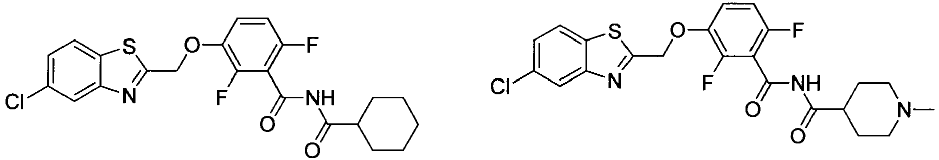 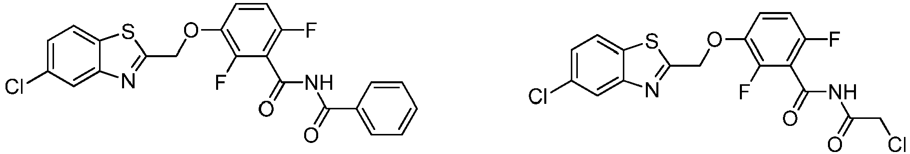 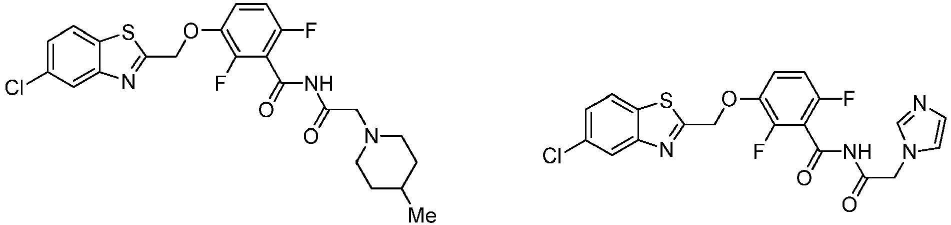 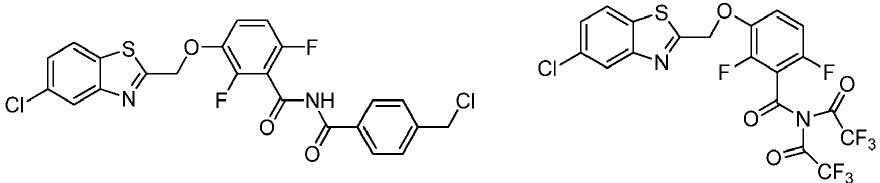 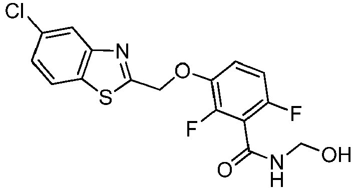 and

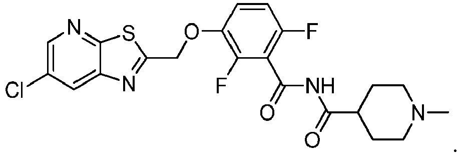

In one embodiment of the invention R³ is aryl, which is optionally substituted with one or more groups independently selected from R^{h}, halo, hydroxy, -NR^{e}R^{f}, -CR^{g}(=N)N(R^{g})₂, -NR^{g}C(=N)-N(R^{g})₂, -NR^{g}-C(=NR^{g})R^{g}, (C₁-C₆)alkyl and (C₃-C₈)cycloalkyl, wherein any (C₁-C₆)alkyl and (C₃-C₈)cycloalkyl is optionally substituted with one or more groups independently selected from halo, hydroxy, -NR^{e}R^{f}, -CR^{g}(=N)N(R^{g})₂, -NR^{g}C(=N)-N(R^{g})₂, -NR^{g}-C(=NR^{g})R^{g}, and (C₁-C₆)alkyl that is optionally substituted with one or more groups independently selected from halo.

In one embodiment of the invention R³ is heteroaryl, which is optionally substituted with one or more groups independently selected from R^{h}, halo, hydroxy, -NR^{e}R^{f}, -CR^{g}(=N)N(R^{g})₂, -NR^{g}C(=N)-N(R^{g})₂, -NR^{g}-C(=NR^{g})R^{g}, (C₁-C₆)alkyl, and (C₃-C₈)cycloalkyl, wherein any (C₁-C₆)alkyl and (C₃-C₈)cycloalkyl is optionally substituted with one or more groups independently selected from halo, hydroxy, -NR^{e}R^{f}, -CR^{g}(=N)N(R^{g})₂, -NR^{g}C(=N)-N(R^{g})₂, -NR^{g}-C(=NR^{g})R^{g}, and (C₁-C₆)alkyl that is optionally substituted with one or more groups independently selected from halo.

In one embodiment of the invention R³ is: 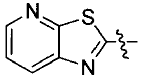 which is optionally substituted with one or more groups independently selected from R^{h}, halo, hydroxy, -NR^{e}R^{f}, -CR^{g}(=N)N(R^{g})₂, -NR^{g}C(=N)-N(R^{g})₂, -NR^{g}-C(=NR^{g})R^{g}, (C₁-C₆)alkyl, and (C₃-C₈)cycloalkyl, wherein any (C₁-C₆)alkyl and (C₃-C₈)cycloalkyl is optionally substituted with one or more groups independently selected from halo, hydroxy, -NR^{e}R^{f}, -CR⁹(=N)N(R^{g})₂, -NR^{g}C(=N)-N(R^{g})₂, -NR^{g}-C(=NR^{g})R^{g}, and (C₁-C₆)alkyl that is optionally substituted with one or more groups independently selected from halo.

In one embodiment of the invention R³ is: 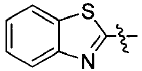 which is optionally substituted with one or more groups independently selected from R^{h}, halo, hydroxy, -NR^{e}R^{f}, -CR^{g}(=N)N(R^{g})₂, -NR^{g}C(=N)-N(R^{g})₂, -NR^{g}-C(=NR^{g})R^{g}, (C₁-C₆)alkyl, and (C₃-C₈)cycloalkyl, wherein any (C₁-C₆)alkyl and (C₃-C₈)cycloalkyl is optionally substituted with one or more groups independently selected from halo, hydroxy, -NR^{e}R^{f}, -CR^{g}(=N)N(R^{g})₂, -NR^{g}C(=N)-N(R^{g})₂, -NR^{g}-C(=NR^{g})R^{g}, and (C₁-C₆)alkyl that is optionally substituted with one or more groups independently selected from halo.

In one embodiment of the invention R³ is: 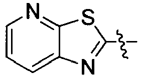 which is optionally substituted with one or more groups independently selected from (C₁-C₆)alkyl, and (C₃-C₈)cycloalkyl, wherein any (C₁-C₆)alkyl and (C₃-C₈)cycloalkyl is optionally substituted with one or more groups independently selected from halo, hydroxy, and (C₁-C₆)alkyl that is optionally substituted with one or more groups independently selected from halo.

In one embodiment of the invention R³ is: 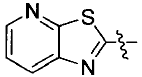 which is substituted with one or more groups independently selected from (C₁-C₆)alkyl, and (C₃-C₈)cycloalkyl, wherein any (C₁-C₆)alkyl and (C₃-C₈)cycloalkyl is optionally substituted with one or more groups independently selected from halo and (C₁-C₆)alkyl that is optionally substituted with one or more groups independently selected from halo.

In one embodiment of the invention R³ is: 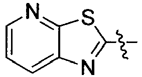 which is substituted with one or more groups independently selected from trifluoromethyl, pentafluoroethyl, or 1-(trifluoromethyl)cyclopropyl.

In one embodiment of the invention R³ is: 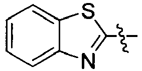 which is optionally substituted with one or more groups independently selected from (C₁₋C₆)alkyl, and (C₃-C₈)cycloalkyl, wherein any (C₁-C₆)alkyl and (C₃-C₈)cycloalkyl is optionally substituted with one or more groups independently selected from halo, hydroxy, and (C₁-C₆)alkyl that is optionally substituted with one or more groups independently selected from halo.

In one embodiment of the invention R³ is: 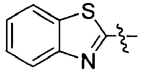 which is substituted with one or more groups independently selected from (C₁-C₆)alkyl, and (C₃-C₈)cycloalkyl, wherein any (C₁-C₆)alkyl and (C₃-C₈)cycloalkyl is optionally substituted with one or more groups independently selected from halo and (C₁-C₆)alkyl that is optionally substituted with one or more groups independently selected from halo.

In one embodiment of the invention R³ is: 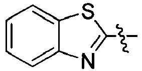 which is substituted with one or more groups independently selected from trifluoromethyl, pentafluoroethyl, or 1-(trifluoromethyl)cyclopropyl.

In one embodiment of the invention R³ is: 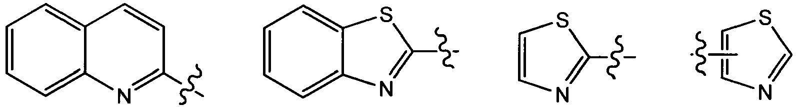 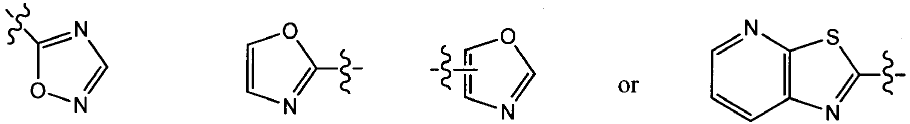 which is optionally substituted with one or more groups independently selected from R^{h}, halo, hydroxy, -NR^{e}R^{f}, -CR^{g}(=N)N(R^{g})₂, -NR^{g}C(=N)-N(R^{g})₂, -NR^{g}-C(=NR^{g})R^{g}, (C₁-C₆)alkyl, and (C₃-C₈)cycloalkyl, wherein any (C₁-C₆)alkyl and (C₃-C₈)cycloalkyl is optionally substituted with one or more groups independently selected from halo, hydroxy, -NR^{e}R^{f}, -CR^{g}(=N)N(R^{g})₂, -NR^{g}C(=N)-N(R^{g})₂, -NR^{g}-C(=NR^{g})R^{g}, and (C₁-C₆)alkyl that is optionally substituted with one or more groups independently selected from halo.

In one embodiment of the invention R³ is: 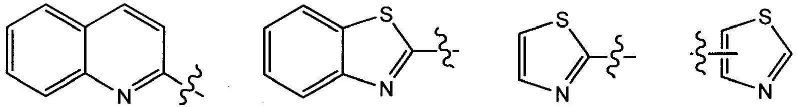 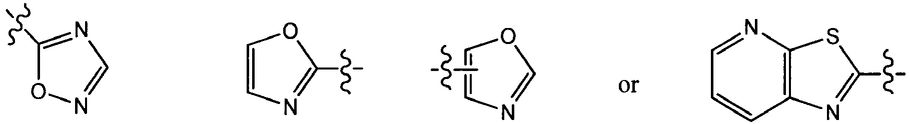 which is optionally substituted with one or more groups independently selected from (C₁-C₆)alkyl, and (C₃-C₈)cycloalkyl, wherein any (C₁-C₆)alkyl and (C₃-C₈)cycloalkyl is optionally substituted with one or more groups independently selected from halo, hydroxy, and (C₁-C₆)alkyl that is optionally substituted with one or more groups independently selected from halo.

In one embodiment of the invention R³ is: 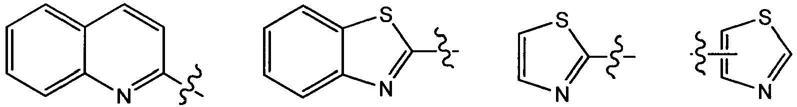 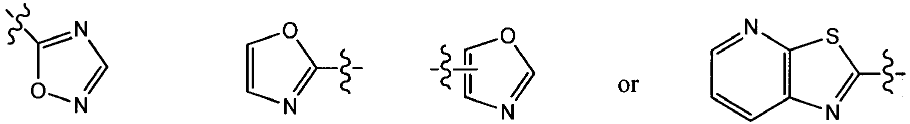 which is optionally substituted with one or more groups independently selected from trifluoromethyl, pentafluoroethyl, or 1-(trifluoromethyl)cyclopropyl.

In one embodiment of the invention: 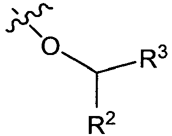 is selected from:

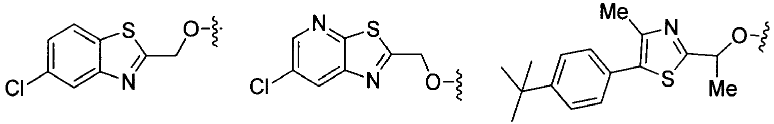

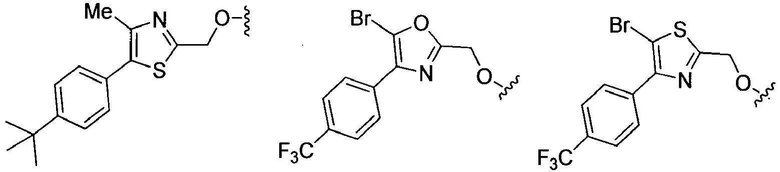

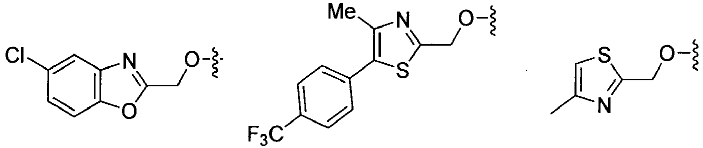

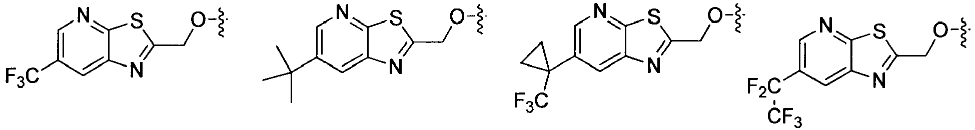

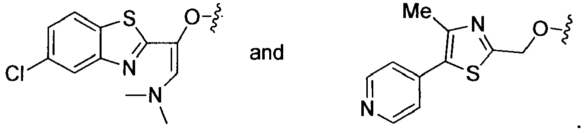

In one embodiment of the invention the compound is not: 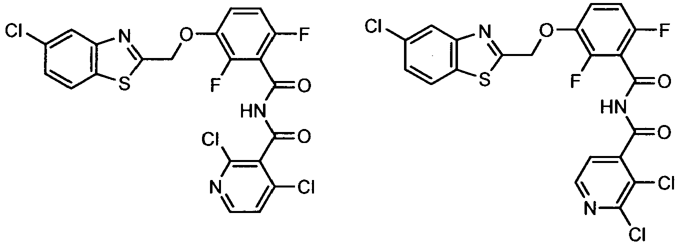 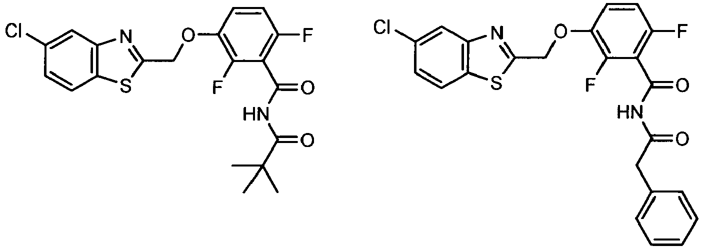 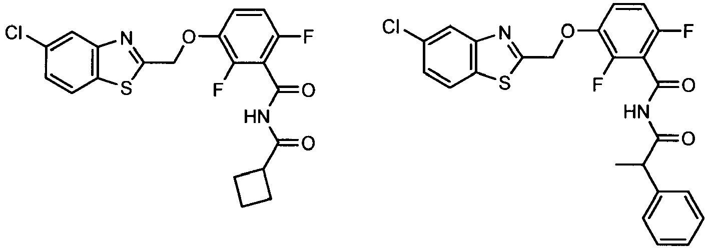 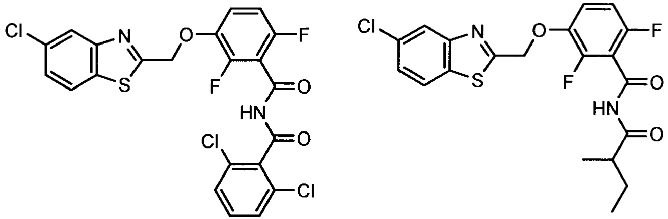 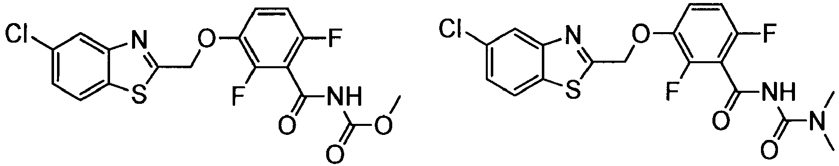 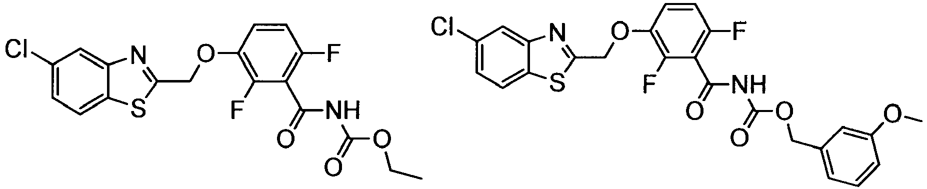 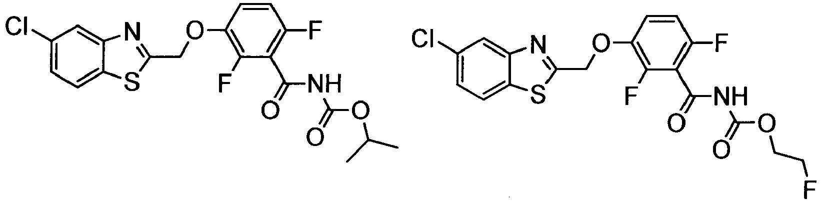 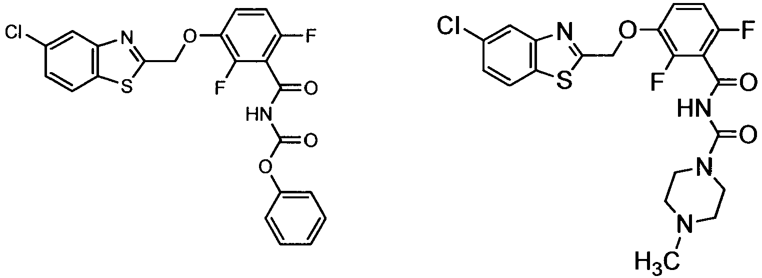 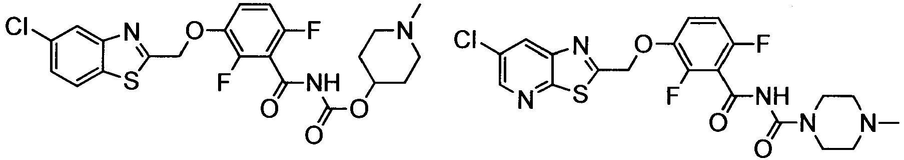 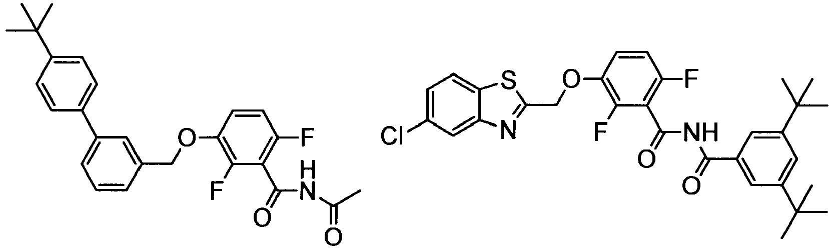 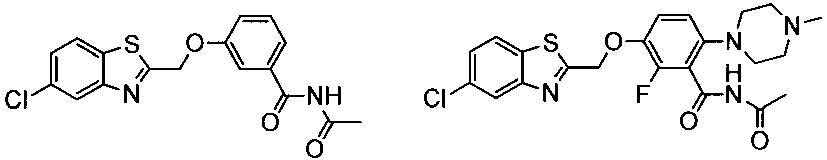 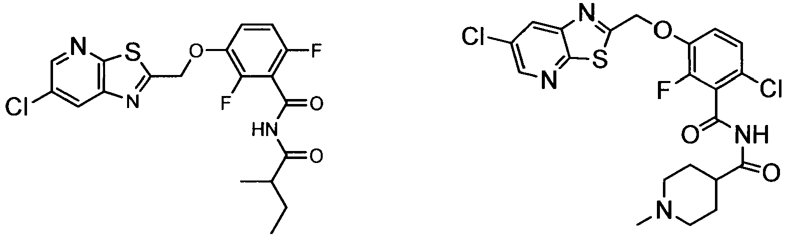 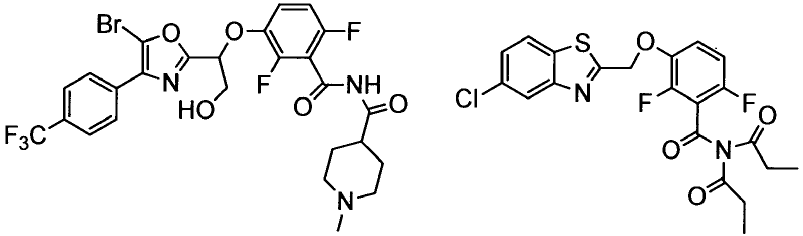 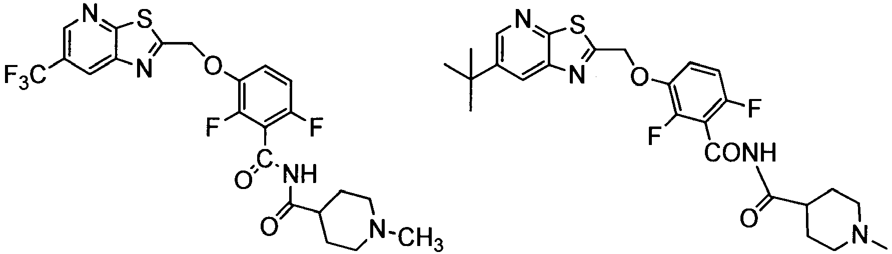 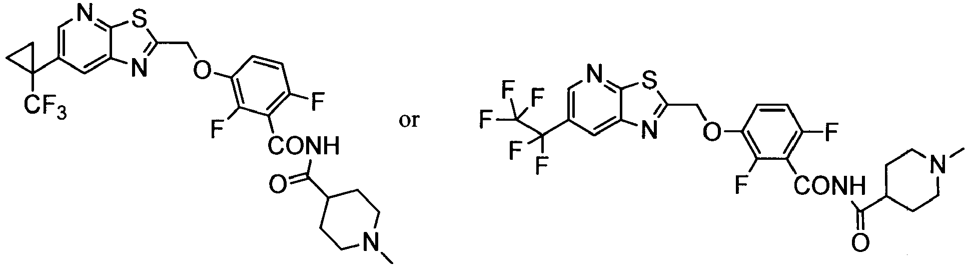 or a salt thereof.

In one embodiment of the invention the compound is not: 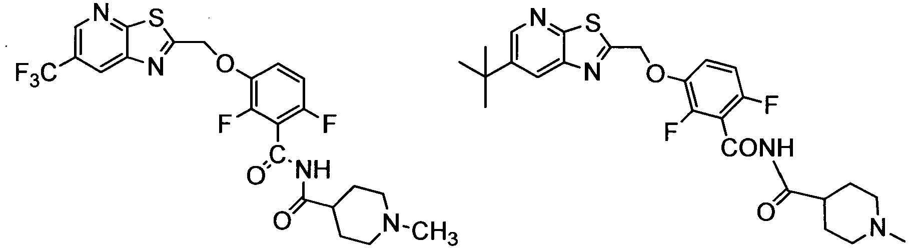 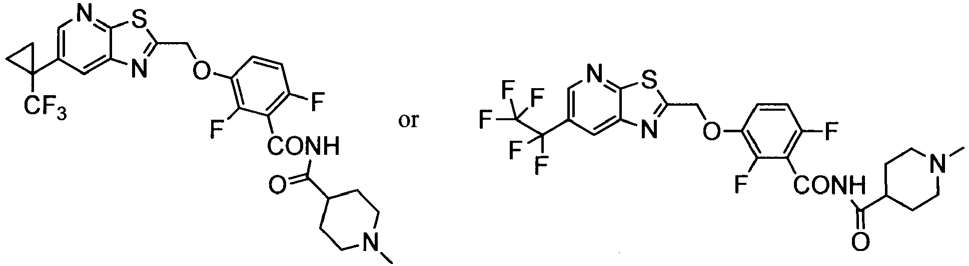 or a salt thereof.

In one embodiment of the invention the compound is not: 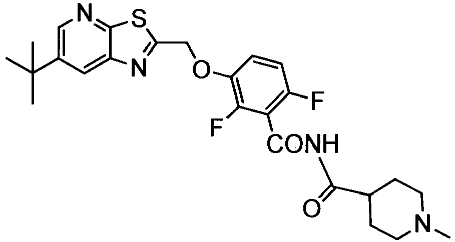 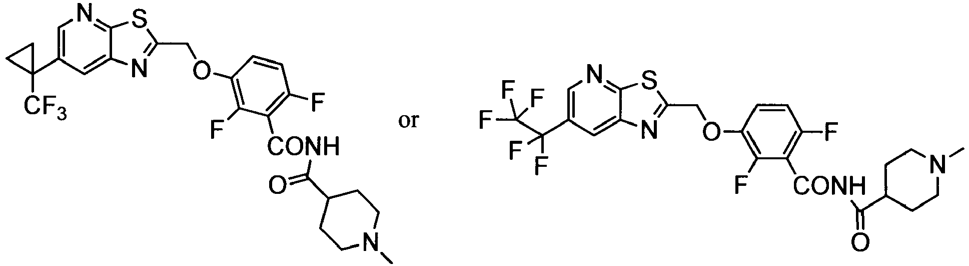 or a salt thereof.

In one embodiment of the invention the compound is not: 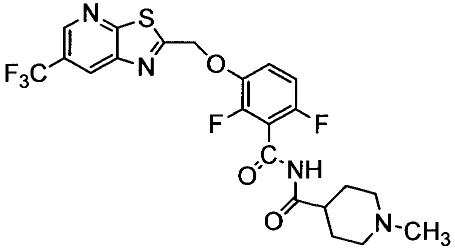 or a salt thereof.

In one embodiment the invention provides a compound selected from compounds of formula I and salts thereof having a minimal inhibitory concentration against MSSA of less than about 8 µg/ml (see Test C below).

In one embodiment the invention provides a compound selected from compounds of formula I and salts thereof having a minimal inhibitory concentration against MSSA of less than about 4 µg/ml.

In one embodiment the invention provides a compound selected from compounds of formula I and salts thereof having a minimal inhibitory concentration against MSSA of less than about 2 µg/ml.

In one embodiment the invention provides a compound selected from compounds of formula I and salts thereof having a minimal inhibitory concentration against MSSA of less than about 1 µg/ml.

In one embodiment the invention provides a compound selected from compounds of formula I and salts thereof having a minimal inhibitory concentration against MSSA of less than about 0.5 µg/ml.

In one embodiment the invention provides a compound of formula I or a salt thereof that increases the survival percentage by at least 25% at 72 hours when administered at a non-lethal dose against MSSA in Test D below.

In one embodiment the invention provides a compound of formula I or a salt thereof that increases the survival percentage by at least 50% at 72 hours when administered at a non-lethal dose against MSSA in Test D below.

In one embodiment the invention provides a compound of formula I or a salt thereof that increases the survival percentage by at least 75% at 72 hours when administered at a non-lethal dose against MSSA in Test D below.

Generally, compounds of formula I as well as synthetic intermediates that can be used for preparing compounds of formula I can be prepared as illustrated in the following General

Methods and Schemes. It is understood that variable groups shown below (e.g. R¹, R², and R³) can represent the final corresponding groups present in a compound of formula I or that these groups can represent groups that can be converted to the final corresponding groups present in a compound of formula I at a convenient point in a synthetic sequence. For example, the variable groups can contain one or more protecting groups that can be removed at a convenient point in a synthetic sequence to provide the final corresponding groups in the compound of formula I.

### General Method for N'-substituted N-(2-aminoacetyl)amides and 2-substituted N-(acyl)amides.

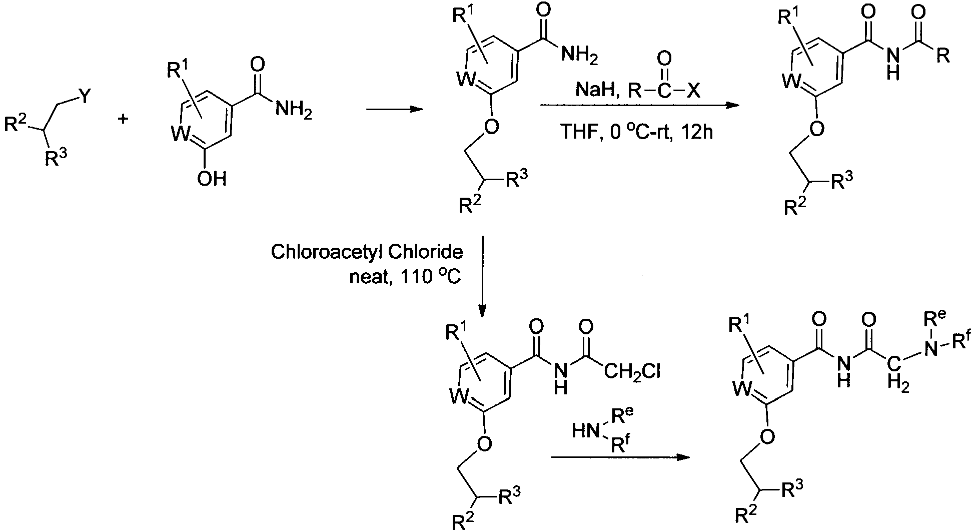

Reaction of the benzamide with an activated acylating agents, such as an acid chloride, anhydride, or mixed anhydride will provide varied N-(acetyl)amides. Alternatively, the use of chloroacetyl chloride provides the N-(2-chloroacetyl)amide, which can be treated with a variety of primary and secondary amines to give various N'subsituted N-(2-aminoacetyl)amides.

### General Method for the preparation of substituted N-(1-aminomethylidene)benzamides.

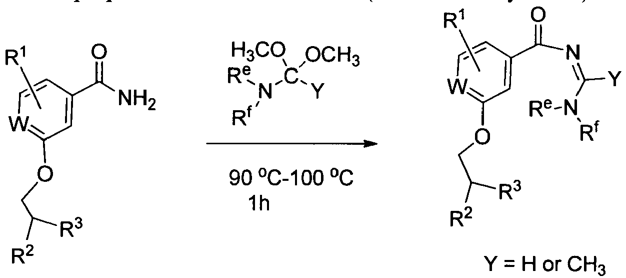

Treatment of the requisite benzamide intermediate with amide acetals, such as N,N-dimethylformamide dimethoxy acetal or N,N-dimethylacetamide dimethoxy acetal, provides the desired N-(1-aminomethylidene)benzamide derivatives.

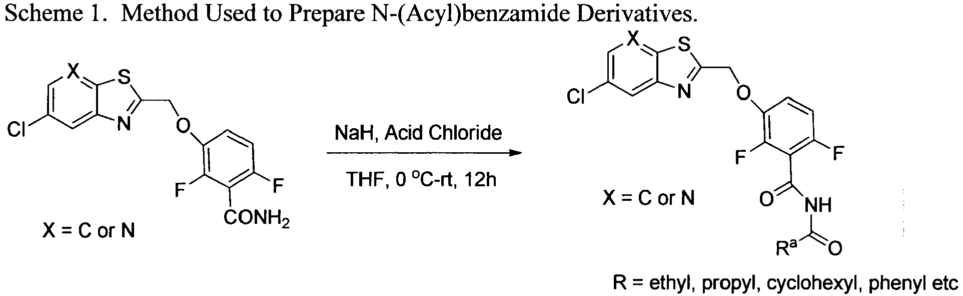

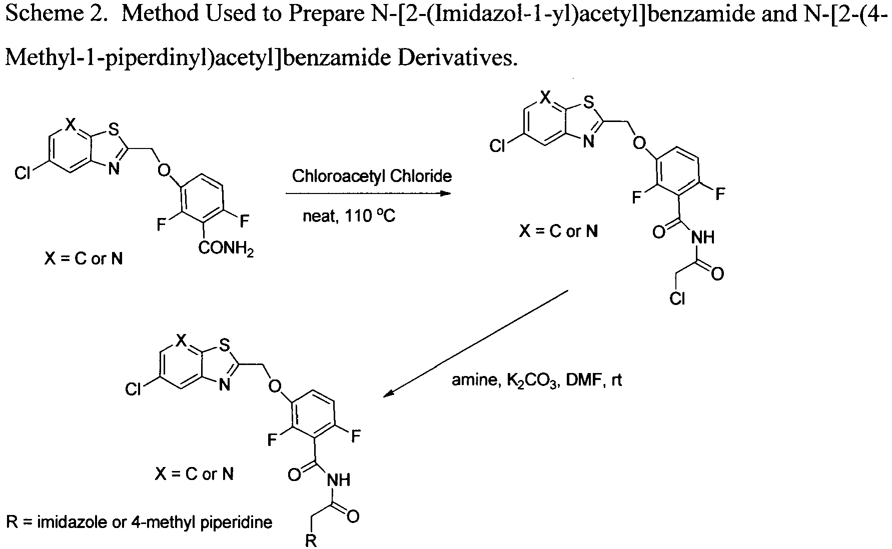

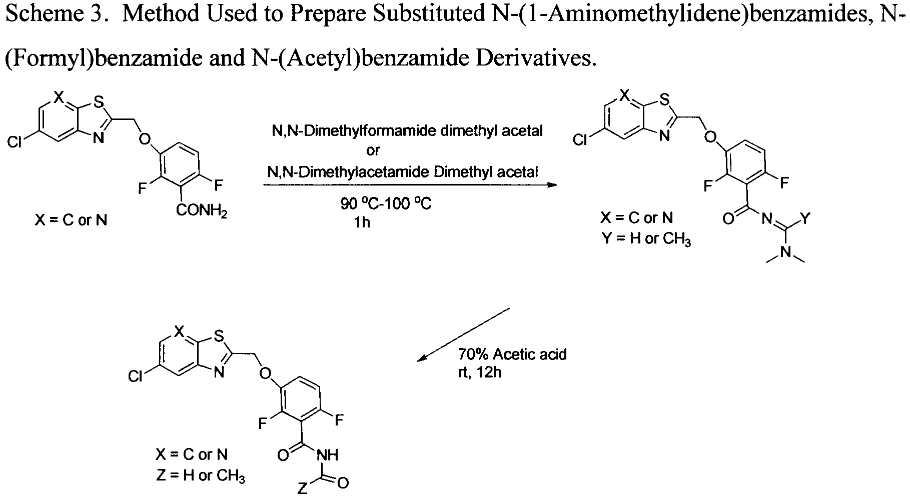

Scheme 4. Methods for the preparation of *t*-butyl-, 1-(trifluoromethyl)cyclopropyl-, and pentafluoroethyl- substituted analogs from bromo or iodo thiazolo[5,4-b]pyridine.

### a) Preparation of t-butyl substituted derivatives

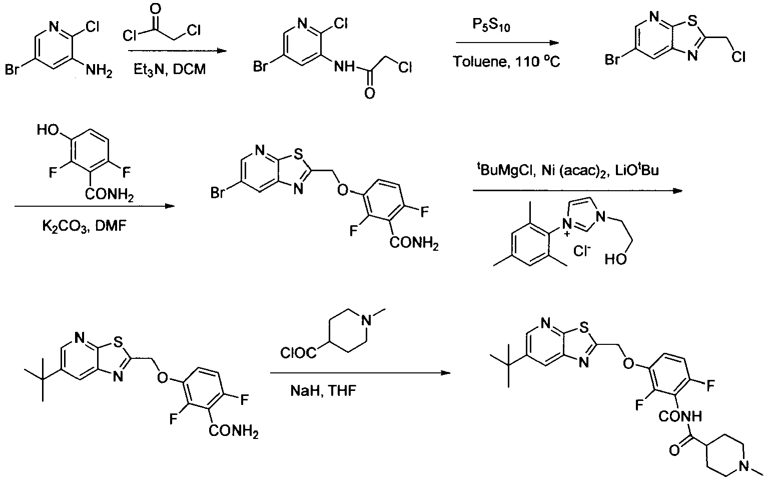

### b) Preparation of 1-(trifluoromethyl)cyclopropyl substituted derivatives

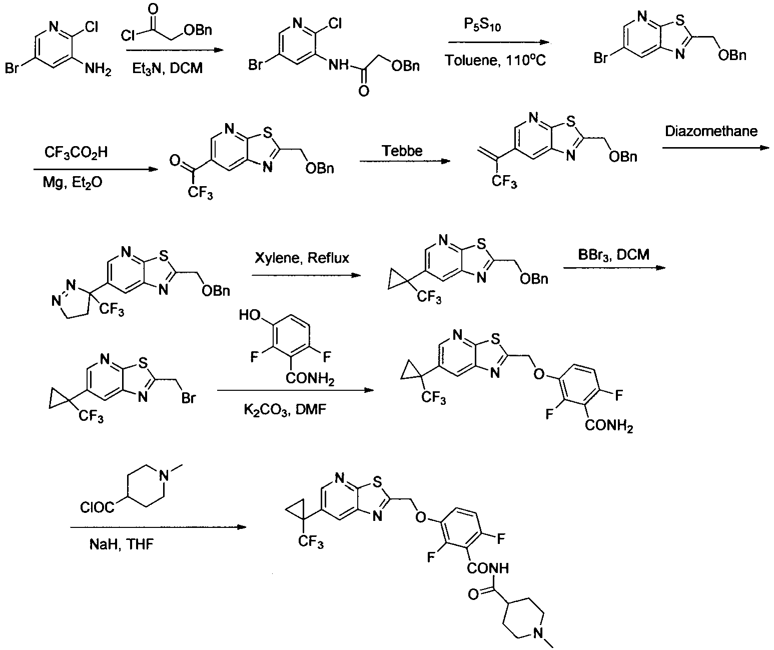

### c) Preparation of pentafluoroethyl substituted derivatives.

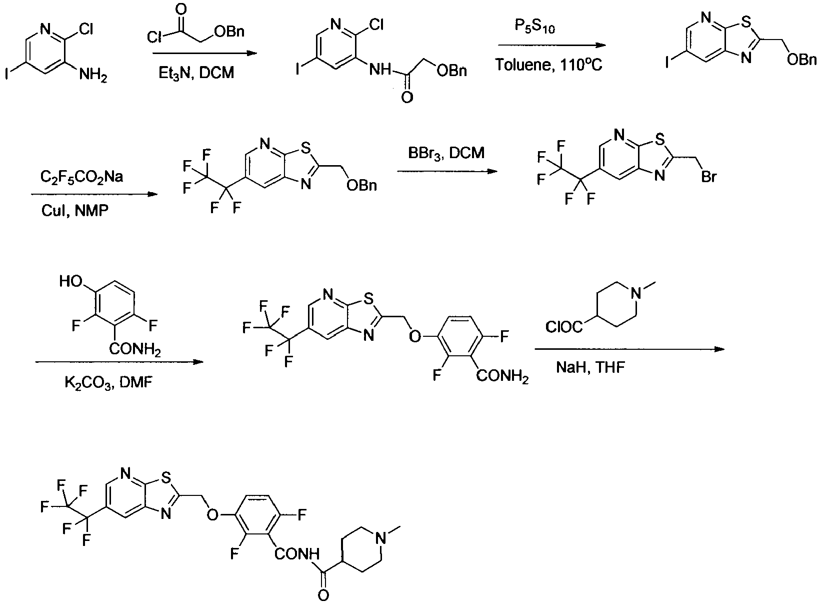

The compounds of the present invention inhibit bacterial Z-ring formation, which is essential for cytokinesis. Since the Z-ring serves as the scaffold for recruitment of all other proteins that comprise the divisome complex, inhibition of Z-ring formation by the compounds of the present invention also results in a corresponding inhibition of divisome protein recruitment.

The compounds of the invention are useful to treat bacterial infections including infections by Gram-positive and Gram-negative bacterial strains, and multiple drug-resistant bacterial strains. For treatment of Gram-negative bacterial strains as well as Gram-positive bacterial strains, the compounds of the invention may be administered in combination with an efflux pump inhibitor to enhance antibacterial activity. See Lomovskaya, O., et al., Nature Reviews (Drug Discovery), 2007, 6, 56-65; and Handzlik, J. et al., Antibiotics, 2013, 2, 28-45.

In one embodiment compounds of the present invention may be administered as a composition used to treat and/or prevent a bacterial infection wherein the bacterial cell uses polymerized FtsZ protein, or a homolog thereof, to facilitate cytokinesis. To this end, compounds of the present invention may be administered to treat Staph Infections, Tuberculosis, Urinary Tract Infections, Meningitis, Enteric Infections, Wound Infections, Acne, Encephalitis, Skin Ulcers, Bed Sores, Gastric and Duodenal Ulcers, Eczema, Periodontal disease, Gingivitis, Halitosis, Anthrax, Tularemia, Endocarditis, Prostatitis, Osteomyelitis, Lyme Disease, Pneumonia, or the like.

The compositions can, if desired, also contain other active therapeutic agents, such as a narcotic, a non-steroid anti-inflammatory drug (NSAID), an analgesic, an anesthetic, a sedative, a local anesthetic, a neuromuscular blocker, an anti-cancer, other antimicrobial (for example, an aminoglycoside, an antifungal, an antiparasitic, an antiviral, a carbapenem, a cephalosporin, a flurorquinolone, a macrolide, a penicillin, a sulfonamide, a tetracycline, another antimicrobial), an anti-psoriatic, a corticosteriod, an anabolic steroid, a diabetes-related agent, a mineral, a nutritional, a thyroid agent, a vitamin, a calcium-related hormone, an antidiarrheal, an anti-tussive, an anti-emetic, an anti-ulcer, a laxative, an anticoagulant, an erythropieitin (for example, epoetin alpha), a filgrastim (for example, G-CSF, Neupogen), a sargramostim (GM-CSF, Leukine), an immunization, an immunoglobulin, an immunosuppressive (for example, basiliximab, cyclosporine, daclizumab), a growth hormone, a hormone replacement drug, an estrogen receptor modulator, a mydriatic, a cycloplegic, an alkylating agent, an anti-metabolite, a mitotic inhibitor, a radiopharmaceutical, an antidepressant, an anti-manic agent, an anti-psychotic, an anxiolytic, a hypnotic, a sympathomimetic, a stimulant, donepezil, tacrine, an asthma medication, a beta agonist, an inhaled steroid, a leukotriene inhibitor, a methylxanthine, a cromolyn, an epinephrine or analog thereof, dornase alpha (Pulmozyme), a cytokine, or any combination thereof.

It will be appreciated that compounds of the invention having a chiral center may exist in and be isolated in optically active and racemic forms. Some compounds may exhibit polymorphism. It is to be understood that the present invention encompasses any racemic, optically-active, polymorphic, or stereoisomeric form, or mixtures thereof, of a compound of the invention, which possess the useful properties described herein, it being well known in the art how to prepare optically active forms (for example, by resolution of the racemic form by recrystallization techniques, by synthesis from optically-active starting materials, by chiral synthesis, or by chromatographic separation using a chiral stationary phase.

When a bond in a compound formula herein is drawn in a non-stereochemical manner (e.g. flat), the atom to which the bond is attached includes all stereochemical possibilities. When a bond in a compound formula herein is drawn in a defined stereochemical manner (e.g. bold, bold-wedge, dashed or dashed-wedge), it is to be understood that the atom to which the stereochemical bond is attached is enriched in the absolute stereoisomer depicted unless otherwise noted. In one embodiment, the compound may be at least 51% the absolute stereoisomer depicted. In another embodiment, the compound may be at least 60% the absolute stereoisomer depicted. In another embodiment, the compound may be at least 80% the absolute stereoisomer depicted. In another embodiment, the compound may be at least 90% the absolute stereoisomer depicted. In another embodiment, the compound may be at least 95 the absolute stereoisomer depicted. In another embodiment, the compound may be at least 99% the absolute stereoisomer depicted.

It will also be appreciated by those skilled in the art that certain compounds of the invention can exist in more than one tautomeric form. For example, a substituent of formula -NH-C(=O)H in a compound of formula (I) could exist in tautomeric form as -N=C(OH)H. The present invention encompasses all tautomeric forms of a compound of formula I as well as mixtures thereof that can exist in equilibrium with non-charged and charged entities depending upon pH, which possess the useful properties described herein.

In cases where compounds are sufficiently basic or acidic, a salt of a compound of formula I can be useful as an intermediate for isolating or purifying a compound of formula I. Additionally, administration of a compound of formula I as a pharmaceutically acceptable acid or base salt may be appropriate. Examples of pharmaceutically acceptable salts are organic acid addition salts formed with acids which form a physiological acceptable anion, for example, tosylate, methanesulfonate, acetate, citrate, malonate, tartrate, succinate, fumarate, benzoate, ascorbate, α-ketoglutarate, and α-glycerophosphate. Suitable inorganic salts may also be formed, including hydrochloride, sulfate, nitrate, bicarbonate, and carbonate salts. Salts may be obtained using standard procedures well known in the art, for example by reacting a sufficiently basic compound such as an amine with a suitable acid affording the corresponding anion. Alkali metal (for example, sodium, potassium or lithium) or alkaline earth metal (for example calcium) salts of carboxylic acids can also be made.

Pharmaceutically suitable counterions include pharmaceutically suitable cations and pharmaceutically suitable anions that are well known in the art. Examples of pharmaceutically suitable anions include, but are not limited to those described above (e.g. physiologically acceptable anions) including Cl⁻, Br⁻, I⁻, CH₃SO₃⁻, H₂PO₄⁻, CF₃SO₃⁻, *p*-CH₃C₆H₄ SO₃⁻, citrate, tartrate, phosphate, malate, fumarate, formate, or acetate.

It will be appreciated by those skilled in the art that a compound of the invention comprising a counterion can be converted to a compound of the invention comprising a different counterion. Such a conversion can be accomplished using a variety of well known techniques and materials including but not limited to ion exchange resins, ion exchange chromatography and selective crystallization.

The compounds of formula I can be formulated as pharmaceutical compositions and administered to a mammalian host, such as a human patient in a variety of forms adapted to the chosen route of administration, i.e., orally or parenterally, by intravenous, intramuscular, topical or subcutaneous routes. For oral administration the compounds can be formulated as a solid dosage form with or without an enteric coating.

Thus, the present compounds may be systemically administered, e.g., orally, in combination with a pharmaceutically acceptable vehicle such as an inert diluent, excipient or an assimilable edible carrier. They may be enclosed in hard or soft shell gelatin capsules, may be compressed into tablets, or may be incorporated directly with the food of the patient's diet. For oral therapeutic administration, the active compound may be combined with one or more excipients and used in the form of ingestible tablets, buccal tablets, troches, capsules, elixirs, suspensions, syrups, wafers, and the like. Such compositions and preparations should contain at least 0.1% of active compound. The percentage of the compositions and preparations may, of course, be varied and may conveniently be between about 2 to about 90% of the weight of a given unit dosage form. The amount of active compound in such therapeutically useful compositions is such that an effective dosage level will be obtained.

The tablets, troches, pills, capsules, and the like may also contain the following: binders such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, fructose, lactose or aspartame or a flavoring agent such as peppermint, oil of wintergreen, or cherry flavoring may be added. When the unit dosage form is a capsule, it may contain, in addition to materials of the above type, a liquid carrier, such as a vegetable oil or a polyethylene glycol. Various other materials may be present as coatings or to otherwise modify the physical form of the solid unit dosage form. For instance, tablets, pills, or capsules may be coated with gelatin, wax, shellac or sugar and the like. A syrup or elixir may contain the active compound, sucrose or fructose as a sweetening agent, methyl and propylparabens as preservatives, a dye and flavoring such as cherry or orange flavor. Of course, any material used in preparing any unit dosage form should be pharmaceutically acceptable and substantially non-toxic in the amounts employed. In addition, the active compound may be incorporated into sustained-release preparations, particles, and devices.

The active compound may also be administered intravenously or intramuscularly by infusion or injection. Solutions of the active compound or its salts can be prepared in water, optionally mixed with a nontoxic surfactant. Dispersions can also be prepared in glycerol, liquid polyethylene glycols, triacetin, and mixtures thereof and in oils. Under ordinary conditions of storage and use, these preparations contain a preservative to prevent the growth of microorganisms.

The pharmaceutical dosage forms suitable for injection or infusion can include sterile aqueous solutions or dispersions or sterile powders comprising the active ingredient which are adapted for the extemporaneous preparation of sterile injectable or infusible solutions or dispersions, optionally encapsulated in liposomes. In all cases, the ultimate dosage form should be sterile, fluid and stable under the conditions of manufacture and storage. The liquid carrier or vehicle can be a solvent or liquid dispersion medium comprising, for example, water, ethanol, a polyol (for example, glycerol, propylene glycol, liquid polyethylene glycols, and the like), vegetable oils, nontoxic glyceryl esters, and suitable mixtures thereof. The proper fluidity can be maintained, for example, by the formation of liposomes, by the maintenance of the required particle size in the case of dispersions or by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars, buffers or sodium chloride. Prolonged absorption of the injectable compositions can be brought about by the use in the compositions of agents delaying absorption, for example, aluminum monostearate and gelatin.

Sterile injectable solutions are prepared by incorporating the active compound in the required amount in the appropriate solvent with various of the other ingredients enumerated above, as required, followed by filter sterilization. In the case of sterile powders for the preparation of sterile injectable solutions, the preferred methods of preparation are vacuum drying and the freeze drying techniques, which yield a powder of the active ingredient plus any additional desired ingredient present in the previously sterile-filtered solutions.

For topical administration, the present compounds may be applied in pure form, i.e., when they are liquids. However, it will generally be desirable to administer them to the skin as compositions or formulations, in combination with a dermatologically acceptable carrier, which may be a solid or a liquid.

Useful solid carriers include finely divided solids such as talc, clay, microcrystalline cellulose, silica, alumina, nanoparticles, and the like. Useful liquid carriers include water, alcohols or glycols or water-alcohol/glycol blends, in which the present compounds can be dissolved or dispersed at effective levels, optionally with the aid of non-toxic surfactants. Adjuvants such as fragrances and additional antimicrobial agents can be added to optimize the properties for a given use. The resultant liquid compositions can be applied from absorbent pads, used to impregnate bandages and other dressings, or sprayed onto the affected area using pump-type or aerosol sprayers.

Thickeners such as synthetic polymers, fatty acids, fatty acid salts and esters, fatty alcohols, modified celluloses or modified mineral materials can also be employed with liquid carriers to form spreadable pastes, gels, ointments, soaps, and the like, for application directly to the skin of the user.

Useful dosages of the compounds of formula I can be determined by comparing their *in vitro* activity, and *in vivo* activity in animal models. Methods for the extrapolation of effective dosages in mice, and other animals, to humans are known to the art; for example, see U.S. Pat. No. 4,938,949.

The amount of the compound, or an active salt or derivative thereof, required for use in treatment will vary not only with the particular salt selected but also with the route of administration, the nature of the condition being treated and the age and condition of the patient and will be ultimately at the discretion of the attendant physician or clinician.

In general, however, a suitable dose will be in the range of from about 0.1 to about 500 mg/kg, e.g., from about 0.5 to about 400 mg/kg of body weight per day, such as 1 to about 250 mg per kilogram body weight of the recipient per day.

The compound is conveniently formulated in unit dosage form; for example, containing 0.5 to 500 mg, 1 to 400 mg, or 0.5 to 100 mg of active ingredient per unit dosage form. In one embodiment, the invention provides a composition comprising a compound of the invention formulated in such a unit dosage form.

The desired dose may conveniently be presented in a single dose or as divided doses administered at appropriate intervals, for example, as two, three, four or more sub-doses per day. The sub-dose itself may be further divided, e.g., into a number of discrete loosely spaced administrations.

The antibacterial activity of a compound of the invention can be determined using a method like Test A described below.

### Test A. Antibacterial Assay.

Antibacterial activity can be determined as per Clinical and Laboratory Standards Institute (CLSI) guidelines using a broth microdilution assay in which log-phase bacteria are grown at 37 °C in appropriate medium containing two-fold serial dilutions of a compound to yield final concentrations ranging from 256 to 0.06 µg/mL. For determination of minimal inhibitory concentration (MIC) values, bacterial growth is monitored after 24 to 48 hours by measuring optical density at 600 nm. MIC values reflect the minimal compound concentrations at which bacterial growth is completely inhibited. Data for representative compounds of the invention are shown in Table 1.

**Table 1. Minimal Inhibitory Concentrations against MSSA for representative compounds of the Invention**

| **Example Number** | **Drug Structure** | **MIC MSSA µg/mL** | **MIC MRS**A **µg/mL** |
|---|---|---|---|
| **1** | 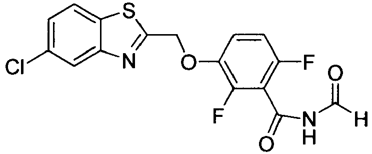 | 0.25 | 0.125 |
| **1b** | 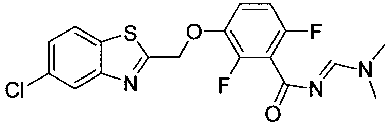 | 0.125 | 0.125 |
| **2** | 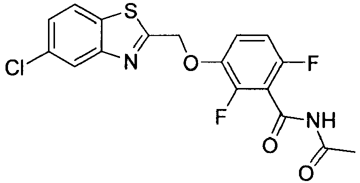 | 2.0 | 4.0 |
| **2a** | 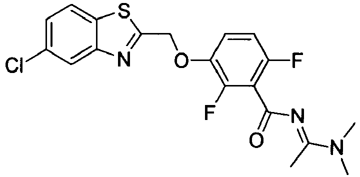 | 2.0 | 4.0 |
| **3** | 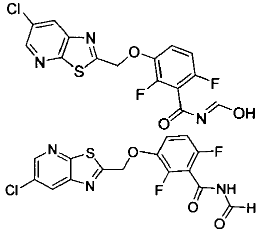 | 0.5 | 0.5 |
| **3b** | 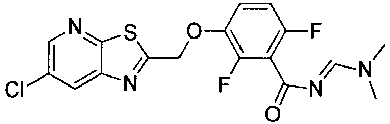 | 0.5 | 1.0 |
| **4** | 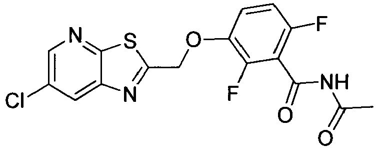 | 2.0* | - |
| **4a** | 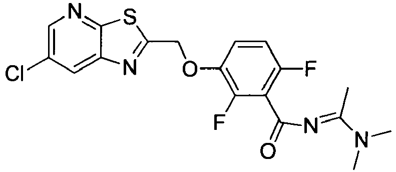 | 4.0 | 4.0 |
| **5** | 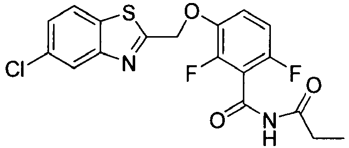 | 2.0** | 2.0** |
| **6** | 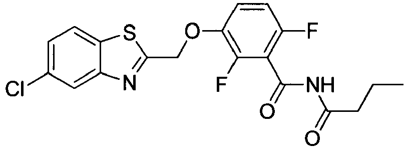 | 0.5** | 1.0** |
| **7** | 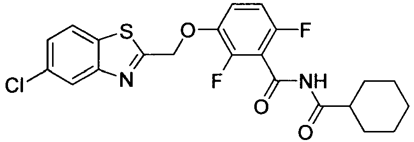 | 0.5** | - |
| **8** | 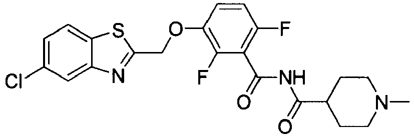 | 0.5 | 0.5 |
| **9** | 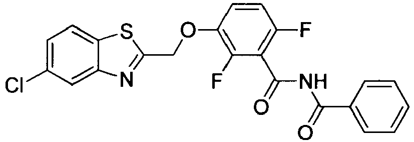 | 0.5** | - |
| **10** | 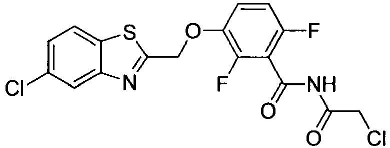 | 0.125 | 0.25 |
| **11** | 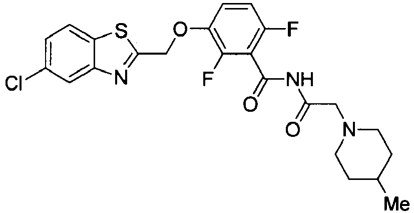 | 0.5 | 1.0 |
| **12** | 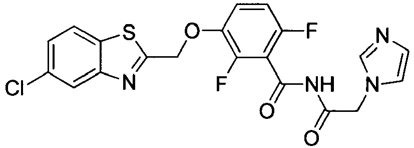 | 0.125 | 0.25 |
| **13** | 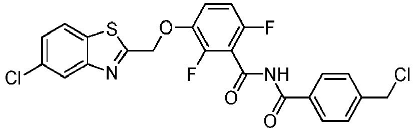 | 4.0* | - |
| **14** | 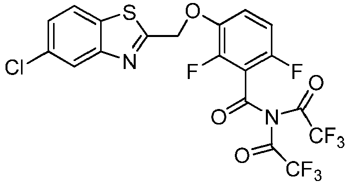 | 2.0* | - |
| **15 (reference example)** | 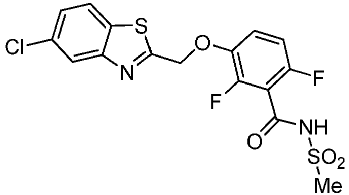 | 8.0 | 8.0 |
| **16** | 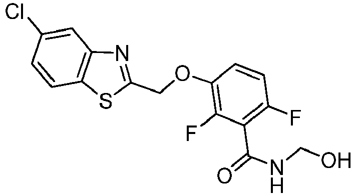 | 2.0 | 2.0 |
| **17** | 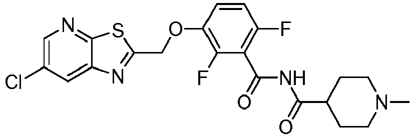 | 2.0 | 4.0 |
| **18** | 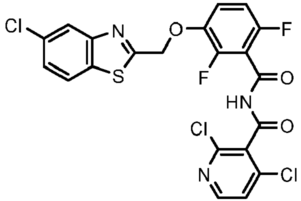 | >64.0 | >64.0 |
| **19** | 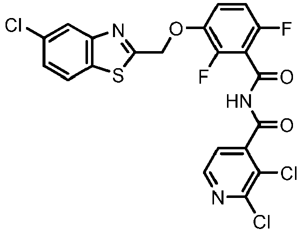 | >64.0 | 4.0 |
| **20** | 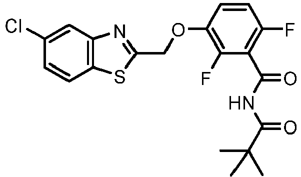 | >64.0 | 4.0 |
| **21** | 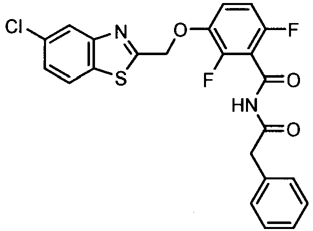 | >64.0 | 2.0 |
| **22** | 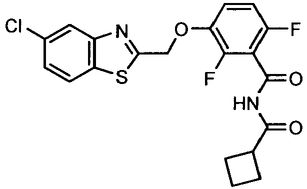 | 0.5 | 2.0 |
| **23** | 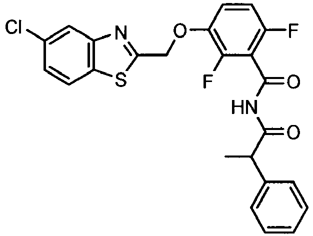 | >64.0 | 2.0 |
| **24** | 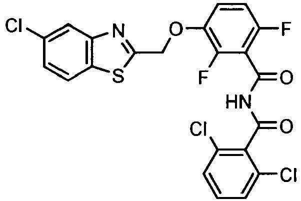 | >64.0 | >64.0 |
| **25** | 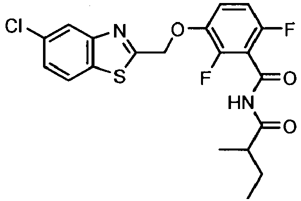 | >64.0 | 2.0 |
| **26** | 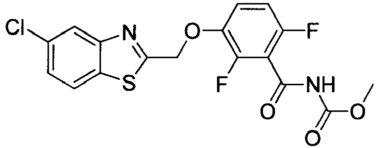 | >64.0 | 8.0 |
| **27** | 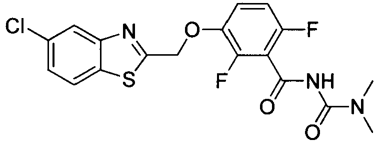 | >64.0 | >64.0 |
| **28** | 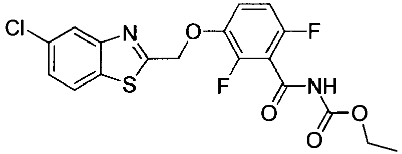 | >64.0 | 8.0 |
| **29** | 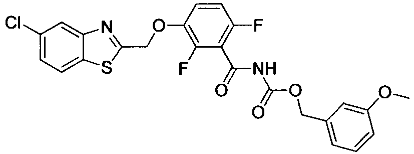 | 8.0 | 32 |
| **30** | 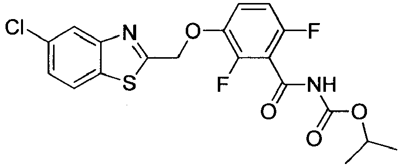 | >64.0 | 2.0 |
| **31** | 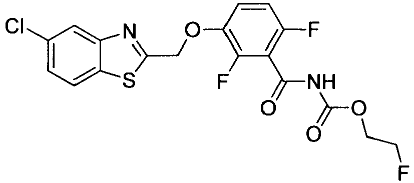 | >64.0 | 4.0 |
| **32** | 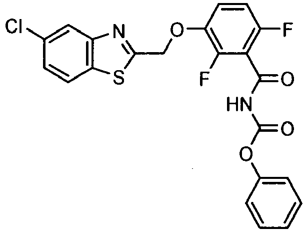 | 4.0 | 4.0 |
| **33** | 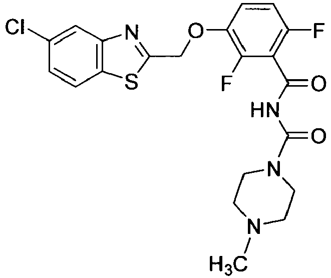 | >64.0 | >64.0 |
| **34** | 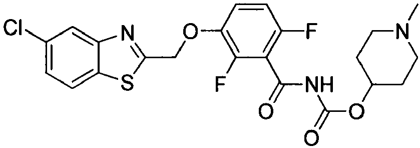 | 32.0 | 4.0 |
| **35** | 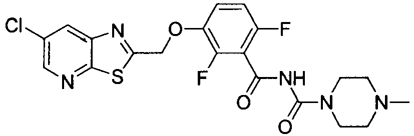 | 0.25 | 2.0 |
| **36** | 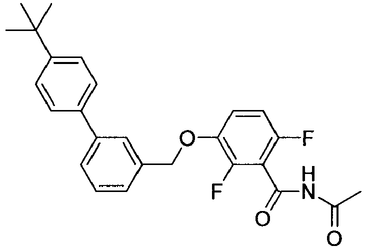 | >64.0 | >64.0 |
| **37** | 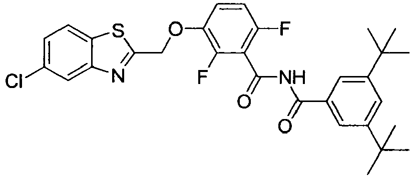 | >64.0 | 8.0 |
| **38** | 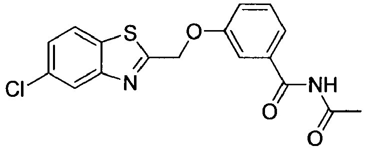 | >64.0 | >64.0 |
| **39** | 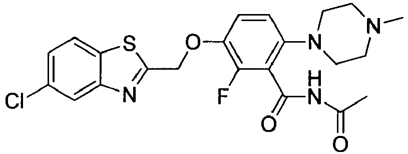 | >64.0 | >64.0 |
| **40** | 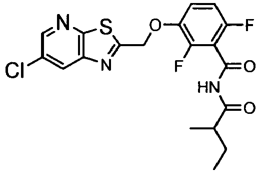 | >64.0 | 2.0 |
| **41** | 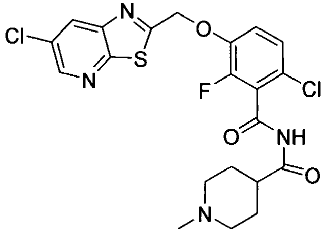 | 4.0 | 8.0 |
| **42** | 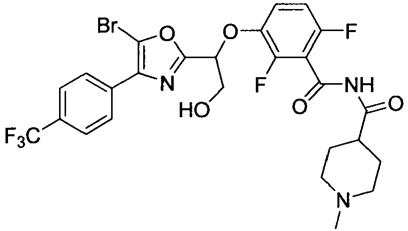 | 1.0 | 16.0 |
| **43** | 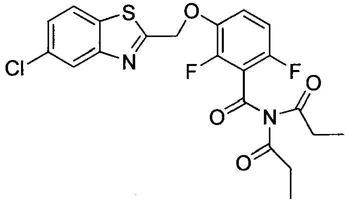 | 64.0 | 64.0 |
| **44** | 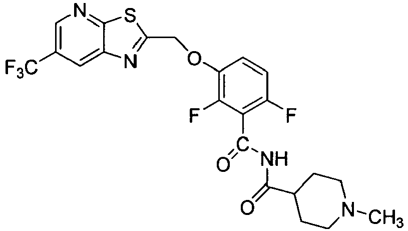 | 0.5 | 2.0 |

| | | | |
|---|---|---|---|
| * MIC determined in the presence of 50% mouse serum ** MIC values may be lower as solubility and aggregate formation may reduce observed activity. | | | |

The impact of a compound of the invention on the dynamics of bacterial FtsZ polymerization can be determined using a method like Test B described below.

### Test B. FtsZ Polymerization Assay.

Compound-induced alteration in the dynamics of FtsZ polymerization can be tested using a turbidity assay with purified FtsZ protein. Upon addition of GTP, FtsZ self-associates to form polymeric structures that scatter light at 340 nm to a greater extent than the monomeric protein. The impact of the compounds of the invention on the polymerization dynamics of FtsZ can be detected by an increase or decrease in the extent of GTP-induced light scattering (as determined by corresponding changes in optical density at 340 nm) relative to that observed in the absence of compound. Quantitation of the overall extent of light scattering as a function of compound concentration provides an indication of the potency of that compound at altering the dynamics of FtsZ polymerization.

The impact of a compound of the invention on FtsZ Z-ring formation in bacteria can be determined using a method like Test C described below.

### Test C. FtsZ Z-Ring Assay.

The impact of a compound on FtsZ Z-ring formation can be determined using a strain of *Bacillus subtilis* (FG347) that expresses a green fluorescent protein (GFP)-ZapA fusion protein upon induction with xylose. ZapA is known to associate with FtsZ Z-rings in B. *subtilis* and, thus, serves as a marker for Z-ring formation. In this assay, log-phase FG347 bacteria are treated with differing concentrations of compound for time periods ranging from 1 to 6 hours. At each time point, aliquots are taken from each culture and then viewed with a fluorescence microscope. In the absence of compound, the bacteria exhibit green fluorescent foci (Z-rings) localized at mid-cell. By contrast, bacteria treated with a compound that disrupts Z-ring formation do not exhibit the green fluorescent Z-rings at mid-cell and are typically associated with an elongated, filamentous phenotype.

The *in vivo* efficacy of a compound of the invention can be determined using a method like Test D described below.

### Test D. In Vivo Efficacy in the Mouse Peritonitis or Mouse Septicemia Model.

Antistaphylococcal efficacy *in vivo* was assessed in a mouse peritonitis model of systemic infection with *S. aureus* ATCC 19636 (MSSA) or ATCC 43300 (MRSA). These studies were conducted in full compliance with the standards established by the US National Research Council's Guide for the Care and Use of Laboratory Animals, and were approved by the Institutional Animal Care and Use Committee (IACUC) of Rutgers University. Groups of 4-6 female Swiss-Webster mice with an average weight of 25 g were infected intraperitoneally with a lethal inoculum of each bacterial strain in saline. The inoculum of *S. aureus* ATCC 43300 contained 1.0 x 10⁸ CFUs/mL of bacteria, while the inoculum of *S. aureus* ATCC 19636 contained 0.8 x 10⁷ CFUs/mL of bacteria. All the inocula also contained porcine mucin (Sigma-Aldrich, Co.) at a (w/v) percentage of 1.5% (in ATCC 19636 inocula) or 5% (in the ATCC 43300 inoculum). The differing compositions of the inocula of these *S. aureus* strains were selected based on the virulence of each strain, with MSSA ATCC 19636 being the more virulent strain and MRSA ATCC 43300 being the less virulent strain.

All compound and vehicle intravenous (i.v.) administrations were by tail vein injection, with 17 being formulated at 2.0 mg/mL and **44** being formulated at both 2.0 and 3.0 mg/ml in 10 mM citrate (pH 2.6).

In the MSSA ATCC 19636 experiments, the first dose of compound was administered 10 minutes after infection, with subsequent doses being administered at 12-minute intervals thereafter unless otherwise noted. In the MRSA studies, the first dose of compound was administered one hour after infection, with subsequent doses being administered at 12-minute intervals thereafter unless otherwise indicated.

The body temperatures of all mice were monitored for a period of 5 days after infection. Body temperatures were recorded at the Xiphoid process using a noninvasive infrared thermometer (Braintree Scientific, Inc., Braintree, MA). Infected mice with body temperatures ≤ 28.9 °C were viewed as being unable to recover from the infection and were euthanized.

| **Compound 17** | **ATCC 19636 (MSSA)** | | | **Survival (%)** | | |
|---|---|---|---|---|---|---|
| | 0.8 x 10⁷ cells in 1.5% mucin | | | | | |
| Route | n/Group | Frequency | Total Dose/Mouse (mg) | 24 Hrs | 48 Hrs | 72 Hrs |
| i.v. | 6 | 1x^{a} | 0.6 | 0 | 0 | 0 |
| i.v | 6 | 2x^{a} | 1.2 | 0 | 0 | 0 |
| i.v | 6 | 3x^{a} | 1.8 | 33.3 | 33.3 | 33.3 |
| i.v | 6 | 4x^{a} | 2.4 | 83.3 | 83.3 | 83.3 |
| Vehicle Only i.v. | 6 | 4x^{a} | - | 0 | 0 | 0 |
| p.o. | 4 | 1x^{b} | 0.8 | 0 | 0 | 0 |
| p.o. | 4 | 2x^{b} | 1.6 | 0 | 0 | 0 |
| p.o. | 6 | 4x | 3.2 | 83.3 | 83.3 | 83.3 |
| p.o. | 6 | 4x^{b} | 3.2 | 100 | 100 | 100 |
| Vehicle Only p.o. | 6 | 4x | - | 0 | 0 | 0 |

| | | | | | | |
|---|---|---|---|---|---|---|
| ^{a} The first (t₁) i.v. dose was administered immediately prior to infection; subsequent doses were administered 15 minutes following the first (t₁) dose. ^{b} The first (t₁) p.o. dose was administered 5 minutes prior to infection; subsequent doses were administered 15 minutes apart following infection. | | | | | | |

| **Compound 17** | **ATCC 43300 (MRSA)** | | | **Survival (%)** | | |
|---|---|---|---|---|---|---|
| | 1.0 x 10⁸ cells in 5% mucin | | | | | |
| Route | n/Group | Frequency | Total Dose/Mouse (mg) | 24 Hrs | 48 Hrs | 72 Hrs |
| p.o. | 6 | 3x^{c} | 2.4 | 66.7 | 16.7 | 16.7 |
| p.o. | 6 | 6x^{c} | 4.8 | 100 | 100 | 100 |
| p.o | 6 | 6x | 4.8 | 83.3 | 50 | 50 |
| p.o | 6 | 6x^{d} | 4.8 | 100 | 100 | 100 |
| Vehicle Only p.o. | 6 | 6x | - | 0 | 0 | 0 |

| | | | | | | |
|---|---|---|---|---|---|---|
| ^{c} The first (t₁) p.o. dose was administered 5 minutes prior to infection; subsequent doses were administered 12 minutes apart following infection. ^{d}The first (t₁) p.o. dose was administered 10 minutes post-infection; subsequent doses were administered 12 minutes apart. | | | | | | |

| **Compound 44** | **ATCC 19636 (MSSA)** | | | **Survival (%)** | | |
|---|---|---|---|---|---|---|
| | 0.8 x 10⁷ cells in 1.5% mucin | | | | | |
| Route | n/Group | Frequency | Total Dose/Mouse (mg) | 24 Hrs | 48 Hrs | 72 Hrs |
| i.v. | 6 | 1x | 0.9 | 83.3 | 83.3 | 83.3 |
| i.v | 6 | 2x | 1.8 | 100 | 100 | 100 |
| Vehicle Only i.v. | 6 | 1x | - | 0 | 0 | 0 |
| p.o. | 6 | 1x | 0.8 | 50 | 50 | 50 |
| p.o. | 6 | 2x | 1.6 | 100 | 100 | 100 |
| p.o | 6 | 3x | 2.4 | 100 | 100 | 100 |
| p.o | 4 | 4x | 3.2 | 100 | 100 | 100 |
| Vehicle Only p.o. | 6 | 3x | - | 0 | 0 | 0 |

The invention will now be illustrated by the following non-limiting examples.

### Examples

### Example 1

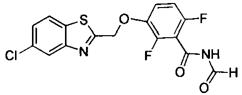

3-((5-Chlorobenzo[d]thiazol-2-yl)methoxy)-N-((dimethylamino)methylene)-2,6-difluorobenzamide (50 mg, 0.122 mmol) was treated with 70% acetic acid (1 ml) at room temperature for 12 hours. Water is added and the resulting solid was collected by filtration and was washed with cold water to afford the product as white solid (39 mg, 83% yield). ¹H NMR (DMSO-*d*6, 300 MHz) δ: 9.2 (s, 1H), 8.21 (d, *J* = 9.0 Hz, 1H), 8.14 (s, 1H), 7.54 (m, 2H), 7.25 (m, 1H), 5.76 (s, 2H).

The requisite intermediates were prepared as follows.

### a. Preparation of Compound

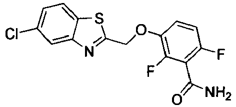

Prepared as described in the literature method by Haydon, Bennett, et al., J. Med. Chem., 2010, 53, 3927.

### b. Preparation of Compound

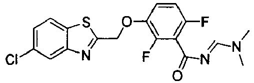

A suspension of 3-((5-chlorobenzo[d]thiazol-2-yl)methoxy)-2,6-difluorobenzamide (110 mg, 0.31 mmol) in 2.0 mL of dimethylformamide dimethyl acetal was stirred at 100 °C for 1 hour. The excess dimethylformamide dimethyl acetal was removed under vacuum and the resulting solid was triturated with diethyl ether to afford the pure product as white solid (90 mg, 71% yield). ¹H NMR (300 MHz, CDCl₃) δ: 8.62 (s, 1H), 8.01 (s, 1H), 7.82 (d, *J* = 8.4 Hz, 1H), 7.4 (m, 1H), 7.07-6.99 (m, 1H), 6.81 (m, 1H), 5.49 (s, 2H), 3.21 (s, 3H), 3.16 (s, 3H).

### Example 2

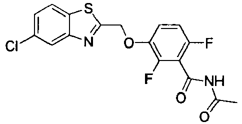

3-((5-Chlorobenzo[d]thiazol-2-yl)methoxy)-N-(1-(dimethylamino)ethylidene)-2,6-difluorobenzamide (50 mg, 0.118 mmol) was treated with 70% acetic acid (1.0 ml) at room temperature for 12 hours. Water is added and the resulting solid was collected by filtration and was washed with cold water to afford the product as white solid (37 mg, 79% yield). ¹H NMR (DMSO-*d*6, 300 MHz) δ: 8.65 (d, *J* = 8.4 Hz, 1H), 8.57 (s, 1H), 7.98 (d, *J* = 9.0 Hz, 1H), 7.92 (m, 1H), 7.62 (m, 1H), 6.17 (s, 2H), 2.65 (s, 3H).

The requisite intermediates were prepared as follows.

### a. Preparation of Compound

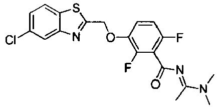

A suspension of 3-((5-chlorobenzo[d]thiazol-2-yl)methoxy)-2,6-difluorobenzamide (110 mg, 0.31 mol) in 1.5 mL of N,N-Dimethylacetamide Dimethyl acetal was stirred at 90 °C for 1 hour. The excess dimethylacetamide dimethyl acetal was removed under vacuum and the resulting solid was triturated with diethyl ether to afford the pure product as off white solid (50 mg, 79% yield). ¹H NMR (300 MHz, CDCl₃) δ: 8.01 (s, 1H), 7.82 (d, *J* = 8.7 Hz, 1H), 7.39 (dd, *J* = 6.6, 1.8 Hz, 1H), 6.99 (m, 1H), 6.8 (m, 1H), 5.48 (s, 2H), 3.17 (s, 3H), 3.14 (s, 3H), 2.44 (s, 3H).

### Example 3

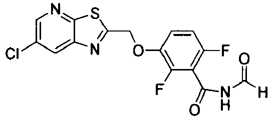

3-((6-Chlorothiazolo[5,4-b]pyridin-2-yl)methoxy)-N-((dimethylamino)methylene)-2,6-difluorobenzamide (100 mg, 0.243 mmol) was treated with 70% acetic acid (1.0 ml) at room temperature for 12 hours. Water is added and the resulting solid was collected by filtration and was washed with cold water to afford the product as white solid (67 mg, 71% yield) . ¹H NMR (DMSO-*d*6, 300 MHz) δ: 9.19 (bs, 1H), 8.76-8.70 (m, 2H), 7.60 (m, 1H), 7.28 (m, 1H), 5,79 (s, 2H).

The requisite intermediates were prepared as follows.

### a. Preparation of Compound

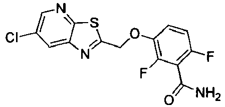

Prepared as described in the literature method by Haydon, Stokes, et al., Science, 2008, 321, 1673, Haydon, Bennett, et al., J. Med. Chem., 2010, 53, 3927, Sorto, et al., J. Org. Chem., 2010, 75, 7946, and Ding et al., Synlett, 2012, 23, 1039.

### b. Preparation of Compound

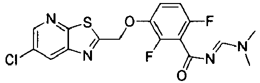

A suspension of 3-((6-chlorothiazolo[5,4-b]pyridin-2-yl)methoxy)-2,6-difluorobenzamide (20 mg, 0.06 mmol) in 1.0 mL of dimethylformamide dimethyl acetal was stirred at 90 °C for 1 hour. The excess dimethylformamide dimethyl acetal was removed under vacuum and the resulting solid was triturated with diethyl ether to afford the pure product as off white solid (10 mg, 45% yield). ¹H NMR (300 MHz, CDCl₃) δ: 8.63 (s, 1H), 8.57 (d, *J* = 2.4 Hz, 1H), 7.05 (m, 1H), 6.92 (m, 1H), 5.47 (s, 2H), 3.22 (s, 3H), 3.16 (s, 3H).

### Example 4

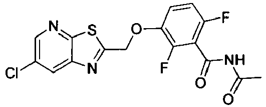

3-((6-Chlorothiazolo[5,4-b]pyridin-2-yl)methoxy)-N-(1-(dimethylamino)ethylidene)-2,6-difluorobenzamide (36 mg, 0.08 mmol) was treated with 70% acetic acid (0.5 ml) at room temperature for 12 hours. Water is added and the resulting solid was collected by filtration and was washed with cold water to afford the product as off white solid (25 mg, 89% yield). ¹H NMR (300 MHz, CDCl₃) δ: 8.58 (d, *J* = 2.4 Hz, 1H), 8.24 (d, *J* = 2.1 Hz, 1H), 7.26-7.18 (m, 1H), 6.97-6.90 (m, 1H), 5.50 (s, 2H), 2.55 (s, 3H).

The requisite intermediate was prepared as follows.

### a. Preparation of Compound

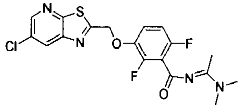

A suspension of 3-((6-chlorothiazolo[5,4-b]pyridin-2-yl)methoxy)-2,6-difluorobenzamide (100 mg, 0.28 mmol) in 1.0 mL of N,N-dimethylacetamide dimethyl acetal was stirred at 90 °C for 1 hour. The excess dimethylacetamide dimethyl acetal was removed under vacuum and the resulting solid was triturated with diethyl ether to afford the pure product as light yellow solid (95 mg, 79% yield). ¹H NMR (300 MHz, CDCl₃) δ: 8.56 (s, 1H), 8.22 (s, 1H), 6.99 (m, 1H), 6.8 (m, 1H), 5.48 (s, 2H), 3.17 (s, 3H), 3.17 (s, 3H), 2.45(s, 3H).

### Example 5

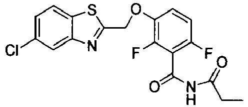

In a round bottom flask 3-((5-chlorobenzo[d]thiazol-2-yl)methoxy)-2,6-difluorobenzamide (35 mg, 0.1 mmol) was dissolved in 2 ml of dry THF, and the solution was cooled to 0 °C under nitrogen. This was followed by portion wise addition of NaH (8 mg, 0.2 mmol, 60% dispersion in mineral oil).The mixture was stirred at 0 °C for 10 minutes and at room temperature for 45 minutes. The mixture was cooled to 0 °C, and a solution of propionyl chloride (8 µl, 0.1 mmol) in 1 ml of THF was added dropwise. The resulting reaction mixture was stirred at 0 °C for 10 minutes and at room temperature for 4 hours. After completion of the reaction, it was quenched by the addition of few drops of 1N HCl, and diluted with ethyl acetate. The organic phase was separated, washed successively with sat. NaHCO₃, brine and dried. The solvent was removed in vacuo, and the resulting residue was purified by ISCO using 20% EtOAc in hexane as the elutant to afford the pure product as white solid (13 mg, 32% yield). ¹H NMR (300 MHz, CDCl₃) δ: 8.23 (s, 1H), 8.04 (s, 1H), 7.85 (d, *J* = 8.4 Hz, 1H), 7.44 (d, *J* = 9.0 Hz, 1H), 7.22 (m, 1H), 6.93 (m, 1H), 5.54 (s, 2H), 2.89 (qt*, J* = 7.2 Hz, 2H), 1.27-1.16 (m, 3H).

### Example 6

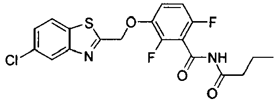

In a round bottom flask 3-((5-chlorobenzo[d]thiazol-2-yl)methoxy)-2,6-difluorobenzamide (55 mg, 0.155 mmol) was dissolved in 3 ml of dry THF, and the solution was cooled to 0 °C under nitrogen. This was followed by portion wise addition of NaH (13 mg, 0.310 mmol, 60% dispersion in mineral oil).The mixture was stirred at 0 °C for 10 minutes and at room temperature for 1 hour. The mixture was cooled to 0 °C, and a solution of butyryl chloride (0.016 ml, 0.155 mmol) in 1 ml of THF was added dropwise. The resulting reaction mixture was stirred at 0 °C for 10 minutes and at room temperature for overnight. After completion of the reaction, it was quenched by the addition of few drops of 1N HCl, and diluted with ethyl acetate. The organic phase was separated, washed successively with sat. NaHCO₃, brine and dried. The solvent was removed in vacuo, and the resulting residue was purified by ISCO using 40% EtOAc in hexane as the elutant to afford the desired product as white solid (20 mg, 31% yield). ¹H NMR (300 MHz, CDCl₃) δ: 8.24 (s, 1H), 8.05 (s, 1H), 7.86 (d, *J* = 8.7 Hz, 1H), 7.45 (d, *J* = 9.0 Hz, 1H), 7.22 (m, 1H), 6.93 (m, 1H), 5.54 (s, 2H), 2.84 (m, 2H), 1.75 (m, 2H), 1.04(m, 3H).

### Example 7

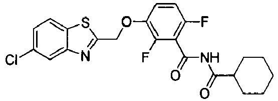

In a round bottom flask 3-((5-chlorobenzo[d]thiazol-2-yl)methoxy)-2,6-difluorobenzamide (35 mg, 0.1 mmol) was dissolved in 2 ml of dry THF, and the solution was cooled to 0 °C under nitrogen. This was followed by portion wise addition of NaH (12 mg, 0.3 mmol, 60% dispersion in mineral oil).The mixture was stirred at 0 °C for 10 minutes and at room temperature for 30 minutes. The mixture was cooled to 0 °C, and a solution of cyclohexanecarbonyl chloride (0.013 ml, 0.1 mmol) in 1 ml of THF was added dropwise. The resulting reaction mixture was stirred at 0 °C for 10 minutes and at room temperature for 12 hours. After completion of the reaction, it was quenched by the addition of few drops of 1N HCl, and diluted with ethyl acetate. The organic phase was separated, washed successively with sat. NaHCO₃, brine and dried. The solvent was removed in vacuo, and the resulting residue was purified by ISCO using 20 % EtOAc in hexane to afford desired product as yellow solid (16 mg, 35% yield). ¹H NMR (300 MHz, CDCl₃) δ: 8.27 (s, 1H), 8.03 (d, *J* = 2.1 Hz, 1H), 7.84 (d, *J* = 8.7 Hz, 1H), 7.43 (dd, *J* = 6.9, 2.1 Hz, 1H), 7.23-7.15 (m, 1H), 6.93-6.87 (m, 1H), 5.53 (s, 2H), 2.80 (m, 1H), 2.20-1.23 (m, 10H).

### Example 8

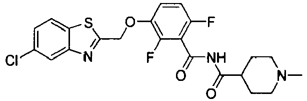

In a round bottom flask 3-((5-chlorobenzo[d]thiazol-2-yl)methoxy)-2,6-difluorobenzamide (25 mg, 0.07 mmol) was dissolved in 2.0 ml of dry THF, and the solution was cooled to 0°C under nitrogen. This was followed by portionwise addition of NaH (11 mg, 0.24 mmol, 60% dispersion in mineral oil).The mixture was stirred at 0°C for 10 minutes and at room temperature for 30 minutes. The mixture was cooled to 0°C, and a solution of acyl chloride 8a (28 mg, 0.14 mmol) in 1 ml of THF was added dropwise. The resulting reaction mixture was stirred at 0°C for 10 minutes and at room temperature overnight. After completion of the reaction, it was quenched by the addition of few drops of 1N NaOH, and diluted with ethyl acetate. The organic phase was separated, washed successively with sat. NaHCO₃, brine and dried. The solvent was removed in vacuo, and the resulting residue was purified by ISCO using 10 % MeOH in CH₂Cl₂ + 1% NH₄OH to afford yellow solid (16 mg, 36% yield). ¹H NMR (300 MHz, CDCl₃) δ: 8.01 (d, *J* = 2.1 Hz, 1H), 7.82 (d, *J* = 8.7 Hz, 1H), 7.40 (dd, *J* = 6.6, 1.8 Hz, 1H), 7.25-7.14 (m, 1H), 6.93-6.87 (m, 1H), 5.51 (s, 2H), 2.94-2.85 (m, 3H), 2.28 (s, 3H), 2.09-1.69 (m, 6H).

The requisite intermediate was prepared as follows.

### a. Preparation of Compound

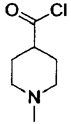

N-Methylisonipecotic acid hydrochloride (0.5 g) was dissolved in dry SOCl₂ (1.5 mL). The mixture was then heated at 80 °C for 2 hours under argon. Cooling and evaporation to dryness afforded a yellow solid which was used without further purification.

### Example 9

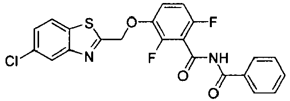

In a round bottom flask 3-((5-chlorobenzo[d]thiazol-2-yl)methoxy)-2,6-difluorobenzamide (30 mg, 0.08 mmol) was dissolved in 2 ml of dry THF, and the solution was cooled to 0°C under nitrogen. This was followed by portion wise addition of NaH (10 mg, 0.24mmol, 60% dispersion in mineral oil).The mixture was stirred at 0 °C for 10 minutes and at room temperature for 30 minutes. The mixture was cooled to 0 °C, and a solution of benzoyl chloride (10 µl, 0.08 mmol) in 1 ml of THF was added dropwise. The resulting reaction mixture was stirred at 0 °C for 10 minutes and at room temperature overnight. After completion of the reaction, it was quenched by the addition of few drops of 1N HCl, and diluted with ethyl acetate. The organic phase was separated, washed successively with sat. NaHCO₃, brine and dried. The solvent was removed in vacuo, and the resulting residue was purified by ISCO using 20% EtOAc in hexane to yield pure product as yellow solid (15 mg, 38% yield). ¹H NMR (300 MHz, CDCl₃) δ: 9.18 (s, 1H), 8.03 (s, 1H), 7.88 (m, 1H), 7.84 (d, *J* = 9.0 Hz, 1H), 7.68-7.63 (m, 1H), 7.56-7.51 (m, 2H), 7.45-7.40 (m, 1H), 7.24-7.17 (m, 1H), 6.96-6.89 (m, 1H), 5.53 (s, 2H).

### Example 10

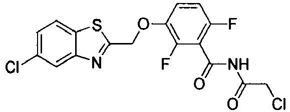

The mixture of 3-((5-chlorobenzo[d]thiazol-2-yl)methoxy)-2,6-difluorobenzamide (40 mg, 0.113 mmol) and chloroacetyl chloride (0.5 mL) is heated at 110 °C for 1 hour in a small reaction vial. The excess chloroacetyl chloride was removed under vacuum and the resulting residue was subjected to purification using ISCO to afford white solid (37 mg, 76% yield). ¹H NMR (300 MHz, CDCl₃) δ: 8.81 (s, 1H), 8.04 (s, 1H), 7.86 (d, *J* = 8.7 Hz, 1H), 7.43 (d, *J* = 8.7 Hz, 1H), 7.27 (m, 1H), 6.95 (m, 1H), 5.55 (s, 2H), 4.60 (s, 2H).

### Example 11

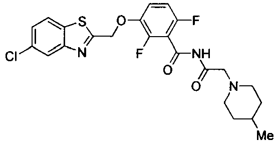

To a mixture of 3-((5-chlorobenzo[d]thiazol-2-yl)methoxy)-2,6-difluorobenzoyl chloride (33 mg, 0.076 mmol), K₂CO₃ (13 mg, 0.09 mmol) in DMF (1.5 ml) was added 4- methylpiperidine (0.010 ml, 0.09 mmol) at room temperature. The reaction mixture was stirred at room temperature for 1 hour, after which it was diluted with ethyl acetate and washed with water once. Evaporation of the solvent followed by ISCO purification using 10 % MeOH in CH₂Cl₂ afforded the pure product as colorless oil (12 mg, 33% yield). ¹H NMR (300 MHz, CDCl₃) δ: 8.04 (s, 1H), 7.85 (d, *J* = 8.7 Hz, 1H), 7.43 (d, *J* = 8.4 Hz, 1H), 7.18 (m, 1H), 6.92 (m, 1H), 5.53 (s, 2H), 3.12 (s, 2H), 2.85 (m, 2H), 2.27 (m, 2H), 1.68 (m, 2H), 1.28 (m, 1H), 0.98 (d, J = 6.0 Hz, 3H).

### Example 12

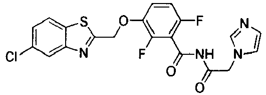

To the solution of 3-((5-chlorobenzo[d]thiazol-2-yl)methoxy)-2,6-difluorobenzoyl chloride (65 mg, 0.14 mmol) in DMF (1.5 ml) was added excess imidazole (48 mg, 0.70 mmol) and the mixture was stirred at room temperature overnight. The reaction mixture was poured into cold water and the solid thus formed was collected by filtration and was washed with ether to afford the desired compound as light yellow solid (58 mg, 90% yield). ¹H NMR (DMSO-*d*6, 300 MHz) δ: 11.97 (s, 1H), 8.20 (d, *J* = 8.7 Hz, 1H), 8.14 (s, 1H), 7.64 (s, 1H), 7.54 (m, 2H), 7.21 (m, 1H), 7.16 (s, 1H), 6.88 (s, 1H), 5.78 (s, 2H), 5.22 (s, 2H).

### Example 13

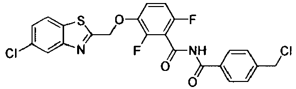

In a round bottom flask 3-((5-chlorobenzo[d]thiazol-2-yl)methoxy)-2,6-difluorobenzamide (55 mg, 0.155 mmol) was dissolved in 3 ml of dry THF, and the solution was cooled to 0 °C under nitrogen. This was followed by portion wise addition of NaH (13 mg, 0.310 mmol, 60% dispersion in mineral oil).The mixture was stirred at 0 °C for 10 minutes and at room temperature for 1 hour. The mixture was cooled to 0 °C, and a solution of chlorobenzoyl chloride (0.1 ml) in 1 ml of THF was added dropwise. The resulting reaction mixture was stirred at 0 °C for 10 minutes and at room temperature for overnight. After completion of the reaction, it was quenched by the addition of few drops of 1N HCl, and diluted with ethyl acetate. The organic phase was separated, washed successively with sat. NaHCO₃, brine and dried. The solvent was removed in vacuo, and the resulting residue was purified by ISCO using 50 % EtOAc in hexane to afford the desired product as white solid (52 mg, 66% yield). ¹H NMR (300 MHz, CDCl₃) δ: 9.10 (s, 1H), 8.04 (s, 1H), 7.91-7.83 (m, 3H), 7.56 (d, *J* = 9.0 Hz, 2H), 7.43 (d, *J* = 6.0 Hz, 1H), 7.25-7.18 (m, 1H), 6.97-6.90 (m, 1H), 5.54 (s, 2H), 4.65 (s, 2H).

### Example 14

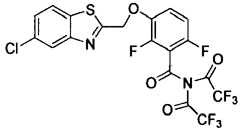

Trifluroacetic anhydride (0.05 ml, 0.339 mmol) is added to the solution of 3-((5-chlorobenzo[d]thiazol-2-yl)methoxy)-2,6-difluorobenzamide (100 mg, 0.282 mmol)in anhydrous THF (2 ml) and anhydrous pyridine (0.045ml) at room temperature with stirring, followed by heating the mixture to 78 °C for 12 hours. The reaction mixture was cooled to room temperature, concentrated on a rotary evaporator and azeotroped with toluene once. The solid is re dissolved in CH₂Cl₂, washed with water, dried over Na₂SO₄ and concentrated to give crude product. Purification using 40% EtOAc in hexane afforded the desired product (10 mg) as white solid. ¹H NMR (DMSO-*d*6, 300 MHz) δ: 8.21 (d, *J* = 8.7 Hz, 1H), 8.14 (s, 1H), 7.84-7.79 (m, 1H), 7.54 (d, *J* = 8.4 Hz, 1H), 7.47-7.40 (m, 1H), 5.76 (s, 2H).

### Reference Example 15

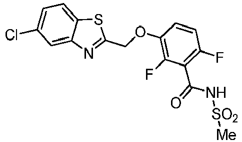

To a mixture of 3-((5-chlorobenzo[d]thiazol-2-yl)methoxy)-2,6-difluorobenzoyl chloride (97 mg, 0.26 mmol), methyl sulfonamide (26 mg, 0.26 mmol) in THF (1.5 mL) was added Et₃N (0.1 ml, 0.66 mmol) followed by catalytic amount of DMAP. The mixture was heated in a sealed tube at 60 °C for 1.5 hours. The reaction mixture was cooled to room temperature, diluted with ethyl acetate, washed with 1N HCl and brine. The organic phase was dried over Na₂SO₄, concentrated and purified by ISCO using 50% EtOAc in hexane to afford the desired product as off white solid (68 mg, 61% yield). ¹H NMR (300 MHz, CDCl₃) δ: 8.04 (s, 1H), 7.85 (d, 1H), 7.40 (d, 1H), 7.18 (m, 1H), 6.89 (m, 1H), 5.52 (s, 2H), 3.42 (s, 3H).

The requisite intermediates were prepared as follows.

### a. Preparation of Compound

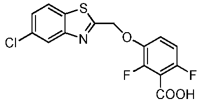

A suspension of 3-((5-chlorobenzo[d]thiazol-2-yl)methoxy)-2,6-difluorobenzamide (354 mg, 1.0 mmol) in 50% H₂SO₄ (6.0 ml) was heated at 120 °C for 3 hours. The reaction mixture was cooled down to room temperature, water was added and the resulting solid was filtered to afford a yellow solid as desired product (301 mg, 86% yield). ¹H NMR (DMSO-*d*6, 300 MHz) δ : 8.20 (d, *J* = 9.0 Hz, 1H), 8.13 (d, *J* = 2.1 Hz, 1H), 7.56-7.47 (m, 2H), 7.21-7.15 (m, 1H), 5.73 (s, 2H).

### b. Preparation of Compound

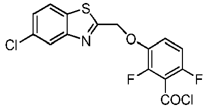

To a suspension of 3-((5-chlorobenzo[d]thiazol-2-yl)methoxy)-2,6-difluorobenzoic acid (200 mg) in CH₂Cl₂ (5.0 ml) was added catalytic amount of DMF followed by 1.5 equiv. oxalyl chloride. The reaction mixture was stirred at room temperature for 2 hours. The solvent was removed to afford crude acid chloride which was used for the next step without further purification.

### Example 16

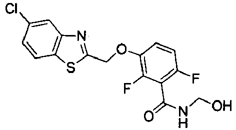

A mixture of 3-((5-chlorobenzo[d]thiazol-2-yl)methoxy)-2,6-difluorobenzamide (200 mg, 0.56 mmol), formaldehyde (1.5 ml), 5% K₂CO₃ (3.0 ml) in THF (1.5 ml) was heated to 65 °C overnight. After cooling to room temperature, water was added and the resulting solid was filtered and was washed with ether to give light yellow solid (190 mg, 88% yield). ¹H NMR (DMSO-*d*6, 300 MHz) δ: 9.34 (bs, 1H), 8.20 (d, *J* = 9.0 Hz, 1H), 8.14 (d, *J* = 2.1 Hz, 1H), 7.54 (m, 1H), 7.45-7.38 (m, 1H), 7.17-7.10 (m, 1H), 5.72 (s, 2H), 4.66(d, *J* = 6.0 Hz, 2H).

### Example 17

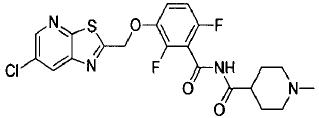

In a round bottom flask 3-((6-chlorothiazolo[5,4-b]pyridin-2-yl)methoxy)-2,6-difluorobenzamide (250 mg, 0.7 mmol) was dissolved in 4 ml of dry THF, and the solution was cooled to 0°C under nitrogen. This was followed by portion wise addition of NaH (110 mg, 2.4 mmol, 60% dispersion in mineral oil).The mixture was stirred at 0 °C for 10 minutes and at room temperature for 30 minutes. The mixture was cooled to 0 °C, and a solution of acyl chloride **8a** (280 mg, 1.4 mmol) in 1 ml of THF was added dropwise. The resulting reaction mixture was stirred at 0 °C for 10 minutes and at room temperature overnight. After completion of the reaction, it was quenched by the addition of few drops of 1N NaOH, and diluted with ethyl acetate. The organic phase was separated, washed successively with sat. NaHCO₃, brine and dried. The solvent was removed in vacuo, and the resulting residue was purified by ISCO using 10 % MeOH in CH₂Cl₂ + 1% NH₄OH to afford a light brown solid (50 mg, 15% yield). ¹H NMR (300 MHz, CDCl₃) δ: 8.58 (s, 1H), 8.25 (s, 1H), 7.24 (m, 1H), 6.92 (m, 1H), 5.5 (s, 2H), 2.91 (m, 3H), 2.3 (s, 3H), 2.07-1.85 (m, 5H).

### Example 18

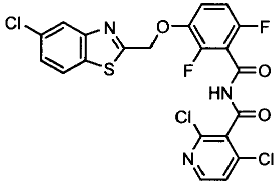

3-((5-Chlorobenzo[d]thiazol-2-yl)methoxy)-2,6-difluorobenzamide (25.3 mg) and a stir bar were placed under vacuum in a 2-dram vial. The vial was then filled with N₂ (g). 3-((5-Chlorobenzo[d]thiazol-2-yl)methoxy)-2,6-difluorobenzamide was dissolved in anhydrous THF (3.5 mL) and the resulting solution was stirred under N₂(g) while at room temperature. 2,4-Dichloropyridine-3-carbonyl chloride (0.01 mL, 1.0 eq., Sigma-Aldrich Co.) was added dropwise via syringe, followed by addition of sodium hydride (60% in oil dispersion) (14.3 mg, 5.0 eq.). The reaction was heated at 50 °C for 1 hour 30 min. After cooling to room temperature, the reaction was concentrated to a solid and then dissolved in EtOAc / H₂O. After shaking, the aqueous phase was separated and then extracted with EtOAc. The combined EtOAc phases were dried over Na₂SO₄, filtered, and concentrated to a solid. Chromatography with solvent gradient 0 > 30% EtOAc / hexanes isolated the product as a solid (23 mg, 61% yield). ¹H NMR (400 MHz) (CDCl₃) δ: 8.73 (br. s, 1H), 8.315 (d, *J* = 5.4 Hz, 1H), 7.93 (d, *J* = 2 Hz, 1H), 7.76 (d, *J* = 8.6 Hz, 1H), 7.34 (dd, H = 8.6 Hz, *J* = 2 Hz, 2H), 7.30 (d, *J* = 5.4 Hz, 1H), 7.18 (m, 1H), 6.88 (ddd, *J* = 9 Hz, *J* = 9 Hz, *J* = 2 Hz, 1H), 5.45 (s, 2H). MS: *m*/*e* = 528 (M+1).

### Example 19

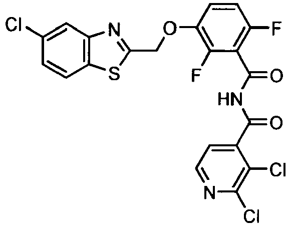

3-((5-Chlorobenzo[d]thiazol-2-yl)methoxy)-2,6-difluorobenzamide (25.1 mg) and a stir bar were placed under vacuum in a 2-dram vial. The vial was then filled with N₂ (g). 3-((5-Chlorobenzo[d]thiazol-2-yl)methoxy)-2,6-difluorobenzamide was dissolved in anhydrous THF (3.5 mL) and the resulting solution was stirred under N₂ (g) while at room temperature. 2,3-Dichloropyridine-4-carbonyl chloride (0.0095 mL, 1.0 eq., Sigma-Aldrich Co.) was added dropwise via syringe, followed by addition of sodium hydride (60% in oil dispersion) (14.2 mg, 5.0 eq.) The reaction was heated at 50 °C for 2 hours. After cooling to room temperature, the reaction was concentrated to a residue and then dissolved in EtOAc / H₂O. After shaking, the aqueous phase was separated and extracted with EtOAc. The combined EtOAc phases were dried over Na₂SO₄, filtered, and concentrated to a solid. Chromatography with solvent gradient 0 > 30% EtOAc / hexanes isolated the product as a solid (15 mg, 39% yield). ¹H NMR (400 MHz) (CDCl₃) δ: 8.60 (br.s, 1H), 8.37 (d, *J=* 4.84 Hz, 1H), 7.95 (d, *J=* 2 Hz, 1H), 7.76 (d, *J* = 8.6 Hz, 1H), 7.33 (dd, *J* = 8.6 Hz, *J* = 2 Hz, 1H), 7.27 (d, *J* = 4.84 Hz, 1H), 7.20 (m, 1H), 6.88 (ddd, *J* = 9 Hz, J = 9 Hz, *J=* 2 Hz, 1H), 5.46 (s, 2H). MS: *m*/*e =* 528 (M+1).

### Example 20

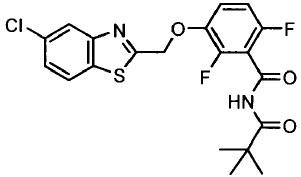

3-((5-Chlorobenzo[d]thiazol-2-yl)methoxy)-2,6-difluorobenzamide (25.8 mg) and a stir bar were placed under vacuum in a 2-dram vial. The vial was then filled with N₂ (g). 3-((5-chlorobenzo[d]thiazol-2-yl)methoxy)-2,6-difluorobenzamide was dissolved in anhydrous THF (3.5 mL) and the resulting solution was stirred under N₂ (g) while at room temperature. Trimethylacetyl chloride (0.009 mL, 1.0 eq., Sigma-Aldrich Co.) was added dropwise via syringe, followed by addition of sodium hydride (60% in oil dispersion) (13.4 mg, 4.6 eq.). The reaction was heated at 50 °C for 2 hours. After cooling to room temperature, the reaction was concentrated to a residue and then dissolved in EtOAc / H₂O. After shaking, the aqueous phase was separated and then extracted with EtOAc. The combined EtOAc phases were dried over Na₂SO₄, filtered, and concentrated to a solid. Chromatography with solvent gradient 0 > 30% EtOAc / hexanes isolated the product as a solid (15.6 mg, 49% yield). ¹H NMR (400 MHz) (CD₃OD) δ: 7.92 (d, *J* = 8.6 Hz, 1H), 7.91 (d, *J* = 2 Hz, 1H), 7.38 (dd, *J=* 8.6 Hz, *J=* 2Hz, 1H), 7.27 (ddd, *J* = 9 Hz, J = 9 Hz, *J* = 5.1Hz, 1H), 6.89 (ddd, *J* = 9 Hz, *J* = 9 Hz, *J* = 2 Hz, 1H), 5.50 (s, 2H), 1.14 (s, 9H). MS: *m*/*e* = 439 (M+1).

### Example 21

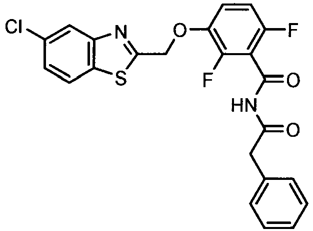

3-((5-Chlorobenzo[d]thiazol-2-yl)methoxy)-2,6-difluorobenzamide (25.3 mg) and a stir bar were placed under vacuum in a 2-dram vial. The vial was then filled with N₂ (g). 3-((5-Chlorobenzo[d]thiazol-2-yl)methoxy)-2,6-difluorobenzamide was dissolved in anhydrous THF (3.5 mL) and the resulting solution was stirred under N₂ (g) while at room temperature. Phenylacetyl chloride (9.4 µL, 1.0 eq., Sigma-Aldrich Co.) was added dropwise via syringe, followed by addition of sodium hydride (60% in oil dispersion) (14.3 mg, 5.0 eq.). The reaction was heated at 50 °C for 2 hours. After cooling to room temperature, the reaction was concentrated to a residue and then dissolved in EtOAc / H₂O. After shaking, the aqueous phase was separated and then extracted with EtOAc. The combined EtOAc phases were dried over Na₂SO₄, filtered, and concentrated to a solid. Chromatography with solvent gradient 0 > 30% EtOAc / hexanes isolated the product as a solid (7.6 mg, 22% yield). ¹H NMR (400 MHz) (CDCl₃) δ: 8.15 (br. s, 1H), 7.95 (d, *J* = 2 Hz, 1H), 7.76 (d, *J=* 8.54 Hz, 1H), 7.34 (dd, *J* = 8.54 Hz, *J* = 2 Hz, 1H), 7.31-7.22 (m, 5H), 7.11 (ddd, *J* = 9 Hz, *J* = 9 Hz, *J* = 5.1 Hz, 1H), 6.82 (ddd, *J=* 9 Hz, *J=* 9 Hz, *J=* 2 Hz, 1H), 5.45 (s, 2H), 4.0 (s, 2H). MS: *m*/*e* = 473 (M+1).

### Example 22

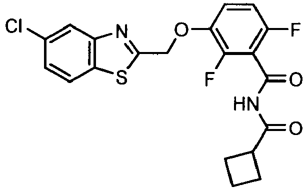

3-((5-Chlorobenzo[d]thiazol-2-yl)methoxy)-2,6-difluorobenzamide (25.1 mg) and a stir bar were placed under vacuum in a 2-dram vial. The vial was then filled with N₂ (g). 3-((5-chlorobenzo[d]thiazol-2-yl)methoxy)-2,6-difluorobenzamide was dissolved in anhydrous THF (4.0 mL) and the resulting solution was stirred under N₂ (g) while at room temperature. Cyclobutanecarbonyl chloride (8.1 µL, 1.0 eq., Sigma-Aldrich Co.) was added dropwise via syringe, followed by addition of sodium hydride (60% in oil dispersion) (14.3 mg, 5.0 eq.). The reaction was heated at 50 °C for 2 hours. After cooling to room temperature, the reaction was concentrated to a residue and then dissolved in EtOAc / H₂O. After shaking, the aqueous phase was separated and then extracted with EtOAc. The combined EtOAc phases were dried over Na₂SO₄, filtered, and concentrated to a solid. Chromatography with solvent gradient 0 > 30% EtOAc / hexanes isolated the product as a solid (22.9 mg, 74% yield). ¹H NMR (400 MHz) (CD₃OD) δ: (d, *J* = 8.5 Hz, 1H), 7.906 (d, *J* = 2.1 Hz, 1H), 7.37 (dd, *J* = 8.5 Hz, *J* = 2.1 Hz, 1H), 7.28 (ddd, *J* = 9.2 Hz, *J* = 9.2 Hz, *J* = 5.1 Hz, 1H), 6.91 (ddd, *J* = 9.2 Hz, *J* = 9.2 Hz, *J* = 2 Hz, 1H), 5.50 (s, 2H), 3.38 (m, 1H), 2.18 (m, 4H), 1.92 (m, 1H), 1.79 (m, 1H). MS: *m*/*e* = 437 (M+1).

### Example 23

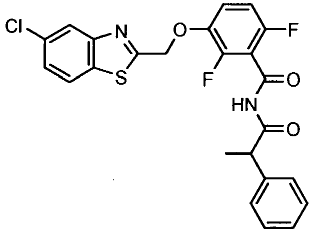

3-((5-Chlorobenzo[d]thiazol-2-yl)methoxy)-2,6-difluorobenzamide (25.6 mg) and a stir bar were placed under vacuum in a 2-dram vial. The vial was then filled with N₂ (g). 3-((5-Chlorobenzo[d]thiazol-2-yl)methoxy)-2,6-difluorobenzamide was dissolved in anhydrous THF (4.0 mL) and the resulting solution was stirred under N₂ (g) while at room temperature. 2-Phenylpropionyl chloride (12.3 mg, 1.0 eq., Sigma-Aldrich Co.) was added dropwise via syringe, followed by addition of sodium hydride (60% in oil dispersion) (8.7 mg, 3.0 eq.). The reaction was heated at 50 °C for 30 min. After cooling to room temperature, the reaction was concentrated to a residue and then dissolved in EtOAc / H₂O. After shaking, the aqueous phase was separated and then extracted with EtOAc. The combined EtOAc phases were dried over Na₂SO₄, filtered, and concentrated to a solid. Chromatography with solvent gradient 0 > 20% EtOAc / hexanes isolated the product as a solid (17.9 mg, 51% yield). ¹H NMR (400 MHz) (CDCl₃) δ: (br. s, 1H), 8.04 (d, *J=* 2 Hz, 1H), 7.85 (d, *J=* 8.56 Hz, 1H), 7.45-7.29 (m, 6H), 7.18 (ddd, *J* = 9.1 Hz, *J* = 9.1 Hz, *J* = 5 Hz, 1H), 6.88 (ddd, *J* = 9.1 Hz, *J* = 9.1 Hz, *J* = 2 Hz, 1H), 5.51 (s, 2H), 4.09 (q, *J* = 7.1 Hz, 1H), 1.54 (d, *J* = 7.1 Hz, 3H). MS: *m*/*e* = 487 (M+1).

### Example 24

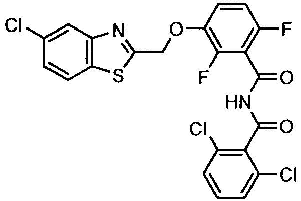

3-((5-Chlorobenzo[d]thiazol-2-yl)methoxy)-2,6-difluorobenzamide (25.2 mg) and a stir bar were placed under vacuum in a 2-dram vial. The vial was then filled with N₂ (g). 3-((5-chlorobenzo[d]thiazol-2-yl)methoxy)-2,6-difluorobenzamide was dissolved in anhydrous THF (4.0 mL) and the resulting solution was stirred under N₂ (g) while at room temperature. 2,6-Dichlorobenzoyl chloride (10 µL, 1.0 eq., Sigma-Aldrich Co.) was added dropwise via syringe, followed by addition of sodium hydride (60% in oil dispersion) (8.5 mg, 3.0 eq.). The reaction was heated at 50 °C for 30 min. After cooling to room temperature, the reaction was concentrated to an oil which was then dissolved in EtOAc / H₂O. After shaking, the aqueous phase was separated and extracted with EtOAc. The combined EtOAc phases were dried over Na₂SO₄, filtered, and concentrated to provide an oil. Chromatography with solvent gradient 0 > 20% EtOAc / hexanes isolated the product as a solid (15.4 mg, 39% yield). ¹H NMR (400 MHz) (CDCl₃) δ: 8.50 (br. s, 1H), 7.94 (d, *J=* 2 Hz, 1H), 7.76 (d, *J=* 8.6 Hz, 1H), 7.33 (dd, *J* = 8.6 Hz, *J=* 2 Hz, 1H), 7.32 - 7.13 (m, 4H), 6.86 (ddd, *J=* 9.12 Hz, *J*= 9.12 Hz, *J=* 2 Hz, 1H), 5.45 (s, 2H). MS: *m*/*e* = 527 (M+1).

### Example 25

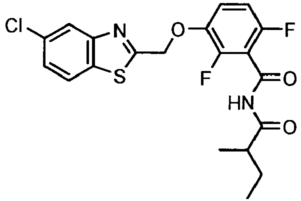

3-((5-Chlorobenzo[d]thiazol-2-yl)methoxy)-2,6-difluorobenzamide (26.6 mg) and a stir bar were placed under vacuum in a 2-dram vial. The vial was then filled with N₂ (g). 3-((5-Chlorobenzo[d]thiazol-2-yl)methoxy)-2,6-difluorobenzamide was dissolved in anhydrous THF (4.0 mL) and the resulting solution was stirred under N₂ (g) while at room temperature. 2-Methylbutyryl chloride (9.3 µL, 1.0 eq., Sigma-Aldrich Co.) was added dropwise via syringe, followed by addition of sodium hydride (60% in oil dispersion) (9.0 mg, 3.0 eq.). The reaction continued to stir under N₂ (g) while at room temperature for 30 min. The reaction was concentrated to a residue which was then dissolved in EtOAc / H₂O. After shaking, the aqueous phase was separated and extracted with EtOAc. The combined EtOAc phases were dried over Na₂SO₄, filtered, and concentrated to a solid. Chromatography with solvent gradient 10 > 30% EtOAc / hexanes isolated the product as a solid (15.5 mg, 47% yield). ¹H NMR (400 MHz) (CDCl₃) δ: 8.21 (br. s, 1H), 8.04 (d, *J=* 2 Hz, 1H), 7.85 (d, *J=* 8.55 Hz, 1H), 7.43 (dd, *J* = 8.55 Hz, *J* = 2 Hz, 1H), 7.20 (ddd, *J* = 9 Hz, *J* = 9 Hz, *J* = 5.1 Hz, 1H), 6.91 (ddd, *J* = 9 Hz, *J* = 9 Hz, *J* = 2 Hz, 1H), 5.05 (s, 2H), 1.81 (m, *J* = 7 Hz, 1H), 1.54 (d, *J*= 7 Hz, 2H), 1.25 (d, *J* = 7 Hz, 3H), 1.00 (t, *J=* 7 Hz, 3H). MS: *m*/*e* = 439 (M+1).

### Example 26

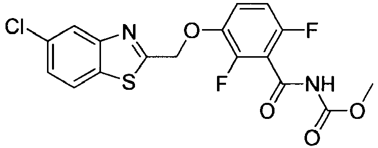

3-((5-Chlorobenzo[d]thiazol-2-yl)methoxy)-2,6-difluorobenzamide (25.4 mg) and a stir bar were placed under vacuum in a 2-dram vial. The vial was then filled with N₂ (g). 3-((5-Chlorobenzo[d]thiazol-2-yl)methoxy)-2,6-difluorobenzamide was dissolved in THF (3.5 mL) and the resulting solution was stirred under N₂ (g) while at room temperature. Methyl chloroformate (5.5 µL, 1.0 eq., Acros Organics) was added dropwise via syringe, followed by addition of sodium hydride (60% in oil dispersion) (14.3 mg, 5.0 eq.). The reaction was heated to 50 °C with gradual warming to 70 °C over a two hour period. After cooling to room temperature, and the reaction was concentrated to a solid and dissolved in EtOAc / H₂O. After shaking, the aqueous phase was separated and extracted with EtOAc. The combined EtOAc phases were dried over Na₂SO₄, filtered, and concentrated to a solid. Chromatography with solvent gradient 10 > 30% EtOAc / hexanes isolated the product as a solid (12.1 mg, 41% yield). ¹H NMR (400 MHz) (CD₃OD) δ: 7.92 (d, *J* = 8.6 Hz, 1H), 7.91 (d, *J* = 2 Hz, 1H), 7.37 (dd, *J=* 8.6 Hz, *J=* 2 Hz, 1H), 7.29 (ddd, *J=* 9 Hz, *J=* 9 Hz, *J=* 5.1 H_{z}, 1H), 6.91 (ddd, *J* = 9 Hz, *J* = 9 Hz, *J* = 2 Hz, 1H), 5.50 (s, 2H), 3.66 (s, 3H). MS: *m*/*e* = 413 (M+1).

### Example 27

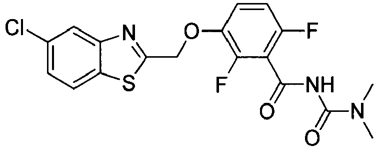

3-((5-Chlorobenzo[d]thiazol-2-yl)methoxy)-2,6-difluorobenzamide (26.4 mg) and a stir bar were placed under vacuum in a 2-dram vial. The vial was then filled with N₂ (g). 3-((5-chlorobenzo[d]thiazol-2-yl)methoxy)-2,6-difluorobenzamide was dissolved in THF (3.5 mL) and the resulting solution was stirred under N₂ (g) while at room temperature. Dimethylcarbamoyl chloride (6.8 µL, 1.0 eq., TCI America, Inc.) was added dropwise via syringe, followed by addition of sodium hydride (60% in oil dispersion) (14.9 mg, 5.0 eq.). The reaction was stirred at 65 °C while under N₂ (g) for 2 hours. After cooling to room temperature, the reaction was concentrated to a solid and dissolved in EtOAc / H₂O. After shaking, the aqueous phase was separated and extracted with EtOAc. The combined EtOAc phases were dried over Na₂SO₄, filtered, and concentrated to a solid. Chromatography with solvent gradient 45 > 50 % EtOAc / hexanes isolated the product as a solid (14.0 mg, 44% yield). ¹H NMR (400 MHz) (CD₃OD) δ: 7.91 (d, *J* = 8.6 Hz, 1H), 7.90 (d, *J* = 2 Hz, 1H), 7.37 (dd, *J* = 8.6 Hz, *J* = 2 Hz, 1H), 7.265 (ddd, *J* = 9 Hz, *J* = 9 Hz, *J* = 5.1, 1H), 6.895 (ddd, *J* = 9 Hz, *J=* 9 Hz, *J=* 2 Hz, 1H), 5.49 (s, 2H), 2.92 (br. s, 6H). MS: *m*/*e* = 426 (M+1).

### Example 28

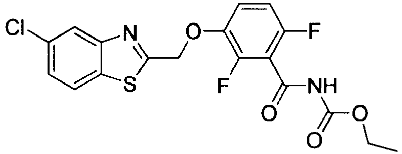

3-((5-Chlorobenzo[d]thiazol-2-yl)methoxy)-2,6-difluorobenzamide (26.9 mg) and a stir bar were placed under vacuum in a 2-dram vial. The vial was then filled with N₂ (g). 3-((5-Chlorobenzo[d]thiazol-2-yl)methoxy)-2,6-difluorobenzamide was dissolved in THF (3.5 mL) and the resulting solution was stirred under N₂ (g) while at room temperature. Ethyl chloroformate (7.25 µL, 1.0 eq., Sigma-Aldrich Co.) was added dropwise via syringe, followed by addition of sodium hydride (60% in oil dispersion) (15.2 mg, 5.0 eq.). The reaction was heated to 75 °C for 2 hours while under N₂ (g). After cooling to room temperature, and the reaction was concentrated to a solid and dissolved in EtOAc / H₂O. After shaking, the aqueous phase was separated and extracted with EtOAc. The combined EtOAc phases were dried over Na₂SO₄, filtered, and concentrated to a solid. Chromatography with solvent gradient 10 > 30% EtOAc / hexanes isolated the product as a solid (17.9 mg, 55% yield). ¹H NMR (400 MHz) (CD₃OD) δ: 7.91 (d, *J=* 8.6 Hz, 1H), 7.91 (d, *J* = 2 Hz, 1H), 7.37 (dd, *J=* 8.6 Hz, *J=* 2 Hz, 1H) 7.28 (ddd, *J* = 9 Hz, *J* = 9 Hz, *J* = 5.1 Hz, 1H), 6.90 (ddd, *J* = 9 Hz, *J* = 9 Hz, *J* = 2 Hz, 1H), 5.50 (s, 2H), 4.10 (q, *J* = 7.12 Hz, 2H), 1.15 (t, *J* = 7.12 Hz, 3H).

### Example 29

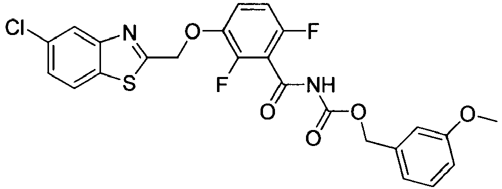

3-((5-Chlorobenzo[d]thiazol-2-yl)methoxy)-2,6-difluorobenzamide (26.0 mg) and a stir bar were placed under vacuum in a 2-dram vial. The vial was then filled with N₂ (g). 3-((5-Chlorobenzo[d]thiazol-2-yl)methoxy)-2,6-difluorobenzamide was dissolved in THF (3.5 mL) and the resulting solution was stirred under N₂ (g) while at room temperature. 3-Methoxyphenylacetyl chloride (11.4 µL, 1.0 eq., Sigma-Aldrich Co.) was added dropwise via syringe, followed by addition of sodium hydride (60% in oil dispersion) (14.7 mg, 5.0 eq.). The reaction was heated to 50 °C for 1 hour and 75 °C for 30 min. After cooling to room temperature, the reaction was concentrated to a solid and dissolved in EtOAc / H₂O. After shaking, the aqueous phase was separated and extracted with EtOAc. The combined EtOAc phases were dried over Na₂SO₄, filtered, and concentrated to a solid. Chromatography with solvent gradient 10 > 30% EtOAc / hexanes isolated the product as a solid (3.2 mg, 8.4% yield). ¹H NMR (400 MHz) (CD₃OD) δ: 7.91 (m, 2H), 7.37 (dd, *J*= 8.68 Hz, *J=* 1.92 Hz, 1H), 7.28 (ddd, *J* = 9 Hz, *J* = 9 Hz, *J* = 5 Hz, 1H), 7.13 (m, 1H), 6.90 (ddd, *J* = 9 Hz, *J* = 9 Hz, *J =* 2 Hz, 1H), 6.78-6.71 (m, 3H), 5.5 (s, 2H).

### Example 30

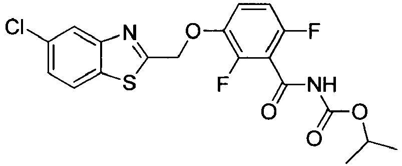

3-((5-Chlorobenzo[d]thiazol-2-yl)methoxy)-2,6-difluorobenzamide (26.8 mg) and a stir bar were placed under vacuum in a 2-dram vial. The vial was then filled with N₂ (g). 3-((5-chlorobenzo[d]thiazol-2-yl)methoxy)-2,6-difluorobenzamide was dissolved in THF (3.5 mL) and the resulting solution was stirred under N₂ (g) while at room temperature. Isopropyl chloroformate (1M in toluene) (75.5 µl, 1.0 eq., Sigma-Aldrich Co.) was added dropwise via syringe, followed by addition of sodium hydride (60% in oil dispersion) (15.1 mg, 5.0 eq.). The reaction was heated to 50 °C, then gradually warmed to 75 °C over 3 hours 45 min. After cooling to room temperature, and the reaction was concentrated to a solid and dissolved in EtOAc / H₂O. After shaking, the aqueous phase was separated and then extracted with EtOAc. The combined EtOAc phases were dried over Na₂SO₄, filtered, and concentrated to a solid. Chromatography with solvent gradient 10 > 30% EtOAc / hexanes isolated a solid (8.5g, 25% yield). ¹H NMR (400 MHz) (CDCl₃) δ: 7.945 (d, *J=* 2 Hz, 1H), 7.76 (d, *J=* 8.6 Hz, 1H), 7.655 (br. s, 1H), 7.34 (dd, *J* = 8.6 Hz, *J* = 2 Hz, 1H), 7.09 (ddd, *J* = 9 Hz, *J* = 9 Hz, *J* = 5.1 Hz, 1H), 6.81 (ddd, *J=* 9 Hz, *J=* 9 Hz, *J* = 2 Hz, 1H), 5.44 (s, 2H), 4.89 (m, *J* = 6.28 Hz, 1H), 1.19 (d, *J=* 6.28 Hz, 6H). MS: *m*/*e* = 441 (M+1).

### Example 31

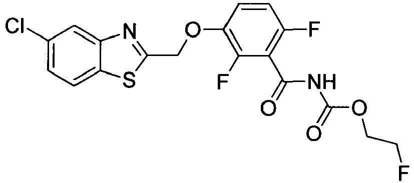

3-((5-Chlorobenzo[d]thiazol-2-yl)methoxy)-2,6-difluorobenzamide (25.7 mg) and a stir bar were placed under vacuum in a 2-dram vial. The vial was then filled with N₂ (g). 3-((5-Chlorobenzo[d]thiazol-2-yl)methoxy)-2,6-difluorobenzamide was dissolved in THF (3.5 mL) and the resulting solution was stirred under N₂ (g) while at room temperature. 2-Fluoroethylformate (7.0 µL, 1.0 eq., Sigma-Aldrich Co.) was added dropwise, followed by addition of sodium hydride (60% in oil dispersion) (14.5 mg, 5.0 eq.). The reaction was heated at 50 °C for 1 hour and then at 75 °C for 2 hours. After cooling to room temperature, and the reaction was concentrated to a solid and dissolved in EtOAc / H₂O. After shaking, the aqueous phase was separated and then extracted with EtOAc. The combined EtOAc phases were dried over Na₂SO₄, filtered, and concentrated to a solid. Chromatography with solvent gradient 0 > 45% EtOAc / hexanes isolated a solid (15.7 mg, 49% yield). ¹H NMR (400 MHz) (CDCl₃) δ: 7.95 (d, *J* = 2 Hz, 1H), 7.84 (br. s, 1H), 7.76 (d, *J* = 8.6 Hz, 1H), 7.34 (dd, *J* = 8.6 Hz, *J* = 2 Hz, 1H), 7.11 (ddd, *J* = 9 Hz, *J* = 9 Hz, *J* = 5.1 Hz, 1H), 6.82 (ddd, *J* = 9 Hz, *J=* 9 Hz, *J=* 2 Hz, 1H), 4.54 (dt, *J* = 47.28 Hz, *J* = 4.1 Hz, 2H), 4.34 (dt, *J* = 28.2 Hz, *J* = 4.1 Hz, 2H). MS: *m*/*e* = 445 (M+1).

### Example 32

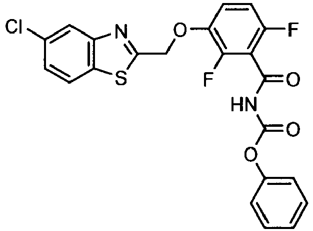

3-((5-Chlorobenzo[d]thiazol-2-yl)methoxy)-2,6-difluorobenzamide (26.5 mg) and a stir bar were placed under vacuum in a 2-dram vial. The vial was then filled with N₂ (g). 3-((5-Chlorobenzo[d]thiazol-2-yl)methoxy)-2,6-difluorobenzamide was dissolved in anhydrous THF (3.5 mL) and the resulting solution was stirred under N₂ (g) while at room temperature. Phenylchloroformate (9.4 µL, 1.0 eq., Sigma-Aldrich Co.) was added dropwise via syringe, followed by addition of sodium hydride (60% in oil dispersion) (15 mg, 5.0 eq.). The reaction was heated at 75 °C for 2 hours. After cooling to room temperature, the reaction was concentrated to a residue and then dissolved in EtOAc / H₂O. After shaking, the aqueous phase was separated and then extracted with EtOAc. The combined EtOAc phases were dried over Na₂SO₄, filtered, and concentrated to a solid. Chromatography with solvent gradient 10 > 45% EtOAc / hexanes isolated the product as a solid (12.8 mg, 36% yield). ¹H NMR (300 MHz) (CD₃OD) δ: 8.03 (d, *J* = 8.6 Hz, 1H), 8.02 (d, *J=* 2 Hz, 1H), 7.49 (dd, *J=* 8.6 Hz, *J=* 2 Hz, 1H), 7.43 (m, 3H), 7.29 (ddd, *J* = 9 Hz, J = 9 Hz, *J* = 5.1 Hz, 1H), 7.20 (m, 2H), 7.05 (ddd, *J*= 9 Hz, *J* = 9 Hz, *J* = 2 Hz, 1H), 5.65 (s, 2H).

### Example 33

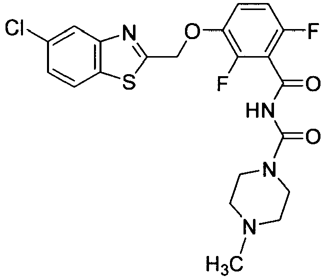

Phenyl(3-((5-chlorobenzo[d]thiazol-2-yl)methoxy)-2,6-difluorobenzoyl)carbamate (6.5 mg, 1.0 eq.) and 1-methylpiperazine (2.0 µL, 1.0 eq.) were placed in toluene (0.2 mL) and stirred at 100 °C for 1 hour. After cooling to room temperature, the reaction was concentrated to a solid. Chromatography with CH₂Cl₂, (90 CH₂Cl₂: 10 MeOH : 1 NH₄OH) isolated the product as a solid (4.9 mg, 74%). ¹H NMR (300 MHz) (CDCl₃) δ: 8.05 (d, *J=* 2 Hz, 1H), 7.85 (d, *J=* 8.7 Hz, 1H), 7.67 (br. s, 1H), 7.43 (dd, *J* = 8.7 Hz, *J* = 2Hz, 1H), 7.16 (ddd, *J* = 9 Hz, J = 9 Hz, *J* = 5.1 Hz, 1H), 6.91 (ddd, *J* = 9 Hz, *J* = 9 Hz, *J* = 2 Hz, 1H), 5.5 (s, 2H), 3.56 (m, 4H), 2.5 (m, 4H), 2.35 (s, 3H). MS: *m*/*e* = 481 (M+1).

### Example 34

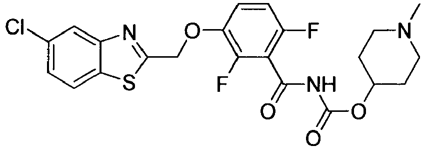

3-((5-Chlorobenzo[d]thiazol-2-yl)methoxy)-2,6-difluorobenzamide (26.3 mg, 0.074 mmol, 1.0 eq.) and a stir bar were placed under vacuum in a 2-dram vial. The vial was then filled with N₂ (g). 3-((5-Chlorobenzo[d]thiazol-2-yl)methoxy)-2,6-difluorobenzamide was dissolved in anhydrous THF (3.5 mL) and stirred at room temperature. 1-Methylpiperidin-4-yl carbonochloridate HCl salt (19 mg, 0.09 mmol, 1.2 eq.) was added, followed by addition of sodium hydride (60% in oil dispersion) (14.8 mg, 0.37 mmol, 5.0 eq.). Stirring continued at room temperature for 1 hour and at 50 °C for 10 minutes. The reaction suspension concentrated to a solid and dissolved in EtOAc / H₂O. After stirring, the aqueous phase was separated and extracted with EtOAc. The combined EtOAc layers were dried over Na₂SO₄, filtered, and concentrated to a solid. Chromatography with (95 CH₂Cl₂ : 5 MeOH : 1 NH₄OH) and (90 CH₂Cl₂: 10 MeOH : 1 NH₄OH) isolated 1-methylpiperidin-4-yl (3-((5-chlorobenzo[d]thiazol-2-yl)methoxy)-2,6-difluorobenzoyl)carbamate as a solid (10 mg, 27%). ¹H NMR (300 MHz) (DMSO) δ: 11.55 (br, s, 1H), 8.2 (d, *J=* 8.5 Hz, 1H), 8.13 (d, *J* = 2 Hz, 1H), 7.55 (dd, *J* = 8.5 Hz, *J* = 2Hz, 1H), 7.47 (ddd, *J* = 9 Hz, J = 9 Hz, *J* = 5.1Hz, 1H), 7.16 (ddd, *J=* 9 Hz, *J=* 9 Hz, *J=* 2 Hz, 1H), 5.72 (s, 2H), 4.62 (m, 1H), 2.13 (s, 3H), 2.09 (m, 2H), 1.81 (m, 3H), 1.55 (m, 3H). MS: *m*/*e* = 496 (M+1).

### a. Preparation of Compound

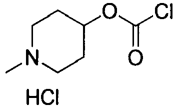

4-Hydroxy-1-methyl piperidine (0.51 mL, 4.34 mmol, 1.0 eq.) was placed under N₂ (g) and dissolved in 10 mL of anhydrous acetonitrile. The resulting solution was cooled to 0 °C in an ice/water bath. Trichloromethylchloroformate (0.68 mL, 5.64 mmol, 1.3 eq.) was added dropwise, and a suspension formed. After stirring for 30 min at 0 °C, the reaction was warmed to room temperature and stirred overnight under N₂ (g). The reaction suspension was filtered, and the collected solid was washed with acetonitrile. After further drying under vacuum, the solid was triturated with diethyl ether and collected by filtration to yield 1-methylpiperidin-4-yl carbonochloridate, HCl salt (0.52 g, 55%). ¹H NMR (300 MHz) (CD₃OD) δ: 4.95 (m,1H), 3.6 (m, 1H), 3.4 (m, 1H), 3,2 (m,2H), 2.85 (s, 3H), 2.5 (m, 1H), 2.2 (m, 2H), 1.9 (m, 1H), MS: *m*/*e* = 178 (M+1).

### Example 35

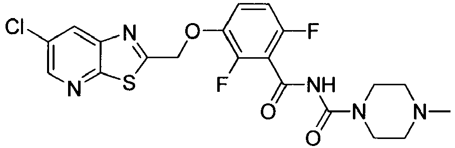

3-((6-Chlorothiazolo[5,4-b]pyridin-2-yl)methoxy)-2,6-difluorobenzamide (0.1 g, 0.281 mmol, 1.0 eq.) was suspended in 3 mL CH₂Cl₂ while stirring at room temperature. Oxalyl chloride (0.1 mL, 1.2 mmol, 4.2 eq.) was added dropwise, and stirring continued in a sealed flask at 45 °C for 20 hours. The reaction was cooled to room temperature and then concentrated to a residue. The residue was partially dissolved in 5 mL CH₂Cl₂. The suspension was cooled to - 78°C in a dry ice / acetone bath. Triethylamine (0.2 mL, 1.44 mmol, 5.1 eq.) was added dropwise and stirring continued for approximately 5 min. 1-Methylpiperazine (32 µL, 0.30 mmol, 1.1 eq.) was added dropwise and the reaction was warmed to room temperature. After stirring for 30 min, the reaction was concentrated to a brown oil, which was then dissolved in EtOAc / H₂O. The EtOAc phase was washed with brine, dried over Na₂SO₄, filtered, and concentrated to a residue. Chromatography with 0 > 10% MeOH / CH₂Cl₂ isolated the product as a solid (27.0 mg, 20%). ¹H NMR (300 MHz) (CDCl₃) δ: 8.61 (d, *J* = 2.2 Hz, 1H), 8.267 (d, 2.2 Hz, 1H), 8.199 (br. s, 1H), 7.18 (ddd, *J* = 9.0 Hz, *J* = 9.0 Hz, *J* = 5.0 Hz, 1H), 6.915 (ddd, *J* = 9.0 Hz, *J=* 9.0 Hz, *J=* 2.1 Hz, 1H), 5.505 (s, 2H), 3.59 (m, 4H), 2.51 (m, 4H), 2.36 (s, 3H). MS: *m*/*e* = 482 (M+1).

### Example 36.

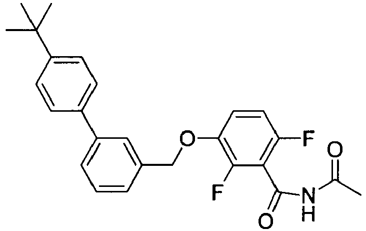

In a 2-dram vial, a suspension of 3-((4'-(*tert*-butyl)-[1,1'-biphenyl]-3-yl)methoxy)-2,6-difluorobenzamide (20 mg, 0.05 mmol) in 1 ml of N,N-dimethylacetamide dimethyl acetal was capped and stirred at 90 °C for 1 hour. The excess dimethylacetamide dimethyl acetal was removed under vacuum and the residue was treated with 70% acetic acid (1.0 mL) at room temperature for 12 hours. After the solvent was removed, the residue was purified on silica gel. Elution with 20% EtOAc/hexanes afforded the desired product as a light yellow solid (15 mg, 68% yield). ¹H NMR (CDCl₃, 300 MHz) δ: 8.40 (broad s, 1H), 7.65-7.33 (m, 8H), 7.13-7.06 (m, 1H), 6.92- 6.82 (m, 1H), 5.19 (s, 2H), 2.57 (s, 3H), 1.36 (s, 9H).

The requisite intermediates were prepared as follows

### a. Preparation of Compound

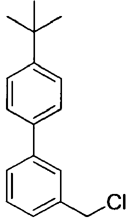

Prepared according to the literature method. See Kaul M, Parhi AK, Zhang Y, LaVoie EJ, Tuske S, Arnold E, Kerrigan JE, Pilch DS; J Med Chem. 2012 Nov 26;55(22):10160-76.

### b. Preparation of Compound

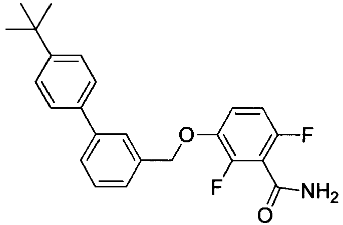

A 10-ml flask was added 4'-(*tert*-butyl)-3-(chloromethyl)-1,1'-biphenyl (20 mg, 0.08 mmol), 2,6-difluoro-3-hydroxybenzamide (14 mg, 0.08 mmol), K₂CO₃ (22 mg, 0.16 mmol), and DMF (1.5 mL). The reaction mixture was stirred at 50 °C overnight. After cooling to room temperature, the reaction mixture was diluted with EtOAc and washed with water, brine, and dried over Na₂SO₄. The organic solvent was removed and the residue was purified on silica gel. Elution with 10% EtOAc/hexanes afforded the desired product as white solid (22 mg, 72% yield). ¹H NMR (CDCl₃, 300 MHz) δ: 7.65-7.33 (m, 8H), 7.09-6.99 (m, 1H), 6.89- 6.79 (m, 1H), 5.95 (broad s, 1H), 5.86 (broad s, 1H), 5.19 (s, 2H), 1.37 (s, 9H).

### Example 37.

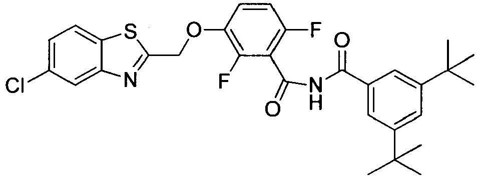

A 2-dram vial was added 3-((5-chlorobenzo[d]thiazol-2-yl)methoxy)-2,6-difluorobenzamide (25 mg, 0.07 mmol), 3,5-di-tert-butylbenzoyl chloride (18 mg, 0.07 mmol), and THF (2 mL). With stirring, NaH (9 mg, 60% in mineral oil, 0.21 mmol) was added. The resulting mixture was stirred at room temperature overnight. The solvent was removed and the residue was purified on silica gel. Elution with 30% EtOAc/hexanes afforded the desired product as white solid (14 mg, 35% yield). ¹H NMR (CDCl₃,300 MHz) δ: 9.08 (broad s, 1H), 8.02 (d, J = 2.1 Hz, 1H), 7.94 (s, 1H), 7.83 (d, *J* = 8.7 Hz, 1H), 7.68 (s, 2H), 7.41(dd, *J* = 6.7, 1.8 Hz), 7.22-7.14 (m, 1H), 6.94- 6.88 (m, 1H), 5.52 (s, 2H), 1.38 (s, 18H).

### Example 38.

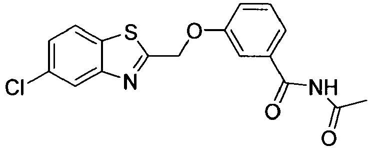

In a 2-dram vial, a suspension of 3-((5-chlorobenzo[d]thiazol-2-yl)methoxy)benzamide (20 mg, 0.05 mmol) in 1 ml of N,N-dimethylacetamide dimethyl acetal was capped and stirred at 100 °C for 1 hour. The excess dimethylacetamide dimethyl acetal was removed under vacuum and the residue was treated with 70% acetic acid (1.0 mL) at room temperature for 12 hours. After the solvent was removed, the residue was purified on silica gel. Elution with 20% EtOAc/hexanes afforded the desired product as white solid (15 mg, 54% yield). ¹H NMR (CDCl₃, 300 MHz) δ: 8.49 (broad s, 1H), 8.04(d, J = 2.4H), 7.83 (d, *J*= 9Hz, 1H), 7.56(s, 1H), 7.45-7.39 (m, 4H), 5.55 (s, 2H), 2.62 (s, 3H).

### a. Preparation of Compound

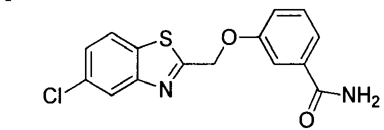

A 2-dram vial was added 2-(bromomethyl)-5-chlorobenzo[d]thiazole (42 mg, 0.16 mmol), 3-hydroxybenzamide (21 mg, 0.15 mmol), K₂CO₃ (44 mg, 0.32 mmol), and DMF (0.5 mL). The reaction mixture was stirred at 50 °C overnight. After cooling to room temperature, water was added. The yellow solid was collected by filtration and washed with water. After air drying, the solid was triturated withCH₂Cl₂. There was obtained the desired product (29 mg, 59%) as yellow solid. ¹H NMR (DMSO, 300 MHz) δ: 8.19 (d, *J=* 9.0 Hz, 1H), 8.14 (d, *J=* 3.0 Hz, 1H), 8.00 (broad s, 1H), 7.61 (s, 1H), 7.56 (s, 1H), 7.53 (s, 1H), 7.45-7.38 (m, 2H), 7.29-7.25 (m, 1H), 5.68 (s, 2H).

### Example 39.

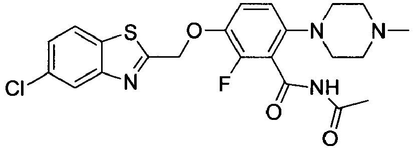

A suspension of 3-((5-chlorobenzo[*d*]thiazol-2-yl)methoxy)-2-fluoro-6-(4-methylpiperazin-1-yl)benzamide (25 mg, 0.06 mmol) in 1 ml of N,N-dimethylacetamide dimethyl acetal was stirred at 90 °C for 1 hour. The excess dimethylacetamide dimethyl acetal was removed under vacuum and the residue was treated with 70% acetic acid (1.0 mL) at room temperature for 12 hours. After the solvent was removed, the residue was purified on silica gel. Elution with 10% MeOH/EtOAc afforded the desired product as an off white solid (19 mg, 70% yield).). ¹H NMR (CDCl₃, 300 MHz) δ: 8.98 (broad s, 1H), 8.01 (d, *J=* 2.0 Hz, 1H), 7.81 (d, *J=* 8.8 Hz, 1H), 7.40(dd, *J* = 6.8, 1.8 Hz), 7.02-6.86 (m, 2H), 5.50 (s, 2H), 3.30 (broad s, 4H), 2.52 (broad s, 4H), 2.57 (s, 3H), 2.35 (s, 3H).

The requisite intermediate was prepared as follows **a. Preparation of Compound**

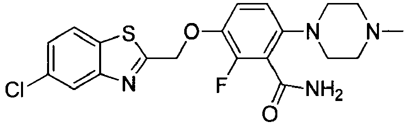

In a 2-dram vial was added 3-((5-chlorobenzo[d]thiazol-2-yl)methoxy)-2,6-difluorobenzamide (40 mg, 0.11 mmol), and 1-methylpiperazine (0.02 mL), then sealed. The reaction mixture was heated to 125 °C with stirring for 1 hour. After cooling to room temperature, water was added. The resulting solid was collected by filtration. After drying, there was obtained the desired product as a light yellow solid (42 mg, 86% yield). ¹H NMR (CDCl₃, 300 MHz) δ: 8.01 (d, *J* = 2.0 Hz, 1H), 7.82 (d, *J* = 8.8 Hz, 1H), 7.41(dd, *J* = 6.8, 1.8 Hz), 6.97-6.80 (m, 2H), 6.02 (broad s, 1H), 5.79 (broad s, 1H), 5.47 (s, 2H), 3.35-3.29 (m, 4H), 2.57-2.48 (m, 4H), 2.34 (s, 3H).

### Example 40.

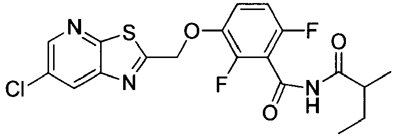

A 2-dram vial was added 3-((6-chlorothiazolo[5,4-*b*]pyridin-2-yl)methoxy)-2,6-difluorobenzamide (25 mg, 0.07 mmol), 2-methylbutanoyl chloride (9 mg, 0.07 mmol), and THF (2 mL). With stirring, NaH (9.0 mg, 60% in mineral oil, 0.21 mmol) was added. The resulting mixture was stirred at 50 °C for 1 hour. The solvent was removed and the residue was purified on silica gel. Elution with 30% EtOAc/hexanes afforded the desired product as off white solid (12 mg, 39% yield). ¹H NMR (CDCl₃, 300 MHz) δ: 8.58 (s, 1H), 8.25 (s, 1H), 8.15 (broad s, 1H), 7.22-7.12 (m, 1H), 6.95-6.86 (m, 1H), 5.48 (s, 2H), 2.81 (m, 1H), 1.76 (m, 1H), 1.22 (d, *J* = 6.6 Hz, 3H), 0.98 (t, J = 7.5 Hz).

### Example 41.

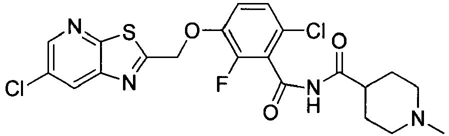

A 25-mL round bottom flask was added 6-chloro-3-((6-chlorothiazolo[5,4-*b*]pyridin-2-yl)methoxy)-2-fluorobenzamide (200 mg, 0.54 mmol), 1-methylpiperidine-4-carbonyl chloride hydrochloride (200 mg, 1.01 mmol), and THF (4 mL). With stirring, NaH (120 mg, 60% in mineral oil, 3.0 mmol) was added in several portions. After 10 min, a solution of water (20 ml) in THF(1 mL) was added via a syringe. After 10 min, the reaction was completed. It was quenched by a few drop of water, and diluted with CH₂Cl_{2.} The organic solution was washed with brine and dried over Na₂SO₄. The solvent was removed and the resulting residue was purified by ISCO using 10% MeOH in CH₂Cl₂ + 1% NH₄OH to afford a beige solid (106 mg, 40% yield). ¹H NMR (CDCl_{3,} 300 MHz) δ: 8.58 (s, 1H), 8.25 (s, 1H), 7.20-7.07 (m, 2H), 5.52 (s, 2H), 3.05-2.84 (m, 3H), 2.39 (s, 3H), 2.33-1.87 (m, 6H).

The requisite intermediate was prepared as follows

### a. Preparation of Compound

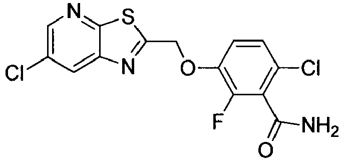

A 25-mL round bottom flask equipped with a magnetic stirrer was charged with 6-chloro-2-(chloromethyl)thiazolo[5,4-*b*]pyridine (326 mg, 1.49 mmol), DMF (4 mL), K₂CO₃ (414 mg, 3.0 mmol), and 6-chloro-2-fluoro-3-hydroxybenzamide (270 mg, 1.42 mmol). The reaction mixture was stirred at room temperature for 12 hours then water was added. The solid was collected by filtration and washed with water. After air drying, the solid was triturated with CH₂Cl₂. There was obtained the desired product (330 mg) as brown solid with 60% yield. ¹H NMR (DMSO, 300 MHz) δ: 8.74 (d, *J* = 2.1 Hz, 1H), 8.70 (d, *J* = 2.1 Hz, 1H), 8.17 (s, 1H), 7.91 (s, 1H), 7.43-7.30 (m, 2H), 5.78 (s, 2H).

### Example 42.

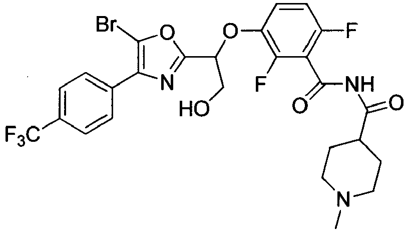

A 15-mL round bottom flask equipped with a magnetic stirrer was charged with3-(1-(5-bromo-4-(4-(trifluoromethyl)phenyl)oxazol-2-yl)-2-hydroxyethoxy)-2,6-difluorobenzamide (25 mg, 0.04 mmol, 1-methylpiperidine-4-carbonyl chloride hydrochloride (50 mg, 0.25 mmol), and THF (2 mL). With stirring NaH (25 mg, 0.60 mmol, 60% dispersion in mineral oil) was added. The resulting reaction mixture was stirred for 10 minutes, then a solution of water (4 µl) in THF (0.5 mL) was added via a syringe. After 10 min, the reaction was completed. it was quenched by the addition of few drops of water, and diluted with dichloromethane. The organic phase was separated, washed with brine and dried over Na₂SO₄. The solvent was removed in vacuo, and the resulting residue was purified by ISCO using 10 % MeOH in CH₂Cl₂ + 1% NH₄OH to afford an off white solid (11 mg, 40% yield). LC-MS: 632, 634 (M+1).

The requisite intermediates were prepared as follows

### a. Preparation of Compound

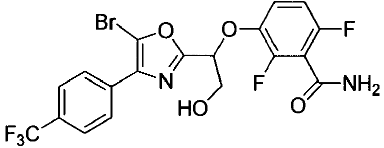

### Prepared according to the literature method:

Haydon, David John; Czaplewski, Lloyd George; Stokes, Neil Robert; Davies, David; Collins, Ian; Palmer, James T.; Mitchell, Jeffrey Peter; Pitt, Gary Robert William; Offermann, Daniel PCT Int. Appl. (2012), WO2012142671A1 2012 1026.

### Example 43.

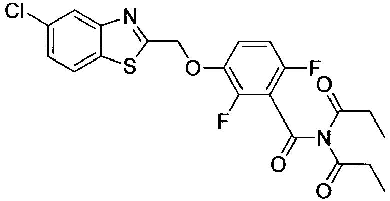

In a round bottom flask 3-((5-chlorobenzo[d]thiazol-2-yl)methoxy)-2,6-difluorobenzamide (35 mg, 0.1 mmol) was dissolved in 2 ml of dry THF, and the solution was cooled to 0 °C under nitrogen. This was followed by portion wise addition of NaH (8 mg, 0.2 mmol, 60% dispersion in mineral oil).The mixture was stirred at 0 °C for 10 minutes and at room temperature for 45 minutes. The mixture was cooled to 0 °C, and a solution of propionyl chloride (8.0 µl, 0.1 mmol) in 1 ml of THF was added drop-wise. The resulting reaction mixture was stirred at 0 °C for 10 minutes and at room temperature for 4 hours. After completion of the reaction, it was quenched by the addition of few drops of IN HCl, and diluted with ethyl acetate. The organic phase was separated, washed successively with sat. NaHCO₃, brine and dried. The solvent was removed in vacuo, and the resulting residue was purified by ISCO using 20% EtOAc in hexane as the elutant to afford the pure product as white solid (8 mg, 18% yield) along with mono acetylated product and recovered starting material. ¹H NMR (300 MHz, CDCl₃) δ: 8.047 (s, 1H), 7.85 (d, J = 8.7 Hz, 1H), 7.43 (dd, J = 8.7, 2.1 Hz, 1H), 7.22-7.16 (m, 1H), 6.93-6.87 (m, 1H), 5.53 (s, 2H), 2.77 (qt, 4H), 1.20 (t, 6H).

### Example 44.

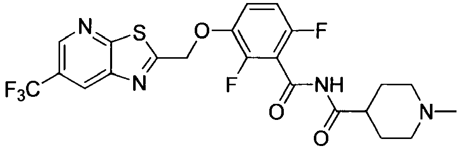

A 25-mL round bottom flask equipped with a magnetic stirrer was charged with 2,6-difluoro-3-((6-(trifluoromethyl)thiazolo[5,4-b]pyridin-2-yl)methoxy)benzamide (300 mg, 0.77 mmol, 1-methylpiperidine-4-carbonyl chloride hydrochloride (50 mg, 0.25 mmol) (300 mg, 1.51 mmol), and THF (6 mL). With stirring NaH (180 mg, 4.5 mmol, 60% dispersion in mineral oil) was added portionwise over 5 min. The resulting reaction mixture was stirred for 10 minutes, then a solution of water (30 ul) in THF (2 mL) was added via a syringe dropwise over 5 min. The reaction mixture changed from suspension to a brown solution. After completion of the reaction, it was quenched by the addition of few drops of water, and diluted with dichloromethane. The organic phase was separated, washed with brine and dried over Na₂SO₄. The solvent was removed in vacuo, and the resulting residue was purified by ISCO using 10% MeOH in CH₂Cl₂ + 1% NH₄OH to afford a light brown solid, which was triturated with EtOAc to give a beige solid (218 mg, 55% yield). ¹H NMR (300 MHz, CDCl₃) δ: 8.58 (s, 1H), 8.31 (broad s, 1H), 8.24 (s, 1H), 7.24-7.14 (m, 1H), 6.94-6.87 (m, 1H), 5.50 (s, 2H), 2.94-2.80 (m, 3H), 2.28 (s, 3H), 2.10-1.74 (m, 6H). LC-MS: 515 (M+1).

The requisite intermediates were prepared as follows **a. Preparation of Compound**

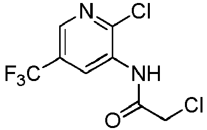

A 100-mL round bottom flask equipped with a magnetic stirrer was charged with 2-chloro-5-(trifluoromethyl)pyridine-3-amine (1.0 g , 5.1 mmol), CH₂Cl₂ (15 mL), TEA (1.42 ml, 10.2 mmol). The reaction mixture was cooled under ice-bath and chloroacetyl chloride (0.81 ml, 10.2 mmol) was slowly added. The reaction mixture was stirred at room temperature for 2 hours. The solvent was removed under vacuum. The residue was purified by column chromatography using 20% EtOAc/hexane to afford the desired product as off-white solid (1.22 g, 88% yield). ). ¹H NMR (300 MHz, CDCl₃) δ: 9.05 (s, 2H), 8.44 (s, 1H), 4.27 (s, 2H).

### b. Preparation of compound

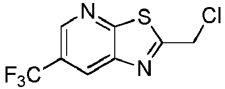

A 100-mL round bottom flask equipped with a magnetic stirrer was charged with 2-chloro-N-(2-chloro-5-(trifluoromethyl)pyridine-3-yl)acetamide (1.22 g, 4.47 mmol), P₅S₁₀ (750 mg,), and toluene (20 mL). The resulting mixture was refluxed for 30 minutes. The reaction mixture was cooled to room temperature and the solids were filtered off. The solvent was removed and the crude product was purified by column chromatography using hexanes to 5% EtOAc/hexane to afford the pure product as a light yellow solid (935 mg, 84%yield). ¹H NMR (300 MHz, CDCl₃) δ: 8.90 (s, 1H), 8.50 (s, 1H), 4.98 (s, 2H). LC-MS: 253 (M+1).

### c. Preparation of Compound

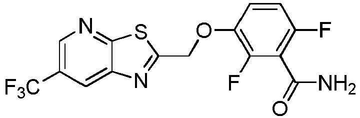

A 25-mL round bottom flask equipped with a magnetic stirrer was charged with 2-(chloromethyl)-6-(trifluoromethyl)thiazolo[5,4-b]pyridine (350 mg, 1.39 mmol), DMF (2.0 mL), NaHCO₃ (277 mg, 3.30 mmol), and 2,6-difluoro-3-hydroxybenzamide (230 mg, 1.32 mmol). The reaction mixture was heated at 50 °C overnight. After cooling to room temperature, water was added to the reaction mixture and the precipitate was collected by filtration to give a brown solid. After drying, the crude product was triturated with CH₂Cl₂ to afford the desired product as light brown solid in high purity (380 mg, 71% yield). ¹H NMR (300 MHz, DMSO-d₆) δ: 9.05 (s, 1H), 8.93 (s, 1H), 8.17 (bs, 1H), 7.89 (bs, 1H), 7.45-7.37 (m, 1H), 7.11 (m, 1H), 5.77 (s, 2H). LC-MS: 390 (M+1).

**Example 45** The following can illustrate representative pharmaceutical dosage forms, containing a compound of formula I ('Compound X') or a pharmaceutically acceptable salt thereof, for therapeutic or prophylactic use in humans. The tablets can optionally comprise an enteric coating.

| (i) Tablet 1 | mg/tablet |
|---|---|
| Compound X= | 100.0 |
| Lactose | 77.5 |
| Povidone | 15.0 |
| Croscarmellose sodium | 12.0 |
| Microcrystalline cellulose | 92.5 |
| Magnesium stearate | 3.0 |
| | 300.0 |

| (ii) Tablet 2 | mg/tablet |
|---|---|
| Compound X= | 20.0 |
| Microcrystalline cellulose | 410.0 |
| Starch | 50.0 |
| Sodium starch glycolate | 15.0 |
| Magnesium stearate | 5.0 |
| | 500.0 |

| (iii) Capsule | mg/capsule |
|---|---|
| Compound X= | 10.0 |
| Colloidal silicon dioxide | 1.5 |
| Lactose | 465.5 |
| Pregelatinized starch | 120.0 |
| Magnesium stearate | 3.0 |
| | 600.0 |

| (iv) Injection 1 (1 mg/ml) | mg/ml |
|---|---|
| Compound X= (free acid form) | 1.0 |
| Dibasic sodium phosphate | 12.0 |
| Monobasic sodium phosphate | 0.7 |
| Sodium chloride | 4.5 |
| 1.0 N Sodium hydroxide solution (pH adjustment to 7.0-7.5) | q.s. |
| Water for injection | q.s. ad 1 mL |

| (v) Injection 2 (10 mg/ml) | mg/ml |
|---|---|
| Compound X= (free acid form) | 10.0 |
| Monobasic sodium phosphate | 0.3 |
| Dibasic sodium phosphate | 1.1 |
| Polyethylene glycol 400 | 200.0 |
| 1.0 N Sodium hydroxide solution (pH adjustment to 7.0-7.5) | q.s. |
| Water for injection | q.s. ad 1 mL |

| (vi) Aerosol | mg/can |
|---|---|
| Compound X= | 20.0 |
| Oleic acid | 10.0 |
| Trichloromonofluoromethane | 5,000.0 |
| Dichlorodifluoromethane | 10,000.0 |
| Dichlorotetrafluoroethane | 5,000.0 |

The above formulations may be obtained by conventional procedures well known in the pharmaceutical art.

## Claims

1. A compound of formula (I): 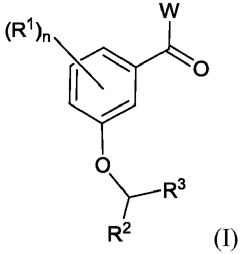 wherein:
each R¹ is independently selected from hydrogen, halo, cyano, nitro, (C₁-C₆)alkyl, (C₁-C₆)alkoxy, (C₁-C₆)alkanoyl, (C₁-C₆)alkoxycarbonyl, (C₁-C₆)alkanoyloxy, aryl, heteroaryl, heterocycle, and NR^{e}R^{f}, wherein each (C₁-C₆)alkyl, (C₁-C₆)alkoxy, (C₁-C₆)alkanoyl, (C₁-C₆)alkoxycarbonyl, (C₁-C₆)alkanoyloxy, aryl, heteroaryl, and heterocycle is optionally substituted with one or more groups independently selected from halo, cyano, nitro, NR^{e}R^{f}, -CR^{g}(=N)N(R^{g})₂, -NR^{g}C(=N)-N(R^{g})₂, -NR^{g}-C(=NR^{g})R^{g}, (C₁-C₃)alkyl , (C₁-C₃)alkoxy, (C₁-C₃)alkanoyl, (C₁-C₃)alkoxycarbonyl, (C₁-C₃)alkanoyloxy, aryl, heteroaryl, and heterocycle;
R² is H or (C₁-C₆)alkylthat is optionally substituted with one or more groups independently selected from -OR^{k}, halo, NR^{e}R^{f}, NR^{e}R^{f}, -CR^{g}(=N)N(R^{g})₂, -NR^{g}C(=N)-N(R^{g})₂, and -NR^{g}-C(=NR^{g})R^{g};
R³ is aryl or heteroaryl, which aryl or heteroaryl is optionally substituted with one or more groups independently selected from R^{h}, halo, hydroxy, -NR^{e}R^{f}, -CR^{g}(=N)N(R^{g})₂, -NR^{g}C(=N)-N(R^{g})₂, -NR^{g}-C(=NR^{g})R^{g}, (C₁-C₆)alkyl, and (C₃-C₈)cycloalkyl, wherein any (C₁-C₆)alkyl and (C₃-C₈)cycloalkyl is optionally substituted with one or more groups independently selected from halo, hydroxy, -NR^{e}R^{f}, -CR^{g}(=N)N(R^{g})₂, -NR^{g}C(=N)-N(R^{g})₂, -NR^{g}-C(=NR^{g})R^{g}, and (C₁-C₆)alkylthat is optionally substituted with one or more groups independently selected from halo;
W is -NHCOR^{a}, -N(COR^{a})(COR^{b}), -N=C(R^{c})NR^{a}R^{b}, -NR^{a}CH₂OR^{a}, -NHC(=O)OR^{a}, -NHC(=O)NR^{a}R^{b}, or -N(R^{a})SOₘR^{d};
each R^{a} is independently selected from H, aryl, heteroaryl, heterocycle, (C₃-C₈)cycloalkyl, (C₃-C₈)cycloalkyl(C₁-C₆)alkyl and (C₁-C₆)alkyl that is optionally substituted with one or more groups independently selected from hydroxy, halo, cyano, (C₁-C₆)alkoxycarbonyl, aryl, heteroaryl, -NR^{e}R^{f}, -CR^{g}(=N)N(R^{g})₂, -NR^{g}C(=N)-N(R^{g})₂, -NR^{g}-C(=NR^{g})R^{g} and heterocycle; wherein any aryl, heteroaryl, heterocycle, and (C₃-C₈)cycloalkyl(C₁-C₆)alkyl of R^{a} is optionally substituted with one or more groups independently selected from hydroxy, halo, cyano, trifluoromethoxy, (C₁-C₆)alkyl, (C₁-C₆)alkoxy, halo(C₁-C₆)alkyl, -NR^{e}R^{f}, -CR^{g}(=N)N(R^{g})₂, -NR^{g}C(=N)-N(R^{g})₂, -NR^{g}-C(=NR^{g})R^{g}, and (C₁-C₆)alkoxycarbonyl;
each R^{b} is independently selected from H and (C₁-C₆)alkyl that is optionally substituted with one or more groups independently selected from hydroxy, halo, cyano, (C₁-C₆)alkoxycarbonyl, aryl, heteroaryl, -NR^{e}R^{f}, -CR^{g}(=N)N(R^{g})₂, -NR^{g}C(=N)-N(R^{g})₂, -NR^{g}-C(=NR^{g})R^{g}, and heterocycle;
each R^{c} is independently selected from H and (C₁-C₆)alkyl that is optionally substituted with one or more groups independently selected from halo;
each R^{d} is independently selected from OH, -NH₂, -NR^{e}R^{f}, heterocycle, and (C₁-C₆)alkyl that is substituted with one or more groups independently selected from hydroxy, halo, cyano, (C₁-C₆)alkoxycarbonyl, aryl, heteroaryl, -NR^{e}R^{f}, -CH(=N)NH₂, -NHC(=N)-NH₂, -NH-C(=NH)R, and heterocycle;
each R^{e} is independently selected from H, aryl, heteroaryl, heterocycle, and (C₁-C₆)alkyl that is optionally substituted with one or more groups independently selected from hydroxy, halo, cyano, (C₁-C₆)alkoxycarbonyl, aryl, heteroaryl, and heterocycle; and each R^{f} is independently selected from H and (C₁-C₆)alkylthat is optionally substituted with one or more groups independently selected from hydroxyl, halo, cyano, (C₁-C₆)alkoxycarbonyl, aryl, heteroaryl, and heterocycle; or R^{e} and R^{f} together with the nitrogen to which they are attached form a aziridino, azetidino, morpholino, piperazino, pyrrolidino or piperidino;
each R^{g} is independently selected from H and (C₁-C₆)alkylthat is optionally substituted with one or more groups independently selected from halo;
each R^{h} is independently selected from aryl and heteroaryl, wherein any aryl and heteroaryl of R^{h} is optionally substituted with one or more groups independently selected from halo, hydroxy, -NR^{e}R^{f}, -CR^{g}(=N)N(R^{g})₂, -NR^{g}C(=N)-N(R^{g})₂, -NR^{g}-C(=NR^{g})R^{g} and (C₁-C₆)alkyl that is optionally substituted with one or more groups independently selected from hydroxy, halo, -NR^{e}R^{f}, -CR^{g}(=N)N(R^{g})₂, -NR^{g}C(=N)-N(R^{g})₂, and -NR^{g}-C(=NR^{g})R^{g};
each R^{k} is independently selected from H or (C₁-C₆)alkylthat is optionally substituted with one or more groups independently selected from hydroxy, halo, oxo, carboxy, (C₁-C₆)alkoxy, (C₁-C₆)alkoxycarbonyl, and (C₁-C₆)alkanoyloxy;
each aryl is independently a single aromatic ring or a multiple condensed ring system having 9 to 20 carbon atoms in which at least one ring is aromatic;
each cycloalkyl is independently a saturated or partially unsaturated carbocyclic ring system;
m is 0, 1, or 2; and
n is 1, 2, 3, or 4;
or a salt thereof.

2. The compound of claim 1 wherein:
each R¹ is independently selected from hydrogen, halo, cyano, nitro, (C₁-C₆)alkyl, (C₁-C₆)alkoxy, (C₁-C₆)alkanoyl, (C₁-C₆)alkoxycarbonyl, (C₁-C₆)alkanoyloxy, aryl, heteroaryl, heterocycle, and NR^{e}R^{f}, wherein each (C₁-C₆)alkyl , (C₁-C₆)alkoxy, (C₁-C₆)alkanoyl, (C₁-C₆)alkoxycarbonyl, (C₁-C₆)alkanoyloxy, aryl, heteroaryl, and heterocycle is optionally substituted with one or more groups independently selected from halo, cyano, nitro, -NR^{e}R^{f}, -CR^{g}(=N)N(R^{g})₂, -NR^{g}C(=N)-N(R^{g})₂, -NR^{g}-C(=NR^{g})R^{g}, (C₁-C₃)alkyl, (C₁-C₃)alkoxy, (C₁-C₃)alkanoyl, (C₁-C₃)alkoxycarbonyl, (C₁-C₃)alkanoyloxy, aryl, heteroaryl, and heterocycle;
R² is H or (C₁-C₆)alkylthat is optionally substituted with one or more groups independently selected from hydroxy, -NR^{e}R^{f}, -CR^{g}(=N)N(R^{g})₂, -NR^{g}C(=N)-N(R^{g})₂, and -NR^{g}-C(=NR^{g})R^{g};
R³ is aryl or heteroaryl, which aryl or heteroaryl is optionally substituted with one or more groups independently selected from R^{h}, halo, hydroxy, -NR^{e}R^{f}, -CR^{g}(=N)N(R^{g})₂, -NR^{g}C(=N)-N(R^{g})₂, -NR^{g}-C(=NR^{g})R^{g} and (C₁-C₆)alkylthat is optionally substituted with one or more groups independently selected from hydroxy, -NR^{e}R^{f}, -CR^{g}(=N)N(R^{g})₂, -NR^{g}C(=N)-N(R^{g})₂, and -NR^{g}-C(=NR^{g})R^{g};
W is -NHCOR^{a}, -N(COR^{a})(COR^{b}), -N=C(R^{c})NR^{a}R^{b}, -NR^{a}CH₂OR^{a}, or -N(R^{a})SOₘR^{d};
each R^{a} is independently selected from H, aryl, heteroaryl, heterocycle, (C₃-C₈)cycloalkyl, (C₃-C₈)cycloalkyl(C₁-C₆)alkyl and (C₁-C₆)alkyl that is optionally substituted with one or more groups independently selected from hydroxyl, halo, cyano, (C₁-C₆)alkoxycarbonyl, aryl, heteroaryl, -NR^{e}R^{f}, -CR^{g}(=N)N(R^{g})₂, -NR^{g}C(=N)-N(R^{g})₂, -NR^{g}-C(=NR^{g})R^{g} and heterocycle; wherein any aryl, heteroaryl, heterocycle, and (C₃-C₈)cycloalkyl(C₁-C₆)alkyl of R^{a} is optionally substituted with one or more groups independently selected from hydroxy, halo, cyano, trifluoromethoxy, (C₁-C₆)alkyl, halo(C₁-C₆)alkyl, -NR^{e}R^{f}, -CR^{g}(=N)N(R^{g})₂, -NR^{g}C(=N)-N(R^{g})₂, -NR^{g}-C(=NR^{g})R^{g}, and (C₁-C₆)alkoxycarbonyl;
each R^{b} is independently selected from H and (C₁-C₆)alkyl that is optionally substituted with one or more groups independently selected from hydroxyl, halo, cyano, (C₁-C₆)alkoxycarbonyl, aryl, heteroaryl, -NR^{e}R^{f}, -CR^{g}(=N)N(R^{g})₂, -NR^{g}C(=N)-N(R^{g})₂, -NR^{g}-C(=NR^{g})R^{g}, and heterocycle;
each R^{c} is independently selected from H and (C₁-C₆)alkyl;
each R^{d} is independently selected from OH, -NH₂, -NR^{e}R^{f}, heterocycle, and (C₁-C₆)alkyl that is substituted with one or more groups independently selected from hydroxy, halo, cyano, (C₁-C₆)alkoxycarbonyl, aryl, heteroaryl, -NR^{e}R^{f}, -CH(=N)NH₂, -NHC(=N)-NH₂, -NH-C(=NH)R, and heterocycle;
each R^{e} is independently selected from H, aryl, heteroaryl, heterocycle, and (C₁-C₆)alkyl that is optionally substituted with one or more groups independently selected from hydroxyl, halo, cyano, (C₁-C₆)alkoxycarbonyl, aryl, heteroaryl, and heterocycle; and each R^{f} is independently selected from H and (C₁-C₆)alkyl that is optionally substituted with one or more groups independently selected from hydroxyl, halo, cyano, (C₁-C₆)alkoxycarbonyl, aryl, heteroaryl, and heterocycle; or R^{e} and R^{f} together with the nitrogen to which they are attached form a aziridino, azetidino, morpholino, piperazino, pyrrolidino or piperidino;
each R^{g} is independently selected from H and (C₁-C₆)alkyl;
each R^{h} is independently selected from aryl and heteroaryl, wherein any aryl and heteroaryl of R^{h} is optionally substituted with one or more groups independently selected from halo, hydroxy, -NR^{e}R^{f}, -CR^{g}(=N)N(R^{g})₂, -NR^{g}C(=N)-N(R^{g})₂, -NR^{g}-C(=NR^{g})R^{g} and (C₁-C₆)alkyl that is optionally substituted with one or more groups independently selected from hydroxy, -NR^{e}R^{f}, -CR^{g}(=N)N(R^{g})₂, -NR^{g}C(=N)-N(R^{g})₂, and -NR^{g}-C(=NR^{g})R^{g};
m is 0, 1, or 2; and
n is 1, 2, 3, or 4;
or a salt thereof.

3. The compound of any one of claims 1 or 2 wherein R³ is heteroaryl, which is optionally substituted with one or more groups independently selected from R^{h}, halo, hydroxy, -NR^{e}R^{f}, -CR^{g}(=N)N(R^{g})₂, -NR^{g}C(=N)-N(R^{g})₂, -NR^{g}-C(=NR^{g})R^{g}, (C₁-C₆)alkyl, and (C₃-C₈)cycloalkyl, wherein any (C₁-C₆)alkyl and (C₃-C₈)cycloalkyl is optionally substituted with one or more groups independently selected from halo, hydroxy, -NR^{e}R^{f}, -CR^{g}(=N)N(R^{g})₂, - NR^{g}C(=N)-N(R^{g})₂, -NR^{g}-C(=NR^{g})R^{g}, and (C₁-C₆)alkyl that is optionally substituted with one or more groups independently selected from halo.

4. The compound of any one of claims 1 or 2 wherein R³ is: 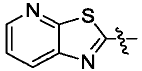 which is optionally substituted with one or more groups independently selected from R^{h}, halo, hydroxy, -NR^{e}R^{f}, -CR^{g}(=N)N(R^{g})₂, -NR^{g}C(=N)-N(R^{g})₂, -NR^{g}-C(=NR^{g})R^{g}, (C₁-C₆)alkyl, and (C₃-C₈)cycloalkyl, wherein any (C₁-C₆)alkyl and (C₃-C₈)cycloalkyl is optionally substituted with one or more groups independently selected from halo, hydroxy, -NR^{e}R^{f}, -CR^{g}(=N)N(R^{g})₂, - NR^{g}C(=N)-N(R^{g})₂, -NR^{g}-C(=NR^{g})R^{g}, and (C₁-C₆)alkyl that is optionally substituted with one or more groups independently selected from halo.

5. The compound of any one of claims 1 or 2 wherein R³ is: 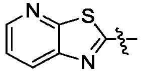 which is optionally substituted with one or more groups independently selected from (C₁-C₆)alkyl, and (C₃-C₈)cycloalkyl, wherein any (C₁-C₆)alkyl and (C₃-C₈)cycloalkyl is optionally substituted with one or more groups independently selected from halo, hydroxy, and (C₁-C₆)alkyl that is optionally substituted with one or more groups independently selected from halo.

6. The compound of any one of claims 1 or 2 wherein R³ is: 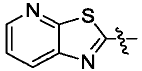 which is substituted with one or more groups independently selected from (C₁-C₆)alkyl, and (C₃-C₈)cycloalkyl, wherein any (C₁-C₆)alkyland (C₃-C₈)cycloalkyl is optionally substituted with one or more groups independently selected from halo and (C₁-C₆)alkyl that is optionally substituted with one or more groups independently selected from halo.

7. The compound of any one of claims 1 or 2 wherein R³ is: 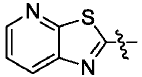 which is substituted with one or more groups independently selected from trifluoromethyl, pentafluoroethyl, or 1-(trifluoromethyl)cyclopropyl.

8. The compound of any one of claims 1 or 2 wherein R³ is: 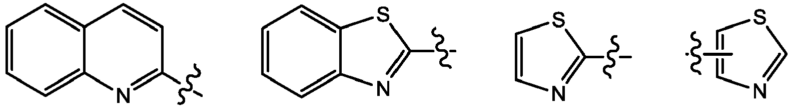 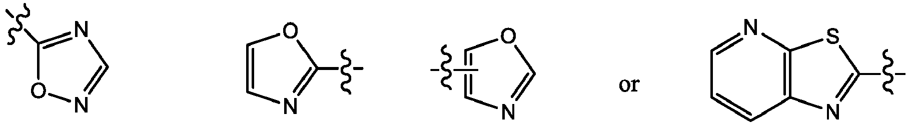 which is optionally substituted with one or more groups independently selected from R^{h}, halo, hydroxy, -NR^{e}R^{f}, -CR^{g}(=N)N(R^{g})₂, -NR^{g}C(=N)-N(R^{g})₂, -NR^{g}-C(=NR^{g})R^{g}, (C₁-C₆)alkyl, and (C₃-C₈)cycloalkyl, wherein any (C₁-C₆)alkyland (C₃-C₈)cycloalkyl is optionally substituted with one or more groups independently selected from halo, hydroxy, -NR^{e}R^{f}, -CR^{g}(=N)N(R^{g})₂, -NR^{g}C(=N)-N(R^{g})₂, -NR^{g}-C(=NR^{g})R^{g}, and (C₁-C₆)alkyl that is optionally substituted with one or more groups independently selected from halo.

9. The compound of any one of claims 1 or 2 wherein R³ is: 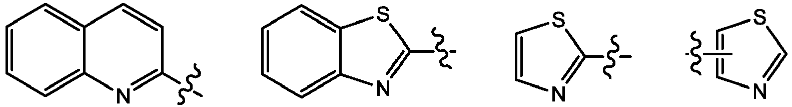 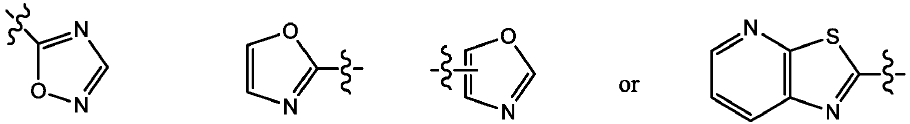 which is optionally substituted with one or more groups independently selected from trifluoromethyl, pentafluoroethyl, or 1-(trifluoromethyl)cyclopropyl.

10. The compound of any one of claims 1 or 2 wherein: 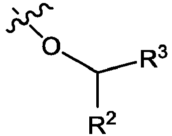 is selected from:

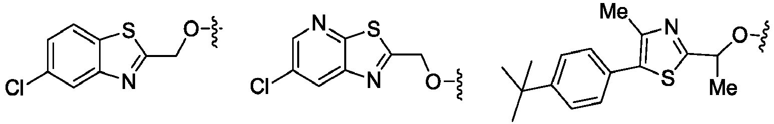

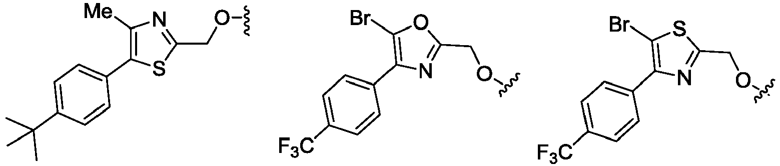

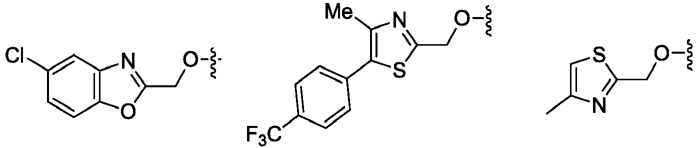

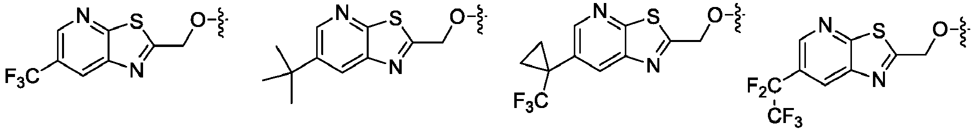

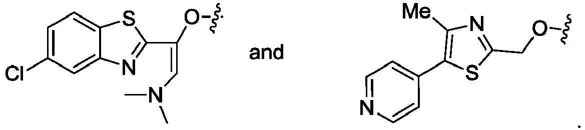

11. The compound of any one of claims 1-2 wherein W is -NHC(=O)H, - NHC(=O)CH₃, -NHC(=O)CH₂CH₃, -NHC(=O)CH₂CH₂CH₃, -N=NCH-N(CH₃)₂, -NHCH₂OH, -N=NC(CH₃)-N(CH₃)₂,

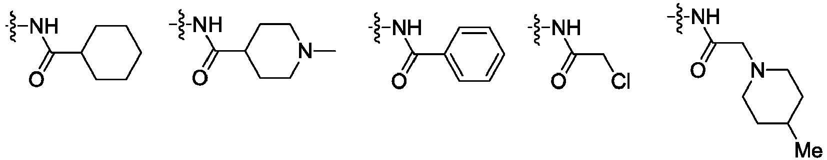

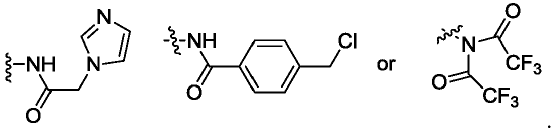

12. The compound of any one of claims 1 or 2 which is a compound of formula (Ia): 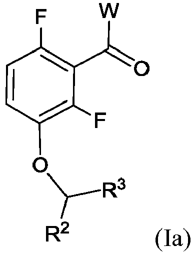 or a salt thereof.

13. A compound of claim 1 which is selected from: 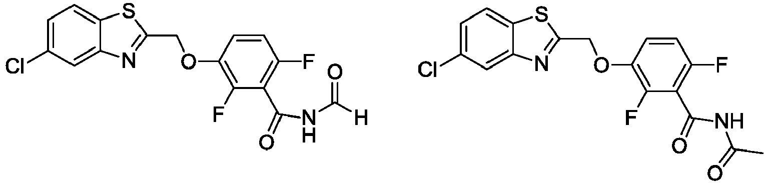 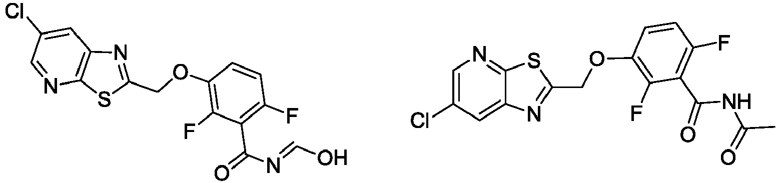 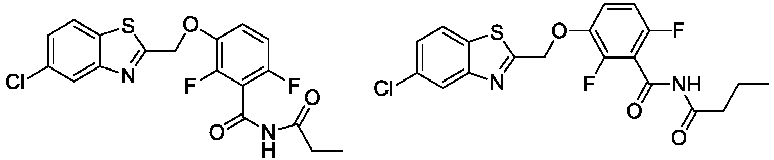 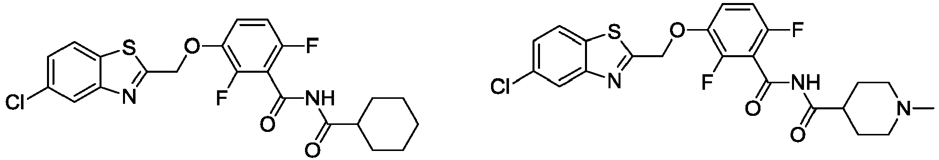 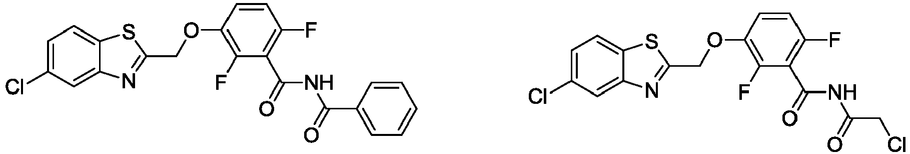 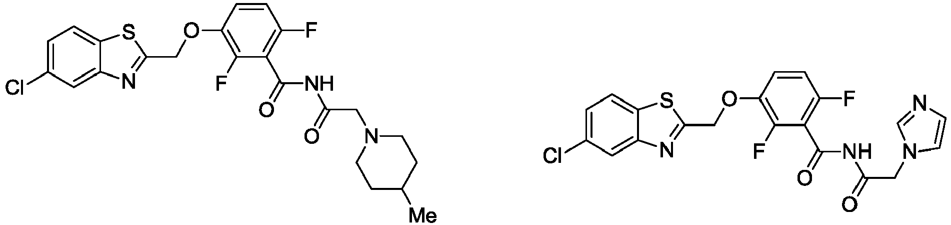 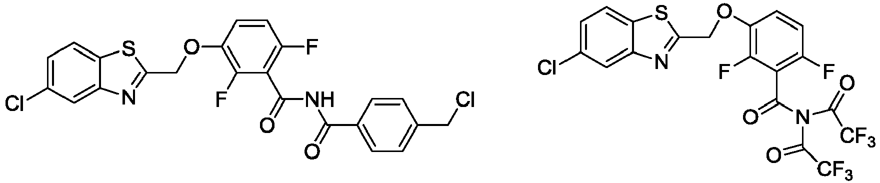 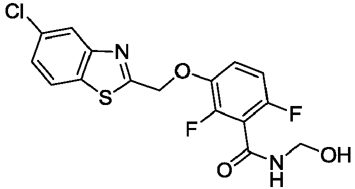 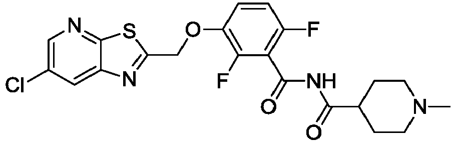 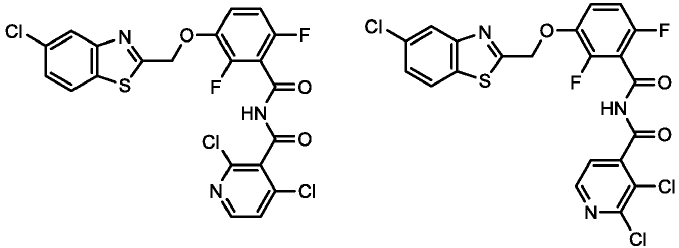 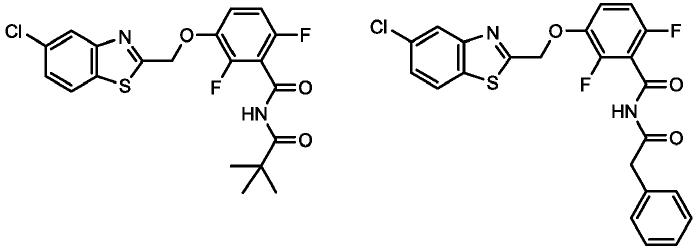 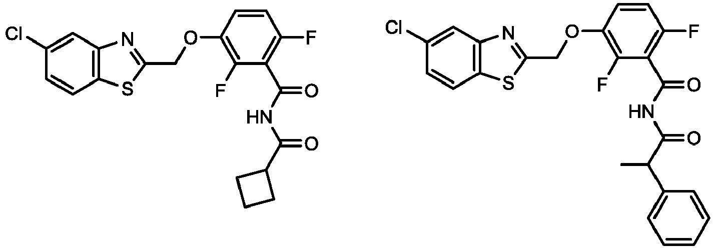 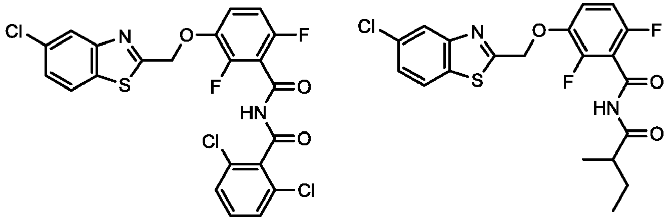 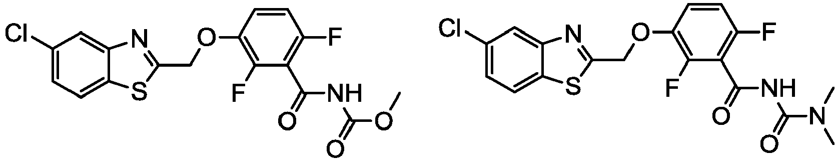 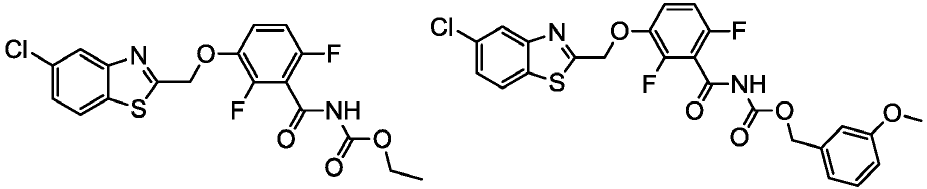 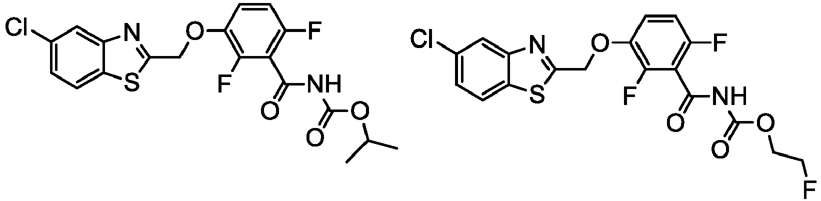 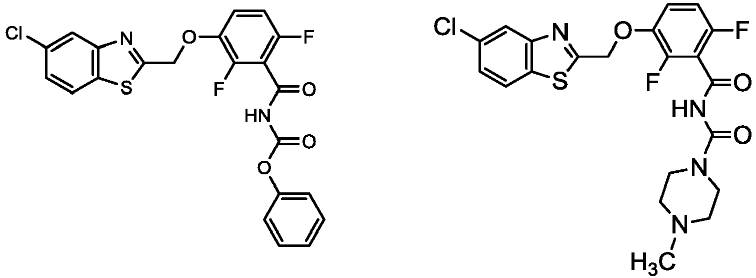 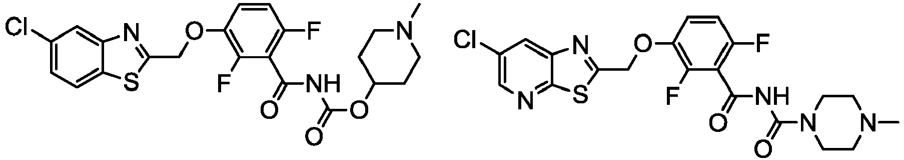 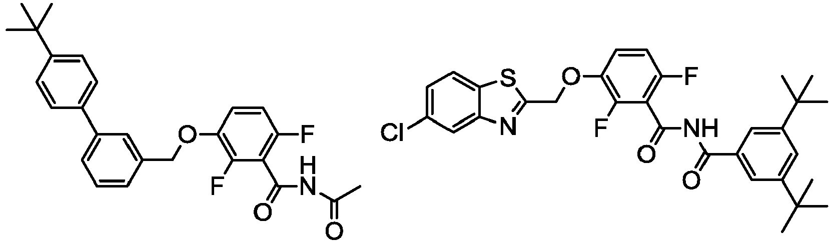 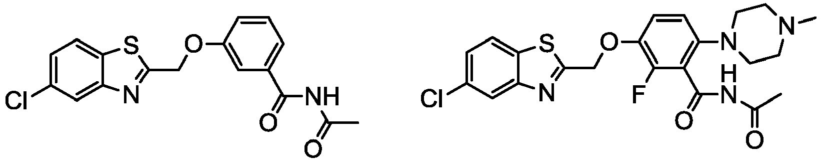 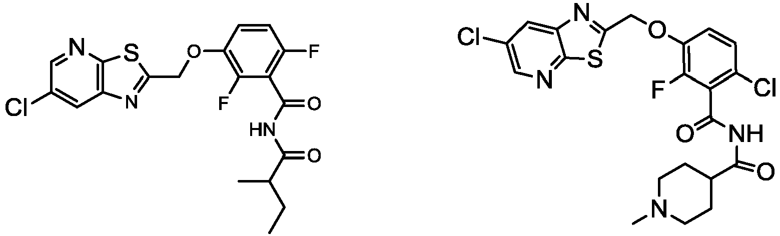 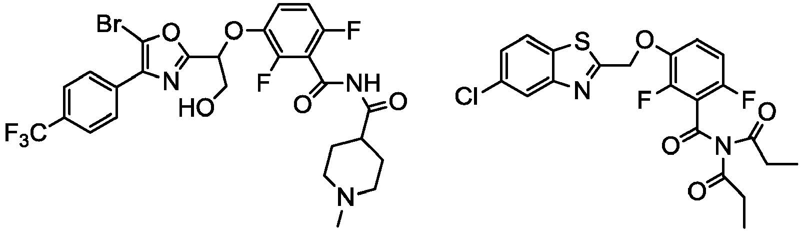 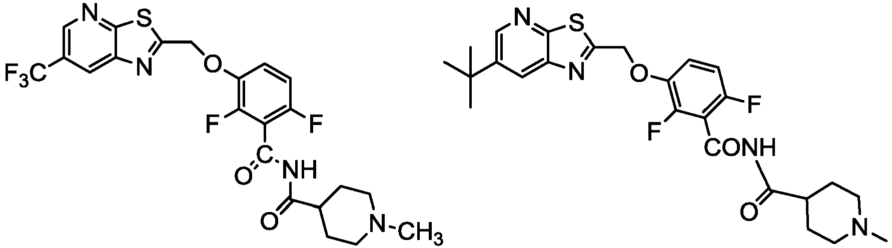 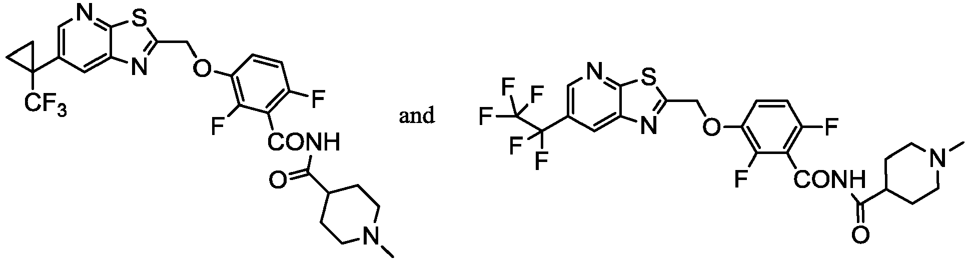 and salts thereof.

14. A compound of claim 1 which is 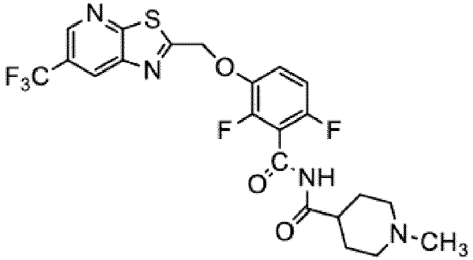 or a salt thereof.

15. A pharmaceutical composition comprising a compound of formula I as described in any one of claims 1-14 or a pharmaceutically acceptable salt thereof, and a pharmaceutically acceptable vehicle.

16. A compound of formula I as described in any one of claims 1-14 or a pharmaceutically acceptable salt thereof for use in the prophylactic or therapeutic treatment of a bacterial infection.

17. A compound of formula I as described in any one of claims 1-14 or a pharmaceutically acceptable salt thereof for use in medical treatment.

18. A method for preparing the compound of claim 14 comprising converting an amide of formula: 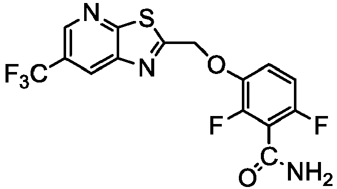 with 1-methylpiperidine-4-carbonyl chloride in the presence of NaH.

19. Amide of formula:

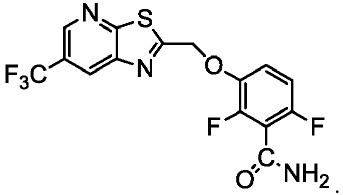

## Patentansprüche

1. Verbindung der Formel (I): 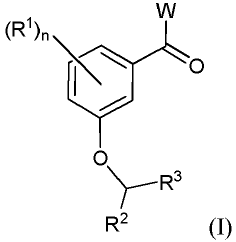 worin:
jedes R¹ unabhängig ausgewählt ist unter Wasserstoff, Halogen, Cyano, Nitro, (C₁-C₆)Alkyl, (C₁-C₆)Alkoxy, (C₁-C₆)Alkanoyl, (C₁-C₆)Alkoxycarbonyl, (C₁-C₆)Alkanoyloxy, Aryl, Heteroaryl, Heterozyklus und NR^{e}R^{f}, wobei jedes (C₁-C₆)Alkyl, (C₁-C₆)Alkoxy, (C₁-C₆)Alkanoyl, (C₁-C₆)Alkoxycarbonyl, (C₁-C₆)Alkanoyloxy, Aryl, Heteroaryl und Heterozyklus optional mit einer oder mehreren, unabhängig unter Halogen, Cyano, Nitro, NR^{e}R^{f}, -CR^{g}(=N)N(R^{g})₂, -NR^{g}C(=N)-N(R^{g})₂, -NR^{g}-C(=NR^{g})R^{g}, (C₁-C₃)Alkyl, (C₁-C₃)Alkoxy, (C₁-C₃)Alkanoyl, (C₁-C₃)Alkoxycarbonyl, (C₁-C₃)Alkanoyloxy, Aryl, Heteroaryl und Heterozyklus ausgewählten Gruppen substituiert ist;
R² für H oder (C₁-C₆)Alkyl steht, welches optional ist mit einer oder mehreren, unabhängig unter -OR^{k}, Halogen, NR^{e}R^{f}, -CR^{g}(=N)N(R^{g})₂, -NR⁸C(=N)-N(R⁸)₂ und -NR^{g}-C(=NR^{g})R^{g} ausgewählten Gruppen substituiert ist;
R³ für Aryl oder Heteroaryl steht, welches Aryl oder Heteroaryl optional mit einer oder mehreren, unabhängig unter R^{h}, Halogen, Hydroxy, -NR^{e}R^{f}, -CR^{g}(=N)N(R^{g})₂, -NR⁸C(=N)-N(R⁸)₂, -NR^{g}-C(=NR^{g})R^{g}, (C₁-C₆)Alkyl und (C₃-C₈)Cycloalkyl ausgewählten Gruppen substituiert ist, wobei jedes (C₁-C₆)Alkyl und (C₃-C₈)Cycloalkyl optional mit einer oder mehreren, unabhängig unter Halogen, Hydroxy, -NR^{e}R^{f}, -CR^{g}(=N)N(R^{g})₂, -NR^{g}C(=N)-N(R^{g})₂, -NR^{g}-C(=NR^{g})R^{g} und (C₁-C₆)Alkyl ausgewählten Gruppen substituiert ist, welche optional mit einer oder mehreren, unabhängig unter Halogen ausgewählten Gruppen substituiert ist;
W für -NHCOR^{a}, -N(COR^{a})(COR^{b}), -N=C(R^{c})NR^{a}R^{b}, -NR^{a}CH₂OR^{a}, -NHC(=O)OR^{a}, -NHC(=O)NR^{a}R^{b} oder -N(R^{a})SOₘR^{d} steht;
jedes R^{a} unabhängig ausgewählt ist unter H, Aryl, Heteroaryl, Heterozyklus, (C₃-C₈)Cycloalkyl, (C₃-C₈)Cycloalkyl-(C₁-C₆)alkyl und (C₁-C₆)Alkyl, welches optional mit einer oder mehreren, unabhängig unter Hydroxy, Halogen, Cyano, (C₁-C₆)Alkoxycarbonyl, Aryl, Heteroaryl, -NR^{e}R^{f}, -CR^{g}(=N)N(R^{g})₂, -NR^{g}C(=N)-N(R^{g})₂, -NR^{g}-C(=NR^{g})R^{g} und Heterozyklus ausgewählten Gruppen substituiert ist; wobei jedes Aryl, Heteroaryl, Heterozyklus und (C₃-C₈)Cycloalkyl-(C₁-C₆)alkyl von R^{a} optional mit einer oder mehreren, unabhängig unter Hydroxy, Halogen, Cyano, Trifluoromethoxy, (C₁-C₆)Alkyl, (C₁-C₆)Alkoxy, Halo(C₁-C₆)alkyl, -NR^{e}R^{f}, -CR^{g}(=N)N(R^{g})₂, -NR^{g}C(=N)-N(R^{g})₂, -NR^{g}-C(=NR^{g})R^{g} und (C₁-C₆)Alkoxycarbonyl ausgewählten Gruppen substituiert ist;
jedes R^{b} unabhängig ausgewählt ist unter H und (C₁-C₆)Alkyl, welches optional mit einer oder mehreren, unabhängig unter Hydroxy, Halogen, Cyano, (C₁-C₆)Alkoxycarbonyl, Aryl, Heteroaryl, -NR^{e}R^{f}, -CR^{g}(=N)N(R^{g})₂, -NR^{g}C(=N)-N(R^{g})₂, -NR^{g}-C(=NR^{g})R^{g} und Heterozyklus ausgewählten Gruppen substituiert ist;
jedes R^{c} unabhängig ausgewählt ist unter H und (C₁-C₆)Alky, welches optional mit einer oder mehreren, unabhängig unter Halogen ausgewählten Gruppen substituiert ist;
jedes R^{d} unabhängig ausgewählt ist unter -OH, -NH₂, -NR^{e}R^{f}, Heterozyklus und (C₁-C₆)Alkyl, welches mit einer oder mehreren, unabhängig unter Hydroxy, Halogen, Cyano, (C₁-C₆)Alkoxycarbonyl, Aryl, Heteroaryl, -NR^{e}R^{f}, -CH(=N)NH₂, -NHC(=N)-NH₂, -NH-C(=NH)R und Heterozyklus ausgewählten Gruppen substituiert ist;
jedes R^{e} unabhängig ausgewählt ist unter H, Aryl, Heteroaryl, Heterozyklus und (C₁-C₆)Alkyl, welches optional mit einer oder mehreren, unabhängig unter Hydroxy, Halogen, Cyano, (C₁-C₆)Alkoxycarbonyl, Aryl, Heteroaryl und Heterozyklus ausgewählten Gruppen substituiert ist; und jedes R^{f} unabhängig ausgewählt ist unter H und (C₁-C₆)Alkyl, welches optional mit einer oder mehreren, unabhängig unter Hydroxyl, Halogen, Cyano, (C₁-C₆)Alkoxycarbonyl, Aryl, Heteroaryl und Heterozyklus ausgewählten Gruppen substituiert ist; oder R^{e} und R^{f} zusammen mit dem Stickstoff, an das sie gebunden sind, einen Aziridino, Azetidino, Morpholino, Piperazino, Pyrrolidino oder Piperidino;
jedes R^{g} unabhängig ausgewählt ist unter H und (C₁-C₆)Alkyl, welches optional mit einer oder mehreren, unabhängig unter Halogen ausgewählten Gruppen substituiert ist;
jedes R^{h} unabhängig ausgewählt ist unter Aryl und Heteroaryl, wobei jedes Aryl und Heteroaryl von R^{h} optional mit einer oder mehreren, unabhängig unter Halogen, Hydroxy, -NR^{e}R^{f}, -CR^{g}(=N)N(R^{g})₂, -NR^{g}C(=N)-N(R^{g})₂, -NR^{g}-C(=NR^{g})R^{g} und (C₁-C₆)Alkyl ausgewählten Gruppen substituiert ist, welche optional mit einer oder mehreren, unabhängig unter Hydroxy, Halogen, -NR^{e}R^{f}, -CR^{g}(=N)N(R^{g})₂, -NR^{g}C(=N)-N(R^{g})₂ und -NR^{g}-C(=NR^{g})R^{g} ausgewählten Gruppen substituiert ist;
jedes R^{k} unabhängig ausgewählt ist unter H oder (C₁-C₆)Alkyl, welches optional mit einer oder mehreren, unabhängig unter Hydroxy, Halogen, Oxo, Carboxy, (C₁-C₆)Alkoxy, (C₁-C₆)Alkoxycarbonyl und (C₁-C₆)Alkanoyloxy ausgewählten Gruppen substituiert ist;
jedes Aryl unabhängig ein einzelner armotisches Ring oder ein mehrfach kondensiertes Ringsystem ist, bestehend aus 9 bis 20 Kohlenstoffatomen, in dem mindestens ein Ring aromatisch ist;
jedes Cycloalkyl unabhängig ein gesättigtes oder teilweise ungesättigtes carbozyklisches Ringsystem ist;
m ist 0, 1, oder 2; und
n ist 1, 2, 3, oder 4;
oder ein Salz davon.

2. Verbindung nach Anspruch 1, worin:
jedes R¹ unabhängig ausgewählt ist unter Wasserstoff, Halogen, Cyano, Nitro, (C₁-C₆)Alkyl, (C₁-C₆)Alkoxy, (C₁-C₆)Alkanoyl, (C₁-C₆)Alkoxycarbonyl, (C₁-C₆)Alkanoyloxy, Aryl, Heteroaryl, Heterozyklus und NR^{e}R^{f}, wobei jedes (C₁-C₆)Alkyl, (C₁-C₆)Alkoxy, (C₁-C₆)Alkanoyl, (C₁-C₆)Alkoxycarbonyl, (C₁-C₆)Alkanoyloxy, Aryl, Heteroaryl und Heterozyklus optional mit einer oder mehreren, unabhängig unter Halogen, Cyano, Nitro, -NR^{e}R^{f}, -CR^{g}(=N)N(R^{g})₂, -NR^{g}C(=N)-N(R^{g})₂, -NR^{g}-C(=NR^{g})R^{g}, (C₁-C₃)Alkyl, (C₁-C₃)Alkoxy, (C₁-C₃)Alkanoyl, (C₁-C₃)Alkoxycarbonyl, (C₁-C₃)Alkanoyloxy, Aryl, Heteroaryl und Heterozylus ausgewählten Gruppen substituiert ist;
R² für H oder (C₁-C₆)Alkyl steht, welches ist mit einer oder mehreren, unabhängig unter Hydroxy, -NR^{e}R^{f}, -CR^{g}(=N)N(R^{g})₂, -NR^{g}C(=N)-N(R^{g})₂ und -NR^{g}-C(=NR^{g})R^{g} ausgewählten Gruppen optional substituiert;
R³ für Aryl oder Heteroaryl steht, welches Aryl oder Heteroaryl mit einer oder mehreren, unabhängig unter R^{h}, Halogen, Hydroxy, -NR^{e}R^{f}, -CR^{g}(=N)N(R^{g})₂, -NR^{g}C(=N)-N(R^{g})₂, -NR^{g}-C(=NR^{g})R^{g} und (C₁-C₆)Alkyl ausgewählten Gruppen optional substituiert ist, welche optional mit einer oder mehreren, unabhängig unter Hydroxy, -NR^{e}R^{f}, -CR^{g}(=N)N(R^{g})₂, -NR^{g}C(=N)-N(R^{g})₂ und -NR^{g}-C(=NR^{g})R^{g} ausgewählten Gruppen substituiert ist;
W für -NHCOR^{a}, -N(COR^{a})(COR^{b}), -N=C(R^{c})NR^{a}R^{b}, -NR^{a}CH₂OR^{a} oder -N(R^{a})SOₘR^{d} steht;
jedes R^{a} unabhängig ausgewählt ist unter H, Aryl, Heteroaryl, Heterozyklus, (C₃-C₈)Cycloalkyl, (C₃-C₈)Cycloalkyl-(C₁-C₆)alkyl und (C₁-C₆)Alkyl, welches optional mit einer oder mehreren, unabhängig unter Hydroxyl, Halogen, Cyano, (C₁-C₆)Alkoxycarbonyl, Aryl, Heteroaryl, -NR^{e}R^{f}, -CR^{g}(=N)N(R^{g})₂, -NR^{g}C(=N)-N(R^{g})₂, -NR^{g}-C(=NR^{g})R^{g} und Heterozyklus ausgewählten Gruppen substituiert ist; wobei jedes Aryl, Heteroaryl, Heterozyklus und (C₃-C₈)Cycloalkyl-(C₁-C₆)alkyl von R^{a} optional mit einer oder mehreren, unabhängig unter Hydroxy, Halogen, Cyano, Trifluoromethoxy, (C₁-C₆)Alkyl, Halo(C₁-C₆)alkyl, -NR^{e}R^{f}, -CR^{g}(=N)N(R^{g})₂, - NR^{g}C(=N)-N(R^{g})₂, -NR^{g}-C(=NR^{g})R^{g} und (C₁-C₆)Alkoxycarbonyl ausgewählten Gruppen substituiert ist;
jedes R^{b} unabhängig ausgewählt ist unter H und (C₁-C₆)Alkyl, welches optional mit einer oder mehreren, unabhängig unter Hydroxyl, Halogen, Cyano, (C₁-C₆)Alkoxycarbonyl, Aryl, Heteroaryl, -NR^{e}R^{f}, -CR^{g}(=N)N(R^{g})₂, -NR^{g}C(=N)-N(R^{g})₂, -NR^{g}-C(=NR^{g})R^{g} und Heterozyklus ausgewählten Gruppen substituiert ist;
jedes R^{c} unabhängig ausgewählt ist unter H und (C₁-C₆)Alkyl;
jedes R^{d} unabhängig ausgewählt ist unter -OH, -NH₂, -NR^{e}R^{f}, Heterozyklus und (C₁-C₆)Alkyl, welches optional mit einer oder mehreren, unabhängig unter Hydroxy, Halo, Cyano, (C₁-C₆)Alkoxycarbonyl, Aryl, Heteroaryl, -NR^{e}R^{f}, -CH(=N)NH₂, -NHC(=N)-NH₂, -NH-C(=NH)R und Heterozyklus ausgewählten Gruppen substituiert ist;
jedes R^{e} unabhängig ausgewählt ist unter H, Aryl, Heteroaryl, Heterozyklus und (C₁-C₆)Alkyl, welches optional mit einer oder mehreren, unabhängig unter Hydroxyl, Halogen, Cyano, (C₁-C₆)Alkoxycarbonyl, Aryl, Heteroaryl und Heterozyklus ausgewählten Gruppen substituiert ist; und jedes R^{f} unabhängig ausgewählt ist unter H und (C₁-C₆)Alkyl, welches optional mit einer oder mehreren, unabhängig unter Hydroxyl, Halogen, Cyano, (C₁-C₆)Alkoxycarbonyl, Aryl, Heteroaryl und Heterozyklus ausgewählten Gruppen substituiert ist; oder R^{e} und R^{f} zusammen mit dem Stickstoff, an das sie gebunden sind, einen Aziridino, Azetidino, Morpholino, Piperazino, Pyrrolidino oder Piperidino;
jedes R^{g} unabhängig ausgewählt ist unter H und (C₁-C₆)Alkyl;
jedes R^{h} unabhängig ausgewählt ist unter Aryl und Heteroaryl, wobei jedes Aryl und Heteroaryl von R^{h} optional mit einer oder mehreren, unabhängig unter Halogen, Hydroxy, -NR^{e}R^{f}, -CR^{g}(=N)N(R^{g})₂, -NR^{g}C(=N)-N(R^{g})₂, -NR^{g}-C(=NR^{g})R^{g} und (C₁-C₆)Alkyl ausgewählten Gruppen substituiert ist, welche optional mit einer oder mehreren, unabhängig unter Hydroxy, -NR^{e}R^{f}, -CR^{g}(=N)N(R^{g})₂, -NR^{g}C(=N)-N(R^{g})₂ und -NR^{g}-C(=NR^{g})R^{g} ausgewählten Gruppen substituiert ist;
m ist 0, 1, oder 2; und
n ist 1, 2, 3, oder 4;
oder ein Salz davon.

3. Verbindung nach einem der Ansprüche 1 oder 2, worin R³ für Heteroaryl steht, welches optional mit einer oder mehreren, unabhängig unter R^{h}, Halogen, Hydroxy, -NR^{e}R^{f}, -CR^{g}(=N)N(R^{g})₂, -NR^{g}C(=N)-N(R^{g})₂, -NR^{g}-C(=NR^{g})R^{g}, (C₁-C₆)Alkyl und (C₃-C₈)Cycloalkyl ausgewählten Gruppen substituiert ist, wobei jedes (C₁-C₆)Alkyl und (C₃-C₈)Cycloalkyl optional mit einer oder mehreren, unabhängig unter Halogen, Hydroxy, -NR^{e}R^{f}, -CR^{g}(=N)N(R^{g})₂, -NR^{g}C(=N)-N(R^{g})₂, -NR^{g}-C(=NR^{g})R^{g} und (C₁-C₆)Alkyl ausgewählten Gruppen substituiert ist, welche optional mit einer oder mehreren, unabhängig unter Halogen ausgewählten Gruppen substituiert ist.

4. Verbindung nach einem der Ansprüche 1 oder 2, worin R³ für 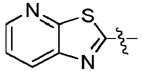 steht, welches optional mit einer oder mehreren, unabhängig unter R^{h}, Halogen, Hydroxy, -NR^{e}R^{f}, -CR^{g}(=N)N(R^{g})₂, -NR^{g}C(=N)-N(R^{g})₂, -NR^{g}-C(=NR^{g})R^{g}, (C₁-C₆)Alkyl und (C₃-C₈)Cycloalkyl ausgewählten Gruppen substituiert ist, wobei jedes (C₁-C₆)Alkyl und (C₃-C₈)Cycloalkyl optional mit einer oder mehreren, unabhängig unter Halogen, Hydroxy, -NR^{e}R^{f}, -CR^{g}(=N)N(R^{g})₂, -NR^{g}C(=N)-N(R^{g})₂, -NR^{g}-C(=NR^{g})R^{g} und (C₁-C₆)Alkyl ausgewählten Gruppen substituiert ist, welche optional mit einer oder mehreren, unabhängig unter Halogen ausgewählten Gruppen substituiert ist.

5. Verbindung nach einem der Ansprüche 1 oder 2, worin R³ für 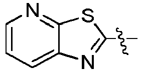 steht, welches optional mit einer oder mehreren, unabhängig unter (C₁-C₆)Alkyl und (C₃-C₈)Cycloalkyl ausgewählten Gruppen substituiert ist, wobei jedes (C₁-C₆)Alkyl und (C₃-C₈)Cycloalkyl optional mit einer oder mehreren, unabhängig unter Halogen, Hydroxy und (C₁-C₆)Alkyl ausgewählten Gruppen substituiert ist, welche optional mit einer oder mehreren, unabhängig unter Halogen ausgewählten Gruppen substituiert ist.

6. Verbindung nach einem der Ansprüche 1 oder 2, worin R³ für 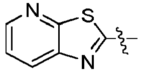 steht, welches mit einer oder mehreren, unabhängig unter (C₁-C₆)Alkyl und (C₃-C₈)Cycloalkyl ausgewählten Gruppen substituiert ist, wobei jedes (C₁-C₆)Alkyl und (C₃-C₈)Cycloalkyl optional mit einer oder mehreren, unabhängig unter Halogen und (C₁-C₆)Alkyl ausgewählten Gruppen substituiert ist, welche optional mit einer oder mehreren, unabhängig unter Halogen ausgewählten Gruppen substituiert ist.

7. Verbindung nach einem der Ansprüche 1 oder 2, worin R³ für 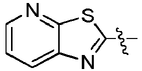 steht, welches mit einer oder mehreren, unabhängig unter Trifluoromethyl, Pentafluoroethyl oder 1-(Trifluoromethyl)cyclopropyl ausgewählten Gruppen substituiert ist.

8. Verbindung nach einem der Ansprüche 1 oder 2, worin R³ für 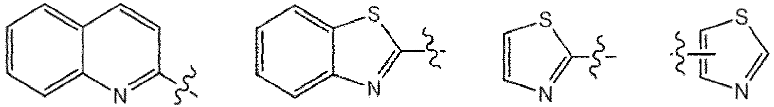 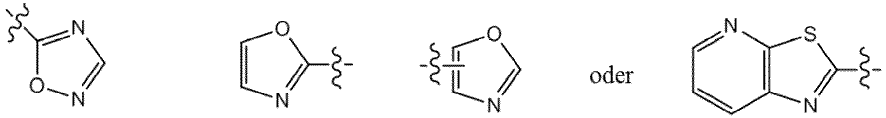 steht, welches optional mit einer oder mehreren, unabhängig unter R^{h}, Halogen, Hydroxy, -NR^{e}R^{f}, -CR^{g}(=N)N(R^{g})₂, -NR^{g}C(=N)-N(R^{g})₂, -NR^{g}-C(=NR^{g})R^{g}, (C₁-C₆)Alkyl und (C₃-C₈)Cycloalkyl ausgewählten Gruppen substituiert ist, wobei jedes (C₁-C₆)Alkyl und (C₃-C₈)Cycloalkyl optional mit einer oder mehreren, unabhängig unter Halogen, Hydroxy, -NR^{e}R^{f}, -CR^{g}(=N)N(R^{g})₂, -NR^{g}C(=N)-N(R^{g})₂, -NR^{g}-C(=NR^{g})R^{g} und (C₁-C₆)Alkyl ausgewählten Gruppen substituiert ist, welche optional mit einer oder mehreren, unabhängig unter Halogen ausgewählten Gruppen substituiert ist.

9. Verbindung nach einem der Ansprüche 1 oder 2, worin R³ für 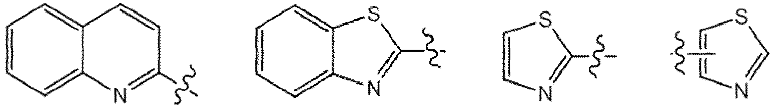 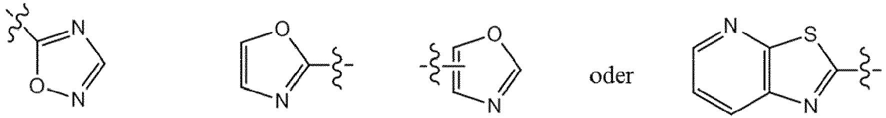 steht, welches optional mit einer oder mehreren, unabhängig unter Trifluoromethyl, Pentafluoroethyl oder 1-(Trifluoromethyl)cyclopropyl ausgewählten Gruppen substituiert ist.

10. Verbindung nach einem der Ansprüche 1 oder 2, worin: 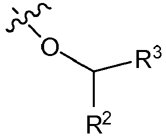 ausgewählt ist unter:

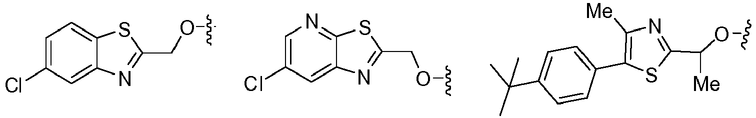

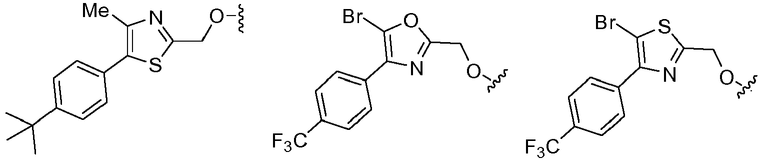

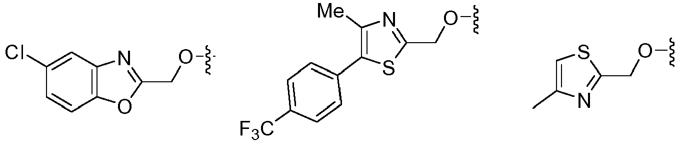

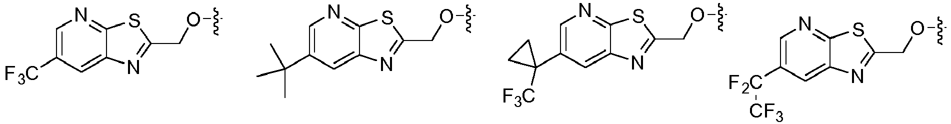

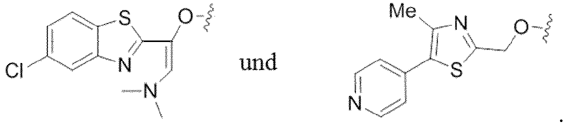

11. Verbindung nach einem der Ansprüche 1-2, worin W für -NHC(=O)H, - NHC(=O)CH₃, -NHC(=O)CH₂CH₃, -NHC(=O)CH₂CH₂CH₃, -N=NCH-N(CH₃)₂, -NHCH₂OH, -N=NC(CH₃)-N(CH₃)₂, 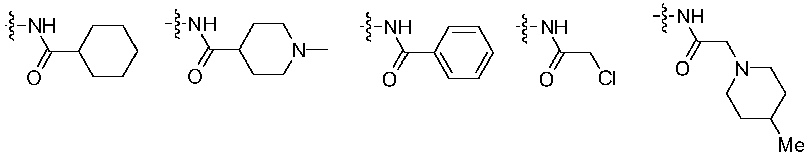 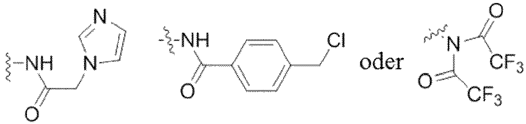 steht.

12. Verbindung nach einem der Ansprüche 1 oder 2, welche eine Verbindung der Formel (Ia) 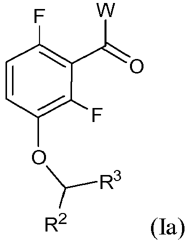 oder ein Salz davon ist.

13. Verbindung nach Anspruch 1, die ausgewählt ist unter: 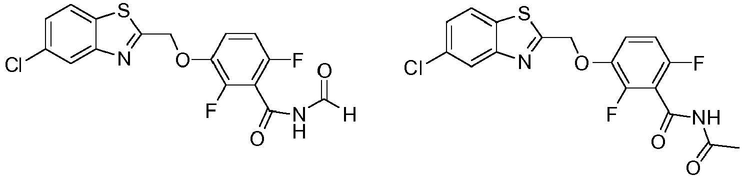 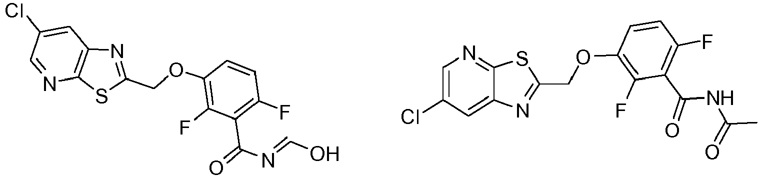 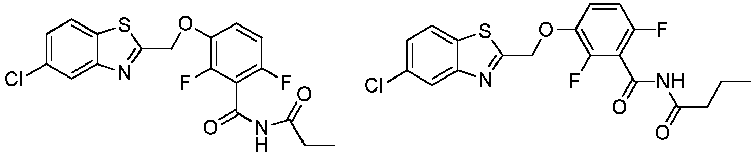 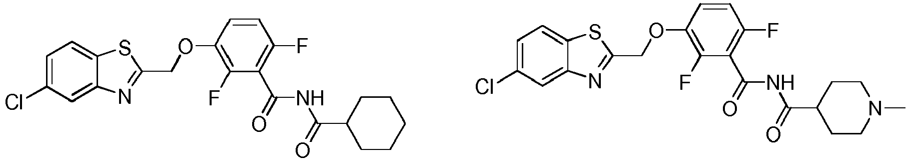 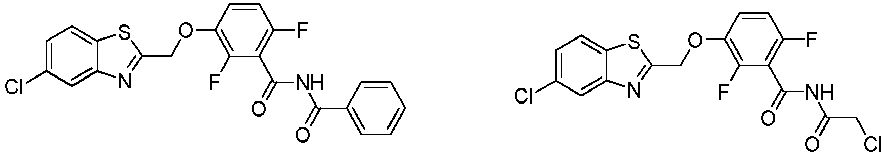 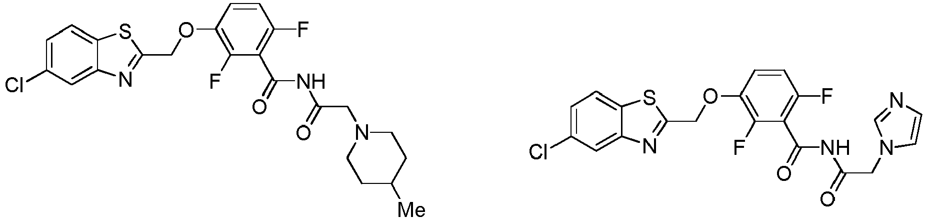 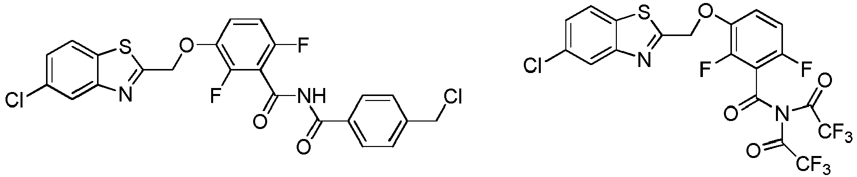 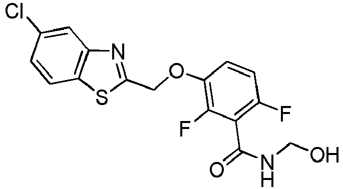 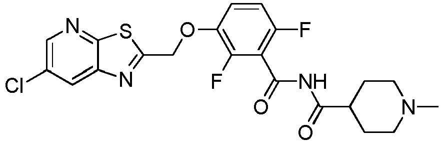 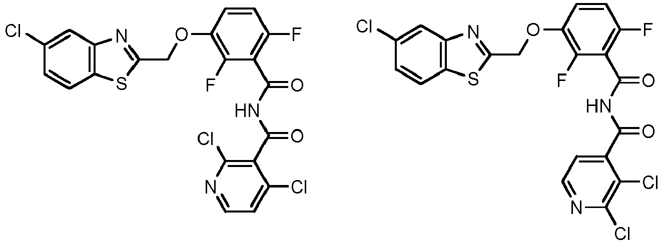 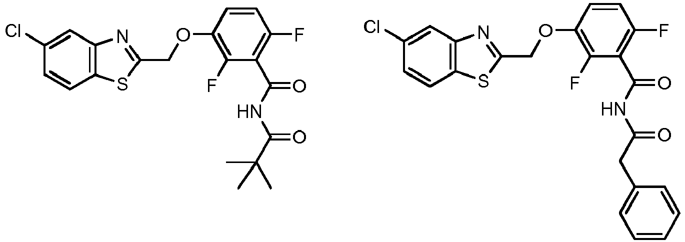 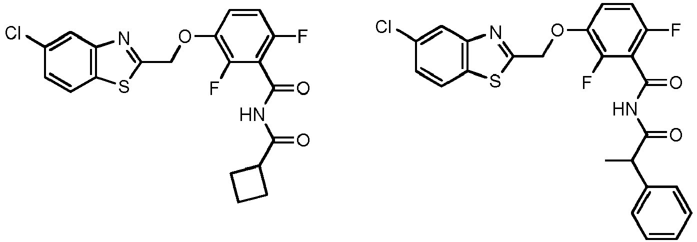 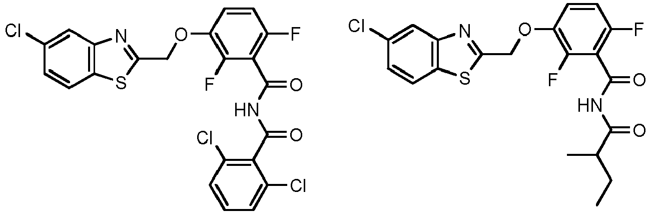 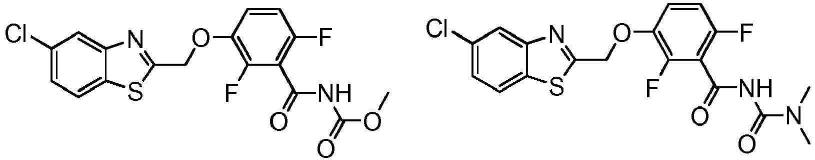 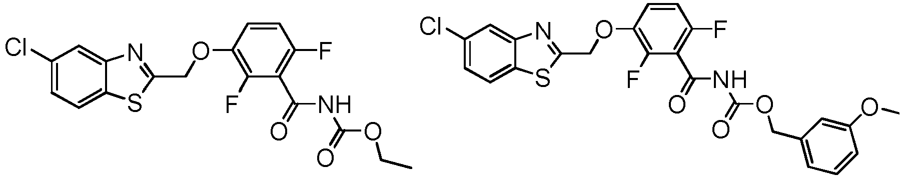 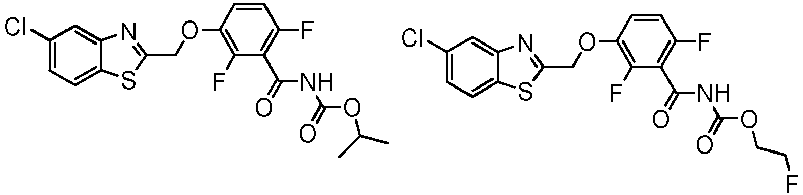 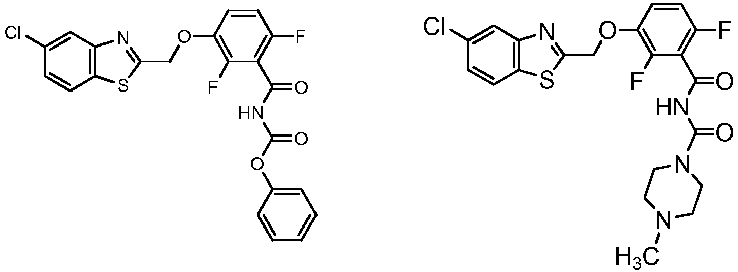 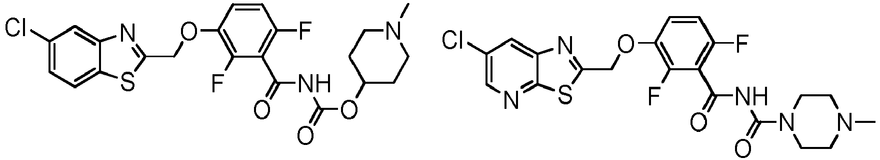 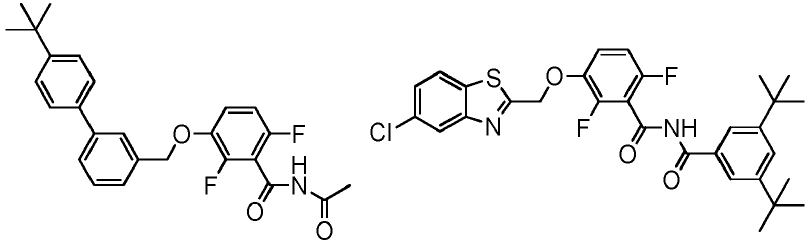 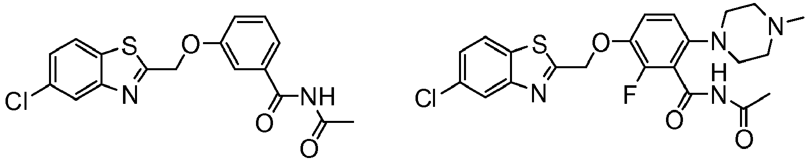 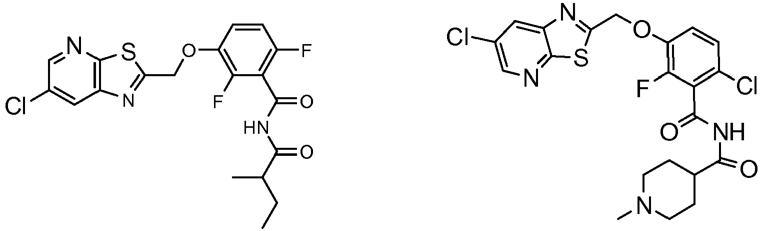 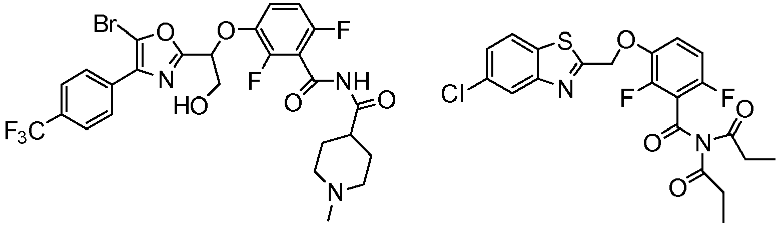 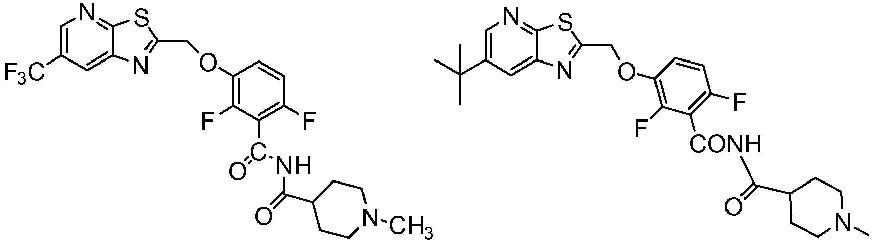 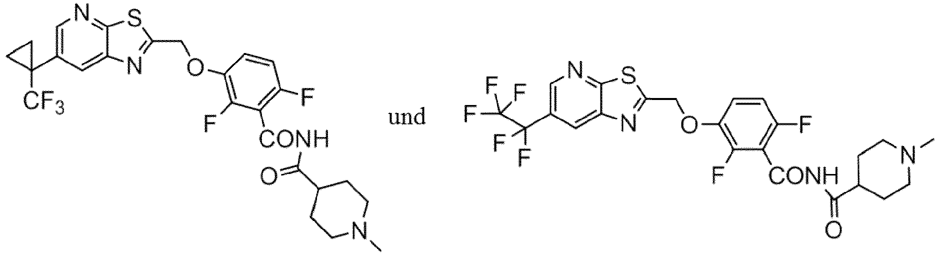 und Salzen davon.

14. Verbindung nach Anspruch 1, die 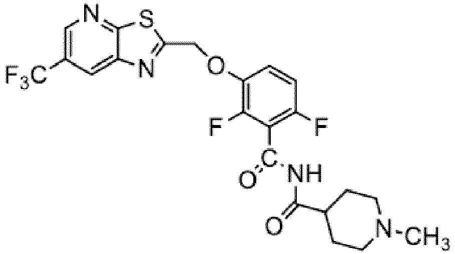 oder ein Salz davon ist.

15. Pharmazeutische Zusammensetzung, umfassend eine Verbindung der Formel I nach einem der Ansprüche 1-14 oder ein pharmazeutisch verträgliches Salz davon und ein pharmazeutisch verträgliches Vehikel.

16. Verbindung der Formel I nach einem der Ansprüche 1-14 oder ein pharmazeutisch verträgliches Salz davon zur Verwendung bei der prophylaktischen oder therapeutischen Behandlung einer bakteriellen Infektion.

17. Verbindung der Formel I nach einem der Ansprüche 1-14 oder ein pharmazeutisch verträgliches Salz davon zur Verwendung bei der medizinischen Behandlung.

18. Verfahren zur Herstellung der Verbindung nach Anspruch 14, bei dem man ein Amid der Formel: 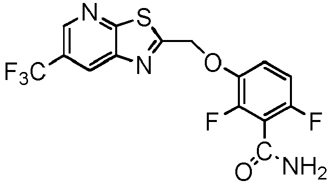 mit 1-Methylpiperidin-4-carbonylchlorid in der Gegenwart von NaH umsetzt.

19. Amid der Formel:

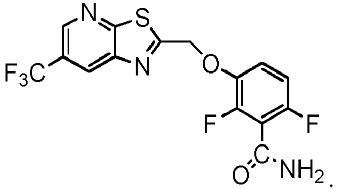

## Revendications

1. Composé de formule (I) : 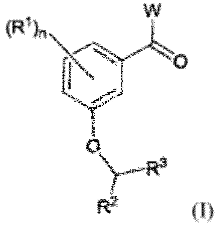
chaque R¹ étant indépendamment choisi parmi hydrogène, halogéno, cyano, nitro, C₁₋₆-alkyle, C₁₋₆-alcoxyle, C₁₋₆-alcanoyle, C₁₋₆-alcoxycarbonyle, C₁₋₆-alcanoyloxyle, aryle, hétéroaryle, hétérocycle et NR^{e}R^{f}, chacun de C₁₋₆-alkyle, C₁₋₆-alcoxyle, C₁₋₆-alcanoyle, C₁₋₆-alcoxycarbonyle, C₁₋₆-alcanoyloxyle, aryle, hétéroaryle et hétérocycle étant éventuellement substitué par un ou plusieurs groupe(s) indépendamment choisi(s) parmi halogéno, cyano, nitro, NR^{e}R^{f}, -CR^{g}(=N)N(R^{g})₂, -NR^{g}C(=N)-N(R^{g})₂, - NR^{g}-C(=NR^{g})R^{g}, C₁₋₃-alkyle, C₁₋₃-alcoxyle, C₁₋₃-alcanoyle, C₁₋₃-alcoxycarbonyle, C₁₋₃-alcanoyloxyle, aryle, hétéroaryle et hétérocycle ;
R² représentant H ou C₁₋₆-alkyle éventuellement substitué par un ou plusieurs groupe(s) indépendamment choisi(s) parmi -OR^{k}, halogéno, NR^{e}R^{f}, NR^{e}R^{f}, -CR^{g}(=N)N(R^{g})₂, -NR^{g}C(=N)-N(R^{g})₂ et - NR^{g}-C(=NR^{g})R^{g} ;
R³ représentant aryle ou hétéroaryle, aryle ou hétéroaryle étant éventuellement substitué par un ou plusieurs groupe(s) indépendamment choisi (s) parmi R^{h}, halogéno, hydroxyle, -NR^{e}R^{f}, -CR^{g}(=N)N(R^{g})₂, -NR^{g}C(=N) -N(R^{g})₂, -NR^{g}-C(=NR^{g})R^{g}, C₁₋₆-alkyle et C₃₋₈-cycloalkyle, l'un quelconque parmi C₁₋₆-alkyle et C₃₋₈-cycloalkyle étant éventuellement substitué par un ou plusieurs groupe(s) indépendamment choisi(s) parmi halogéno, hydroxyle, -NR^{e}R^{f}, -CR^{g}(=N)N(R^{g})₂, - NR^{g}C(=N) -N(R^{g})₂, -NR^{g}-C(=NR^{g})R^{g} et C₁₋₆-alkyle qui est éventuellement substitué par un ou plusieurs groupe(s) indépendamment choisi(s) parmi halogéno ;
W représentant -NHCOR^{a}, -N(COR^{a})(COR^{b}), - N=C(R^{c})NR^{a}R^{b}, -NR^{a}CH₂OR^{a}, -NHC(=O)OR^{a}-NHC(=O)NR^{a}R^{b} ou -N(R^{a})SOₘR^{d};
chaque R^{a} étant indépendamment choisi parmi H, aryle, hétéroaryle, hétérocycle, C₃₋₈-cycloalkyle, (C₃₋₈-cycloalkyl) -C₁₋₆-alkyle et C₁₋₆-alkyle qui est éventuellement substitué par un ou plusieurs groupe(s) indépendamment choisi(s) parmi hydroxyle, halogéno, cyano, C₁₋₆-alcoxycarbonyle, aryle, hétéroaryle, -NR^{e}R^{f}, - CR^{g}(=N)N(R^{g})₂, -NR^{g}C(=N)-N(R^{g})₂, -NR^{g}-C(=NR^{g})R^{g} et hétérocycle ; l'un quelconque parmi aryle, hétéroaryle, hétérocycle et (C₃₋₈-cycloalkyl) -C₁₋₆-alkyle de R^{a} étant éventuellement substitué par un ou plusieurs groupe(s) indépendamment choisi(s) parmi hydroxyle, halogéno, cyano, trifluorométhoxyle, C₁₋₆-alkyle, C₁₋₆-alcoxyle, C₁₋₆-halogénoalkyle, -NR^{e}R^{f}, -CR^{g}(=N)N(R^{g})₂, - NR^{g}C(=N) -N(R^{g})₂, -NR^{g}-C(=NR^{g})R^{g} et C₁₋₆-alcoxycarbonyle ;
chaque R^{b} étant indépendamment choisi parmi H et C₁₋₆-alkyle qui est éventuellement substitué par un ou plusieurs groupe(s) indépendamment choisi(s) parmi hydroxyle, halogéno, cyano, C₁₋₆-alcoxycarbonyle, aryle, hétéroaryle, -NR^{e}R^{f}, - CR^{g}(=N)N(R^{g})₂, -NR^{g}C(=N) -N(R^{g})₂, -NR^{g}-C(=NR^{g})R^{g} et hétérocycle ;
chaque R^{c} étant indépendamment choisi parmi H et C₁₋₆-alkyle qui est éventuellement substitué par un ou plusieurs groupe(s) indépendamment choisi(s) parmi halogéno ;
chaque R^{d} étant indépendamment choisi parmi OH, -NH₂, -NR^{e}R^{f}, hétérocycle et C₁₋₆-alkyle qui est éventuellement substitué par un ou plusieurs groupe(s) indépendamment choisi(s) parmi hydroxyle, halogéno, cyano, C₁₋₆-alcoxycarbonyle, aryle, hétéroaryle, -NR^{e}R^{f}, -CH(=N)NH₂, -NHC(=N)-NH₂, -NH-C(=NH)R et hétérocycle ;
chaque R^{e} étant indépendamment choisi parmi H, aryle, hétéroaryle, hétérocycle et C₁₋₆-alkyle qui est éventuellement substitué par un ou plusieurs groupe(s) indépendamment choisi(s) parmi hydroxyle, halogéno, cyano, C₁₋₆-alcoxycarbonyle, aryle, hétéroaryle et hétérocycle ; et chaque R^{f} étant indépendamment choisi parmi H et C₁₋₆-alkyle éventuellement substitué par un ou plusieurs groupe(s) indépendamment choisi(s) parmi hydroxyle, halogéno, cyano, C₁₋₆-alcoxycarbonyle, aryle, hétéroaryle et hétérocycle ; ou R^{e} et R^{f} conjointement avec l'azote auquel ils sont liés formant aziridino, azétidino, morpholino, pipérazino, pyrrolidino ou pipéridino ;
chaque R^{g} étant indépendamment choisi parmi H et C₁₋₆-alkyle éventuellement substitué par un ou plusieurs groupe(s) indépendamment choisi(s) parmi halogéno ;
chaque R^{h} étant indépendamment choisi parmi aryle et hétéroaryle, l'un quelconque parmi aryle et hétéroaryle de R^{h} étant éventuellement substitué par un ou plusieurs groupe(s) indépendamment choisi(s) parmi halogéno, hydroxyle, -NR^{e}R^{f}, -CR^{g}(=N)N(R^{g})₂, -NR^{g}C(=N) - N(R^{g})₂, -NR^{g}-C(=NR^{g})R^{g} et C₁₋₆-alkyle qui est éventuellement substitué par un ou plusieurs groupe(s) indépendamment choisi(s) parmi hydroxyle, halogéno, -NR^{e}R^{f}, -CR^{g}(=N)N(R^{g})₂, - NR^{g}C(=N)-N(R^{g})₂, -NR^{g}-C(=NR^{g})R^{g} ;
chaque R^{k} étant indépendamment choisi parmi H et C₁₋₆-alkyle éventuellement substitué par un ou plusieurs groupe(s) indépendamment choisi(s) parmi hydroxyle, halogéno, oxo, carboxyle, C₁₋₆-alcoxyle, C₁₋₆-alcoxycarbonyle et C₁₋₆-alcanoyloxyle ;
chaque aryle étant indépendamment un seul cycle aromatique ou un système à plusieurs cycles condensés possédant 9 à 20 atomes de carbone, dans lequel au moins un cycle est aromatique ;
chaque cycloalkyle étant indépendamment un système de cycles carbocycliques saturé ou partiellement insaturé ;
m étant 0, 1, ou 2 ; et
n étant 1, 2, 3 ou 4 ;
ou un sel correspondant.

2. Composé selon la revendication 1 :
chaque R¹ étant indépendamment choisi parmi hydrogène, halogéno, cyano, nitro, C₁₋₆-alkyle, C₁₋₆-alcoxyle, C₁₋₆-alcanoyle, C₁₋₆-alcoxycarbonyle, C₁₋₆-alcanoyloxyle, aryle, hétéroaryle, hétérocycle et NR^{e}R^{f}, chacun de C₁₋₆-alkyle, C₁₋₆-alcoxyle, C₁₋₆-alcanoyle, C₁₋₆-alcoxycarbonyle, C₁₋₆-alcanoyloxyle, aryle, hétéroaryle et hétérocycle étant éventuellement substitué par un ou plusieurs groupe(s) indépendamment choisi(s) parmi halogéno, cyano, nitro, -NR^{e}R^{f}, -CR^{g}(=N)N(R^{g})₂, -NR^{g}C(=N)-N(R^{g})₂, - NR^{g}-C(=NR^{g})R^{g}, C₁₋₃-alkyle, C₁₋₃-alcoxyle, C₁₋₃-alcanoyle, C₁₋₃-alcoxycarbonyle, C₁₋₃-alcanoyloxyle, aryle, hétéroaryle et hétérocycle ;
R² représentant H ou C₁₋₆-alkyle éventuellement substitué par un ou plusieurs groupe(s) indépendamment choisi(s) parmi hydroxyle, - NR^{e}R^{f}, -CR^{g}(=N)N(R^{g})₂, -NR^{g}C(=N) -N(R^{g})₂ et -NR^{g}-C(=NR^{g})R^{g} ;
R³ représentant aryle ou hétéroaryle, aryle ou hétéroaryle étant éventuellement substitué par un ou plusieurs groupe(s) indépendamment choisi (s) parmi R^{h}, halogéno, hydroxyle, -NR^{e}R^{f}, -CR^{g}(=N)N(R^{g})₂, -NR^{g}C(=N)-N(R^{g})₂, -NR^{g}-C(=NR^{g})R^{g} et C₁₋₆-alkyle qui est éventuellement substitué par un ou plusieurs groupe(s) indépendamment choisi(s) parmi hydroxyle, -NR^{e}R^{f}, - CR^{g}(=N)N(R^{g})₂, -NR^{g}C(=N)-N(R^{g})₂ et -NR^{g}-C(=NR^{g})R^{g} ;
W représentant -NHCOR^{a}, -N(COR^{a})(COR^{b}), - N=C(R^{c})NR^{a}R^{b}, -NR^{a}CH₂OR^{a} ou -N(R^{a})SOₘR^{d} ;
chaque R^{a} étant indépendamment choisi parmi H, aryle, hétéroaryle, hétérocycle, C₃₋₈-cycloalkyle, (C₃₋₈-cycloalkyl) -C₁₋₆-alkyle et C₁₋₆-alkyle qui est éventuellement substitué par un ou plusieurs groupe(s) indépendamment choisi(s) parmi hydroxyle, halogéno, cyano, C₁₋₆-alcoxycarbonyle, aryle, hétéroaryle, -NR^{e}R^{f}, - CR^{g}(=N)N(R^{g})₂, -NR^{g}C(=N) -N(R^{g})₂, -NR^{g}-C(=NR^{g})R^{g} et hétérocycle ; l'un quelconque parmi aryle, hétéroaryle, hétérocycle et (C₃₋₈-cycloalkyl) -C₁₋₆-alkyle de R^{a} étant éventuellement substitué par un ou plusieurs groupe(s) indépendamment choisi(s) parmi hydroxyle, halogéno, cyano, trifluorométhoxyle, C₁₋₆-alkyle, C₁₋₆-halogénoalkyle, -NR^{e}R^{f}, -CR^{g}(=N)N(R^{g})₂, -NR^{g}C(=N)-N(R^{g})₂, -NR^{g}-C(=NR^{g})R^{g} et C₁₋₆-alcoxycarbonyle ;
chaque R^{b} étant indépendamment choisi parmi H et C₁₋₆-alkyle éventuellement substitué par un ou plusieurs groupe(s) indépendamment choisi(s) parmi hydroxyle, halogéno, cyano, C₁₋₆-alcoxycarbonyle, aryle, hétéroaryle, -NR^{e}R^{f}, - CR^{g}(=N)N(R^{g})₂, -NR^{g}C(=N) -N(R^{g})₂, -NR^{g}-C(=NR^{g})R^{g} et hétérocycle ;
chaque R^{c} étant indépendamment choisi parmi H et C₁₋₆-alkyle ;
chaque R^{d} étant indépendamment choisi parmi OH, -NH₂, -NR^{e}R^{f}, hétérocycle et C₁₋₆-alkyle qui est éventuellement substitué par un ou plusieurs groupe(s) indépendamment choisi(s) parmi hydroxyle, halogéno, cyano, C₁₋₆-alcoxycarbonyle, aryle, hétéroaryle, -NR^{e}R^{f}, -CH(=N)NH₂, -NHC(=N)-NH₂, -NH-C(=NH)R et hétérocycle ;
chaque R^{e} étant indépendamment choisi parmi H, aryle, hétéroaryle, hétérocycle et C₁₋₆-alkyle qui est éventuellement substitué par un ou plusieurs groupe(s) indépendamment choisi(s) parmi hydroxyle, halogéno, cyano, C₁₋₆-alcoxycarbonyle, aryle, hétéroaryle et hétérocycle ; et chaque R^{f} étant indépendamment choisi parmi H et C₁₋₆-alkyle éventuellement substitué par un ou plusieurs groupe(s) indépendamment choisi(s) parmi hydroxyle, halogéno, cyano, C₁₋₆-alcoxycarbonyle, aryle, hétéroaryle et hétérocycle ; ou R^{e} et R^{f} conjointement avec l'azote auquel ils sont liés formant aziridino, azétidino, morpholino, pipérazino, pyrrolidino ou pipéridino ;
chaque R^{g} étant indépendamment choisi parmi H et C₁₋₆-alkyle ;
chaque R^{h} étant indépendamment choisi parmi aryle et hétéroaryle, l'un quelconque parmi aryle et hétéroaryle de R^{h} étant éventuellement substitué par un ou plusieurs groupe(s) indépendamment choisi(s) parmi halogéno, hydroxyle, -NR^{e}R^{f}, -CR^{g}(=N)N(R^{g})₂, -NR^{g}C(=N) - N(R^{g})₂, -NR^{g}-C(=NR^{g})R^{g} et C₁₋₆-alkyle qui est éventuellement substitué par un ou plusieurs groupe(s) indépendamment choisi(s) parmi hydroxyle, -NR^{e}R^{f}, -CR^{g}(=N)N(R^{g})₂, -NR^{g}C(=N) - N(R^{g})₂ et -NR^{g}-C(=NR^{g})R^{g} ;
m étant 0, 1 ou 2 ; et
n étant 1, 2, 3 ou 4 ;
ou un sel correspondant.

3. Composé selon l'une quelconque des revendications 1 ou 2, R³ étant hétéroaryle, éventuellement substitué par un ou plusieurs groupe(s) indépendamment choisi(s) parmi R^{h}, halogéno, hydroxyle, -NR^{e}R^{f}, -CR^{g}(=N)N(R^{g})₂, -NR^{g}C(=N)-N(R^{g})₂, -NR^{g}-C(=NR^{g})R^{g}, C₁₋₆-alkyle et C₃₋₈-cycloalkyle, l'un quelconque parmi C₁₋₆-alkyle et C₃₋₈-cycloalkyle étant éventuellement substitué par un ou plusieurs groupe(s) indépendamment choisi(s) parmi halogéno, hydroxyle, -NR^{e}R^{f}, - CR^{g}(=N)N(R^{g})₂, -NR^{g}C(=N)-N(R^{g})₂, -NR^{g}-C(=NR^{g})R^{g} et C₁₋₆-alkyle qui est éventuellement substitué par un ou plusieurs groupe(s) indépendamment choisi(s) parmi halogéno.

4. Composé selon l'une quelconque des revendications 1 ou 2, R³ étant : 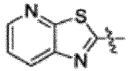 qui est éventuellement substitué par un ou plusieurs groupe(s) indépendamment choisi(s) parmi R^{h}, halogéno, hydroxyle, -NR^{e}R^{f}, - CR^{g}(=N)N(R^{g})₂, -NR^{g}C(=N)-N(R^{g})₂, -NR^{g}-C(=NR^{g})R^{g}, C₁₋₆-alkyle et C₃₋₈-cycloalkyle, l'un quelconque parmi C₁₋₆-alkyle et C₃₋₈-cycloalkyle étant éventuellement substitué par un ou plusieurs groupe(s) indépendamment choisi(s) parmi halogéno, hydroxyle, -NR^{e}R^{f}, -CR^{g}(=N)N(R^{g})₂, - NR^{g}C(=N)-N(R^{g})₂, -NR^{g}-C(=NR^{g})R^{g} et C₁₋₆-alkyle qui est éventuellement substitué par un ou plusieurs groupe(s) indépendamment choisi(s) parmi halogéno.

5. Composé selon l'une quelconque des revendications 1 ou 2, R³ étant : 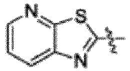 éventuellement substitué par un ou plusieurs groupe (s) indépendamment choisi (s) parmi C₁₋₆-alkyle et C₃₋₈-cycloalkyle, l'un quelconque parmi C₁₋₆-alkyle et C₃₋₈-cycloalkyle étant éventuellement substitué par un ou plusieurs groupe(s) indépendamment choisi(s) parmi halogéno, hydroxyle et C₁₋₆-alkyle qui est éventuellement substitué par un ou plusieurs groupe(s) indépendamment choisi(s) parmi halogéno.

6. Composé selon l'une quelconque des revendications 1 ou 2, R³ étant : 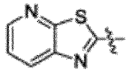 qui est substitué par un ou plusieurs groupe (s) indépendamment choisi (s) parmi C₁₋₆-alkyle et C₃₋₈-cycloalkyle, l'un quelconque parmi C₁₋₆-alkyle et C₃₋₈-cycloalkyle étant éventuellement substitué par un ou plusieurs groupe(s) indépendamment choisi (s) parmi halogéno et C₁₋₆-alkyle qui est éventuellement substitué par un ou plusieurs groupe(s) indépendamment choisi(s) parmi halogéno.

7. Composé selon l'une quelconque des revendications 1 ou 2, R³ étant : 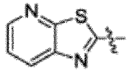 qui est substitué par un ou plusieurs groupe (s) indépendamment choisi(s) parmi trifluorométhyle, pentafluoroéthyle ou 1-(trifluorométhyl)cyclopropyle.

8. Composé selon l'une quelconque des revendications 1 ou 2, R³ étant : 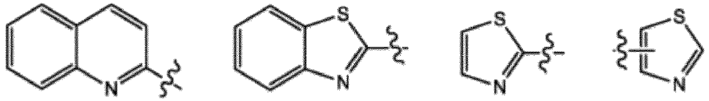 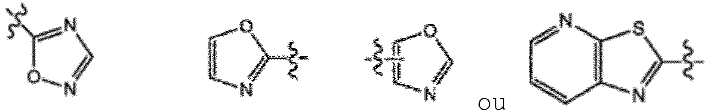 qui est éventuellement substitué par un ou plusieurs groupe(s) indépendamment choisi(s) parmi R^{h}, halogéno, hydroxyle, -NR^{e}R^{f},-CR^{g}(=N)N(R^{g})₂, -NR^{g}C(=N)-N(R^{g})₂, -NR^{g}-C(=NR^{g})R^{g}, C₁₋₆-alkyle et C₃₋₈-cycloalkyle, l'un quelconque parmi C₁₋₆-alkyle et C₃₋₈-cycloalkyle étant éventuellement substitué par un ou plusieurs groupe(s) indépendamment choisi(s) parmi halogéno, hydroxyle, -NR^{e}R^{f}, -CR^{g}(=N)N(R^{g})₂,-NR^{g}C(=N)-N(R^{g})₂, -NR^{g}-C(=NR^{g})R^{g} et C₁₋₆-alkyle qui est éventuellement substitué par un ou plusieurs groupe(s) indépendamment choisi(s) parmi halogéno.

9. Composé selon l'une quelconque des revendications 1 ou 2, R³ étant : 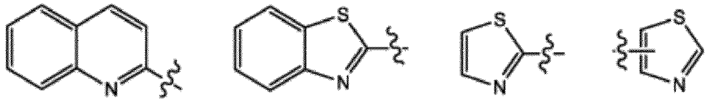 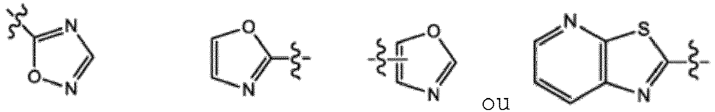 qui est éventuellement substitué par un ou plusieurs groupe(s) indépendamment choisi(s) parmi trifluorométhyle, pentafluoroéthyle ou 1-(trifluorométhyl)cyclopropyle.

10. Composé selon l'une quelconque des revendications 1 ou 2 : 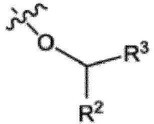 étant choisi parmi :

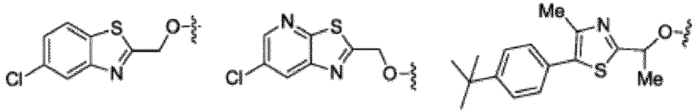

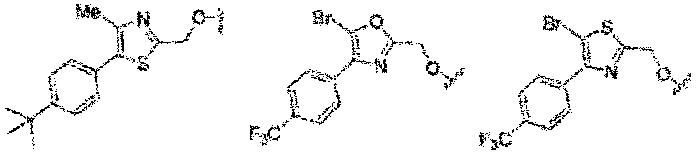

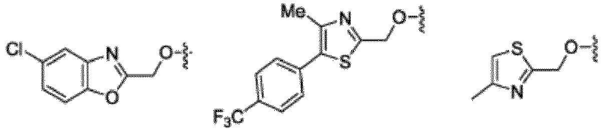

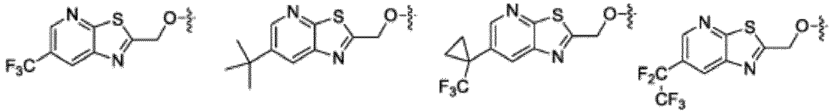

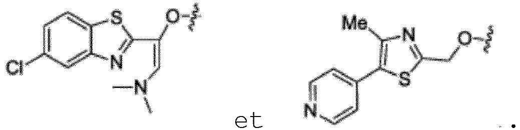

11. Composé selon l'une quelconque des revendications 1-2, W représentant -NHC(=O)H,-NHC(=O)CH₃, -NHC(=O)CH₂CH₃, -NHC(=O)CH₂CH₂CH₃,-N=NCH-N(CH₃)₂, -NHCH₂OH, -N=NC (CH₃)-N(CH₃)₂,

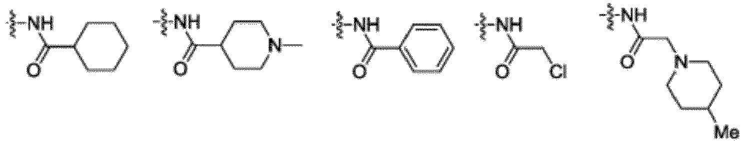

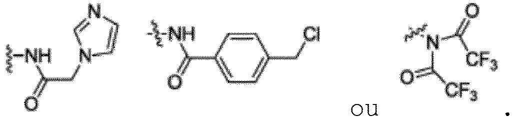

12. Composé selon l'une quelconque des revendications 1 ou 2, qui est un composé de formule (Ia) : 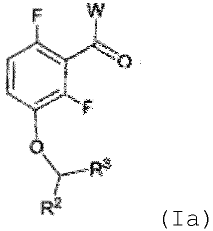 ou un sel correspondant.

13. Composé selon la revendication 1 choisi parmi : 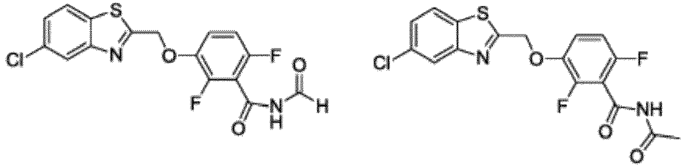 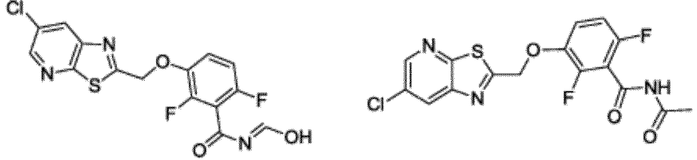 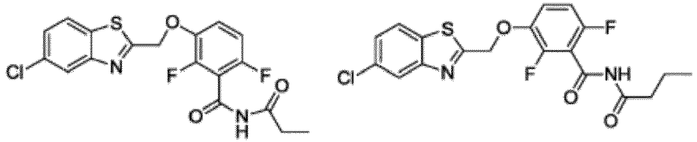 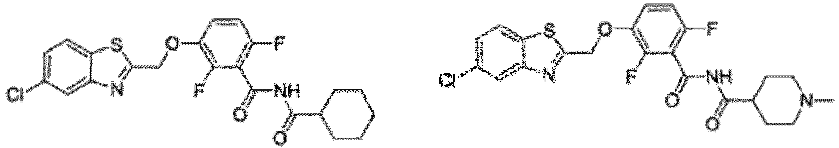 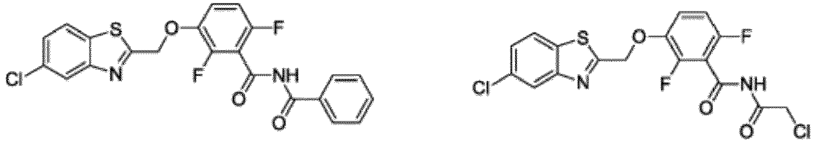 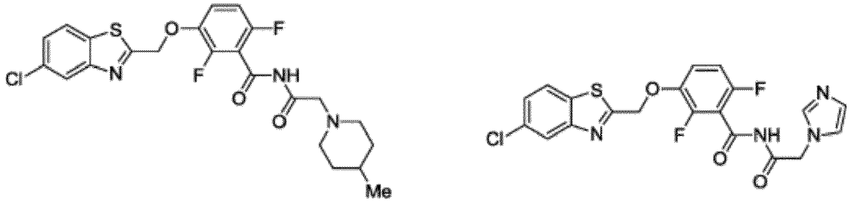 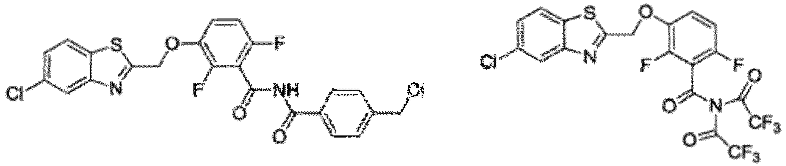 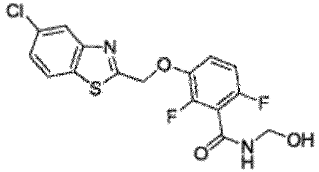 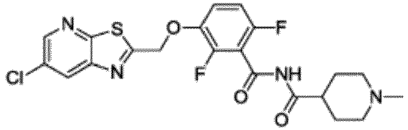 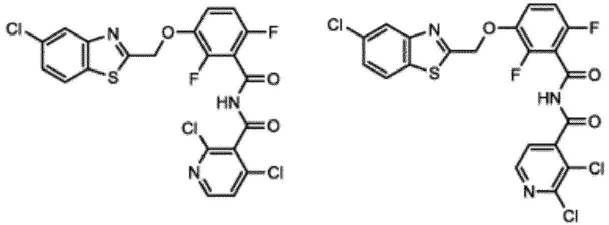 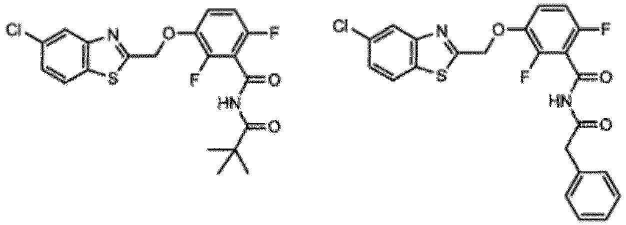 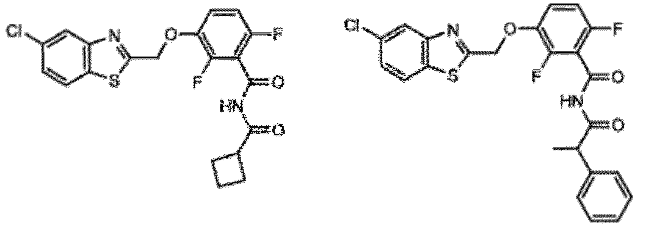 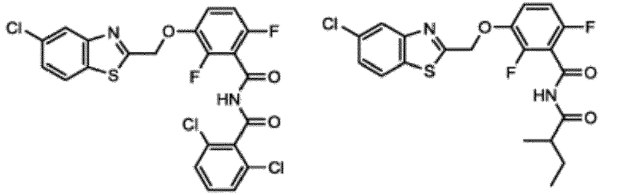 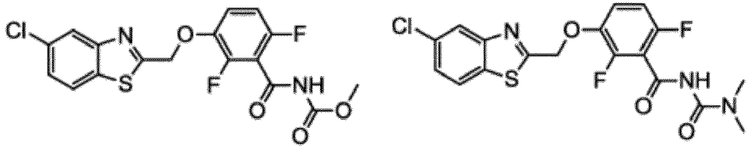 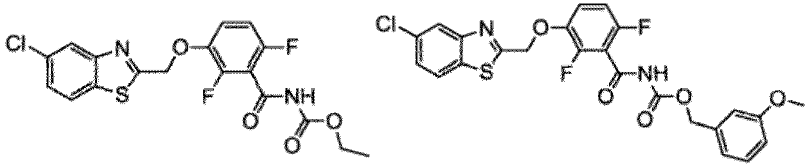 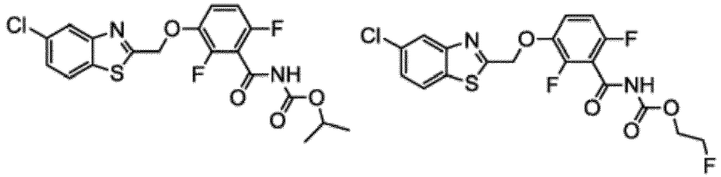 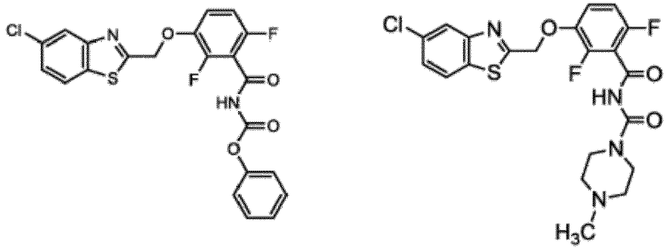 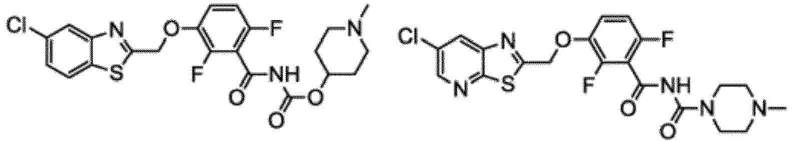 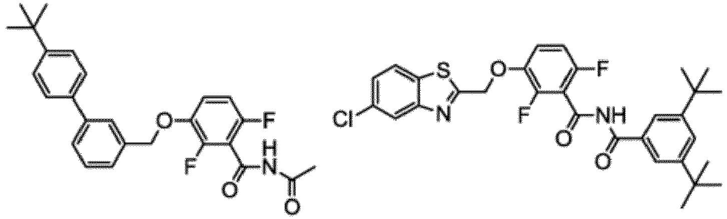 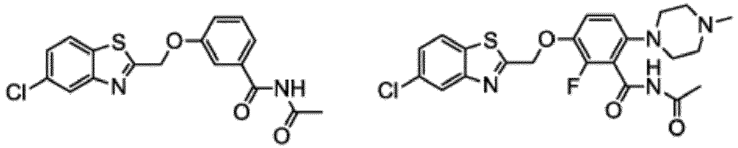 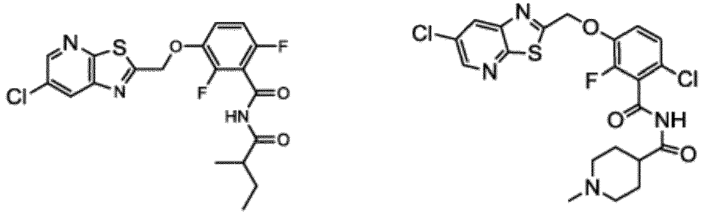 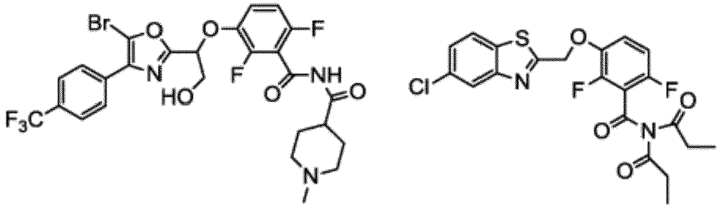 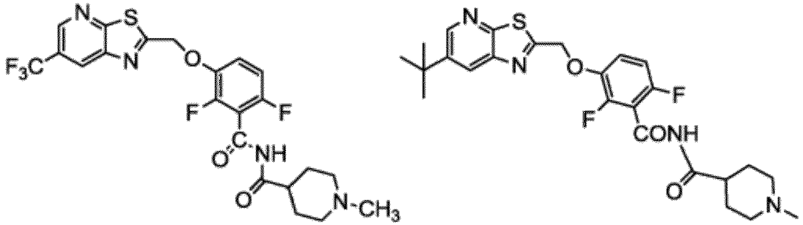 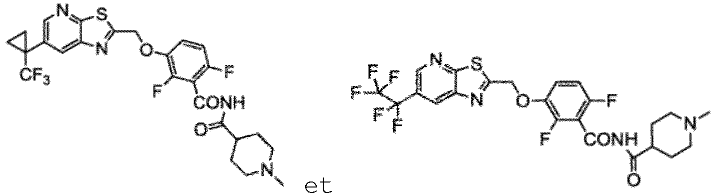 et des sels correspondants.

14. Composé selon la revendication 1, qui est 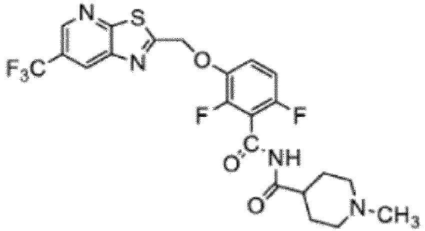 ou un sel correspondant.

15. Composition pharmaceutique comprenant un composé de formule I tel que décrit selon l'une quelconque des revendications 1-14 ou un sel pharmaceutiquement acceptable correspondant et un véhicule pharmaceutiquement acceptable.

16. Composé de formule I tel que décrit selon l'une quelconque des revendications 1-14 ou un sel pharmaceutiquement acceptable correspondant pour une utilisation dans le traitement prophylactique ou thérapeutique d'une infection bactérienne.

17. Composé de formule I tel que décrit selon l'une quelconque des revendications 1-14 ou un sel pharmaceutiquement acceptable correspondant pour une utilisation dans un traitement médical.

18. Procédé de préparation du composé selon la revendication 14 comprenant la transformation d'un amide de formule : 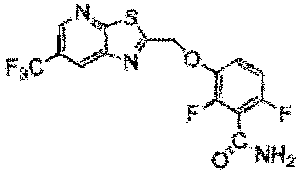 avec du chlorure de 1-méthylpipéridine-4-carbonyle en présence de NaH.

19. Amide de formule :
